(19) Europäisches Patentamt
European Patent Office
Office européen des brevets

(11) EP 4 512 863 A1

(12) **EUROPEAN PATENT APPLICATION**
published in accordance with Art. 153(4) EPC

(43) Date of publication:
**26.02.2025 Bulletin 2025/09**

(21) Application number: **23904797.0**

(22) Date of filing: **06.09.2023**

(51) International Patent Classification (IPC):
***C09B 19/00*** (2006.01) ***C09B 57/00*** (2006.01)
***C07D 451/00*** (2006.01) ***A61K 31/46*** (2006.01)
***G01N 33/533*** (2006.01) ***G01N 33/58*** (2006.01)

(86) International application number:
**PCT/CN2023/117328**

(87) International publication number:
**WO 2025/007415 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.07.2023 CN 202310809062**

(71) Applicants:
- **Peking University**
**Beijing 100871 (CN)**
- **Nanjing Genvivo Biotech Co., Ltd.**
**Nanjing, Jiangsu 211899 (CN)**

(72) Inventors:
- **CHEN, Zhixing**
**Beijing 100871 (CN)**
- **ZHANG, Junwei**
**Beijing 100871 (CN)**
- **LIU, Mingqiao**
**Beijing 100871 (CN)**
- **CHEN, Peng**
**Beijing 100871 (CN)**
- **SUN, Jingfu**
**Beijing 100871 (CN)**

(74) Representative: **Barker Brettell LLP**
**100 Hagley Road**
**Edgbaston**
**Birmingham B16 8QQ (GB)**

# (54) AZA[3.2.1] BRIDGED RING SUBSTITUTED FLUORESCENT DYE, AND PREPARATION METHOD THEREFOR AND USE THEREOF

(57) The present disclosure provides an aza[3.2.1] bridged ring substituted fluorescent dye and a preparation method and use thereof. According to the fluorescent dye, by adopting an aza[3.2.1]bridged ring as a substituent of the fluorescent dye, not only are the brightness and photostability of the fluorescent dye obviously improved, but also the phenomenon of spectral blue shift caused by irradiation of the dye for a long time is completely avoided. Meanwhile, the water solubility of the fluorescent dye can be effectively improved, and good membrane permeability of the fluorescent dye is maintained. The present disclosure further provides embodiments of functionalized derivatives of the dye in single molecule imaging, confocal imaging and super-resolution imaging of biological samples.

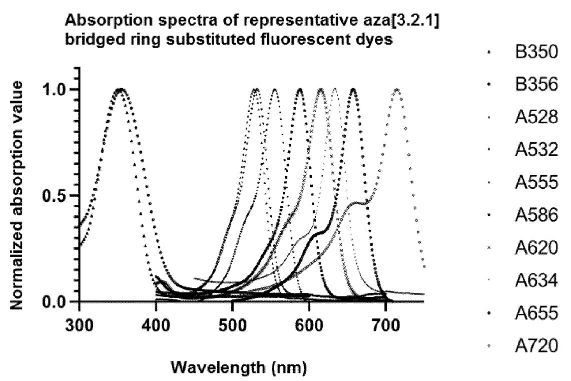


Fig. 1

EP 4 512 863 A1

## Description

### Cross-Reference to Related Application

The present disclosure claims the priority of a Chinese patent application No. 2023108090626, filed to China National Intellectual Property Administration on July 03, 2023, with the title of "Aza[3.2.1]bridged Ring Substituted Fluorescent Dye and Preparation Method and Use thereof", the entire contents of which are incorporated herein by reference.

### Technical Field

The present disclosure relates to the field of fluorescent dyes in biofluorescence analysis, and specifically relates to an aza[3.2.1]bridged ring substituted fluorescent dye and a preparation method and use thereof.

### Background

In recent years, fluorescent dyes have been widely used in the fields of biological macromolecular labeling, ion detection, single molecule imaging, super-resolution imaging and the like, and have become an irreplaceable important tool in life science research. Thus, higher requirements on physical and chemical properties of the fluorescent dyes have been put forward. Traditional fluorescent dyes, such as fluorescein, tetramethylrhodamine and coumarin, are usually in lack of sufficient brightness, water solubility and photostability. In the past three decades, different novel substituents have been introduced into matrices of the fluorescent dyes by chemists to improve the fluorescence quantum yield, photostability, resistance to photooxidation dealkylation (spectral blue shift), water solubility and membrane permeability of the dyes. However, the challenge is that currently reported strategies for optimizing the fluorescent dyes can be used for only optimizing a few properties separately and cannot be used for comprehensively improving all the core properties of the dyes.

According to the document Nature Methods. 2015, 12: 244-250, the brightness of a fluorescent dye is systematically improved by an azacyclobutane substitution method, but the dye has poor photostability and no resistance to photooxidation dealkylation (spectral blue shift). According to the document J. Am. Chem. Soc. 2008, 130, 52: 17652-17653, the brightness, photostability and resistance to photooxidation dealkylation (spectral blue shift) of a dye are improved by a method of substituting rhodamine with azabicyclo[2.2.1]heptane, but the dye has poor water solubility and membrane permeability, so that use of the dye in living cell imaging is hindered. By introducing a sulfonic acid group into a matrix of a fluorescent dye, the water solubility of the dye can be greatly improved, the antibody labeling efficiency is improved, and non-specific dyeing is reduced, but the dye is also prevented from permeating a cell membrane, so that the sulfonated dye cannot be used in living cells.

Therefore, it is urgent for persons skilled in the art to develop a class of fluorescent dyes capable of simultaneously improving the fluorescence quantum yield, the photostability, the resistance to photooxidation dealkylation (spectral blue shift), the water solubility and the membrane permeability.

### SUMMARY

The main purpose of the present disclosure is to provide an aza[3.2.1]bridged ring substituted fluorescent dye and a preparation method and use thereof, so as to solve the problems that the fluorescence quantum yield, photostability, resistance to photooxidation dealkylation (spectral blue shift), water solubility and membrane permeability of fluorescent dyes in the prior art cannot be optimized simultaneously.

In order to achieve the above purpose, an aza[3.2.1]bridged ring substituted fluorescent dye is provided according to one aspect of the present disclosure. The aza[3.2.1]bridged ring substituted fluorescent dye has a structure shown in Formula (I), Formula (II) or Formula (III) below:

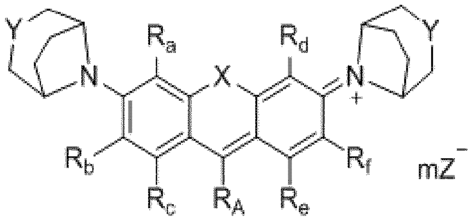

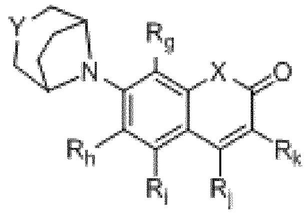

Formula (I) Formula (II)

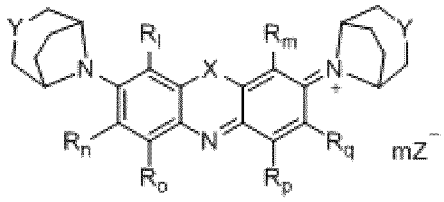

Formula (III)

$R_a$, $R_b$, $R_c$, $R_d$, $R_e$, $R_f$, $R_g$, $R_b$, $R_i$, $R_j$, $R_k$, $R_l$, $R_m$, $R_n$, $R_o$, $R_p$ and $R_q$ are independently selected from hydrogen, halogen, carboxyl, substituted or unsubstituted $C_1$-$C_{20}$ alkyl, substituted or unsubstituted $C_3$-$C_{20}$ cycloalkyl, substituted or unsubstituted $C_4$-$C_{20}$ cycloalkylalkyl, $C_4$-$C_{20}$ alkylcycloalkyl, substituted or unsubstituted $C_1$-$C_{20}$ alkoxyl, substituted or unsubstituted $C_1$-$C_{20}$ ester group, and substituted or unsubstituted $C_1$-$C_{20}$ acylamino;

$R_A$ is selected from substituted or unsubstituted $C_6$-$C_{40}$ aryl, substituted or unsubstituted $C_6$-$C_{40}$ aralkyl, and substituted or unsubstituted $C_5$-$C_{40}$ heteroaryl;

X is selected from oxygen, sulfur, substituted or unsubstituted $C_1$-$C_{20}$ alkylene,

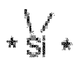,

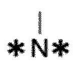, 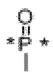, 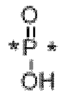, 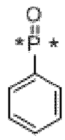, and 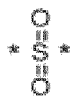;

Y is selected from oxygen, sulfur, nitrogen, substituted or unsubstituted $C_1$-$C_{20}$ alkylene, substituted or unsubstituted $C_1$-$C_{20}$ alkylenoxyl, substituted or unsubstituted $C_1$-$C_{20}$ acyl, substituted or unsubstituted $C_1$-$C_{20}$ ester group,

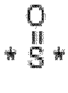, 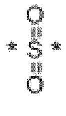, 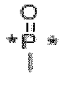, 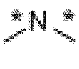, and 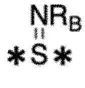,

where $R_B$ is selected from substituted or unsubstituted $C_1$-$C_{20}$ alkyl, substituted or unsubstituted $C_1$-$C_{20}$ alkoxyl, substituted or unsubstituted $C_1$-$C_{20}$ acyl, and substituted or unsubstituted $C_1$-$C_{20}$ ester group;

$Z^-$ is selected from $F^-$, $Cl^-$, Br, $I^-$, $OAc^-$, $HSO_4^-$, $H_2PO_4^-$, $ClO_4^-$, $CF_3COO^-$, $CF_3SO_3^-$, $CH_3SO_3^-$, $NO_3^-$, $C_5H_7O_5COO^-$, and $PF_6^-$; and m is an integer ranged from 1 to 3.

Further, the $R_a$, the $R_b$, the $R_c$, the $R_d$, the $R_e$, the $R_f$, the $R_g$, the $R_b$, the $R_i$, the $R_j$, the $R_k$, the $R_l$, the $R_m$, the $R_b$, the

$R_o$, the $R_p$ and the $R_q$ are independently selected from hydrogen, halogen, carboxyl, substituted or unsubstituted $C_1$-$C_{10}$ alkyl, substituted or unsubstituted $C_3$-$C_{10}$ cycloalkyl, substituted or unsubstituted $C_4$-$C_{10}$ cycloalkylalkyl, $C_4$-$C_{10}$ alkylcycloalkyl, substituted or unsubstituted $C_1$-$C_{10}$ alkoxyl, substituted or unsubstituted $C_1$-$C_{10}$ ester group, and substituted or unsubstituted $C_1$-$C_{10}$ acylamino; and/or, the $R_a$, is selected from substituted or unsubstituted $C_6$-$C_{30}$ aryl, substituted or unsubstituted $C_6$-$C_{30}$ aralkyl, and substituted or unsubstituted $C_5$-$C_{30}$ heteroaryl; and/or, the X is selected from oxygen, sulfur, substituted or unsubstituted $C_1$-$C_{10}$ alkyl,

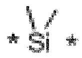 ,

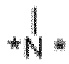 , 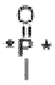 , 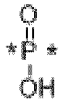 , 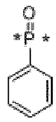 , and 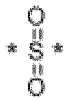 ;

and/or, the Y is selected from oxygen, sulfur, nitrogen, substituted or unsubstituted $C_1$-$C_{10}$ alkyl, substituted or unsubstituted $C_1$-$C_{10}$ alkoxyl, substituted or unsubstituted $C_1$-$C_{10}$ acyl, substituted or unsubstituted $C_1$-$C_{10}$ ester group,

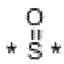 , 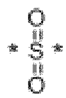 , 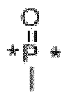 ,

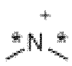 , and 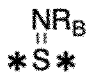 ,

where the $R_B$ is selected from substituted or unsubstituted $C_1$-$C_{20}$ alkyl, substituted or unsubstituted $C_1$-$C_{20}$ alkoxyl, substituted or unsubstituted $C_1$-$C_{20}$ acyl, and substituted or unsubstituted $C_1$-$C_{20}$ ester group; and/or, the $Z^-$ is selected from $F^-$, $Cl^-$, Br, $I^-$, $OAc^-$, $HSO_4^-$, $H_2PO_4^-$, $ClO_4^-$, $CF_3COO^-$, $CF_3SO_3^-$, $CH_3SO_3^-$, $NO_3^-$, $C_5H_7O_5COO^-$, and $PF_6^-$; and/or, the m is an integer ranged from 1 to 3.

Further, the aza[3.2.1]bridged ring substituted fluorescent dye shown in Formula (I) has a structure shown in Formula (IV) below:

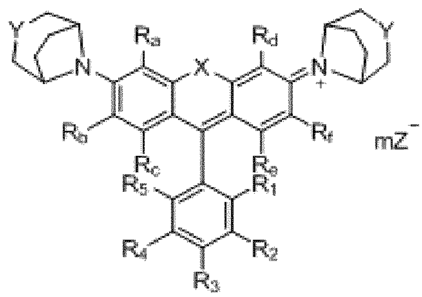

Formula (IV)

$R_1$ and $R_5$ are independently selected from hydrogen, carboxyl, halogen, substituted or unsubstituted $C_1$-$C_6$ alkyl, substituted or unsubstituted $C_1$-$C_6$ alkoxyl, nitro, amino, hydroxyl,

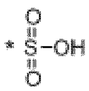 , 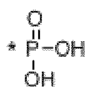 , 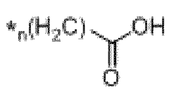 , 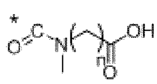 , 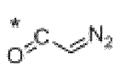 , 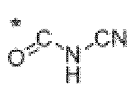 , 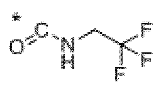 ,

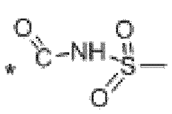 , 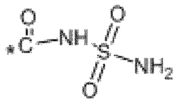 , 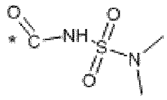 , 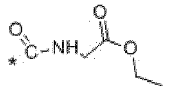 , and 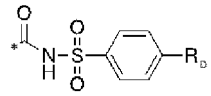 ,

where $R_D$ is selected from hydrogen, halogen, cyano, substituted or unsubstituted $C_1$-$C_6$ alkyl, and substituted or unsubstituted $C_1$-$C_6$ alkoxyl, and n is an integer ranged from 1 to 6; and $R_2$, $R_3$ and $R_4$ are independently selected from hydrogen, halogen, carboxyl, substituted or unsubstituted $C_1$-$C_{10}$ alkyl, substituted or unsubstituted $C_3$-$C_{10}$ cycloalkyl, substituted or unsubstituted $C_4$-$C_{10}$ cycloalkylalkyl, $C_4$-$C_{10}$ alkylcycloalkyl, substituted or unsubstituted $C_1$-$C_{10}$ alkoxyl, substituted or unsubstituted $C_1$-$C_{10}$ ester group,

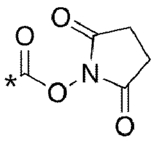 , 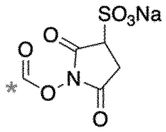 , 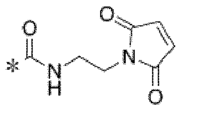 , 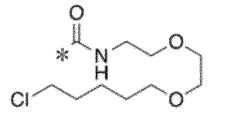 ,

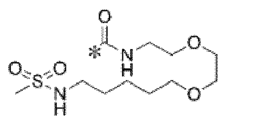 , 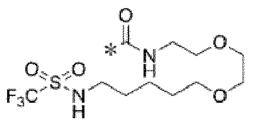 , 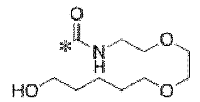 , 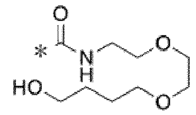 ,

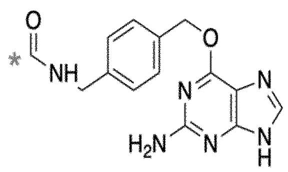 , 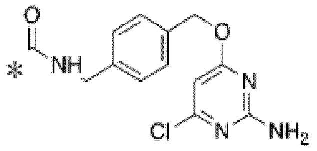 , 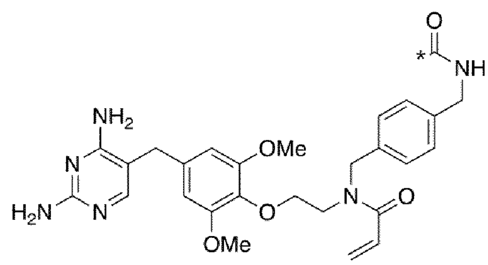 ,

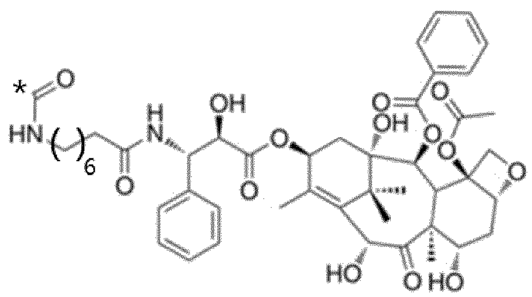 , 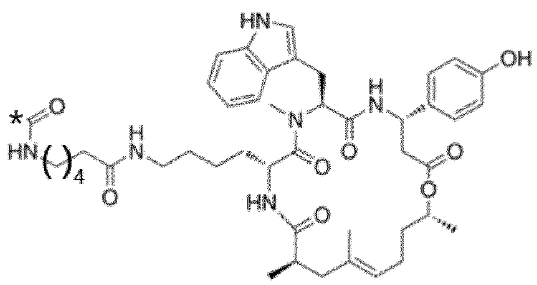 ,

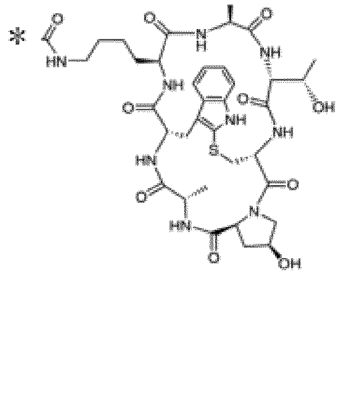 , 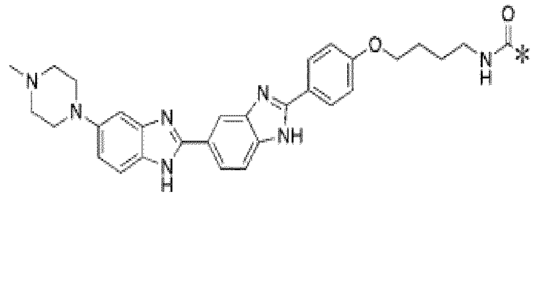 ,

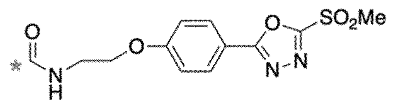,
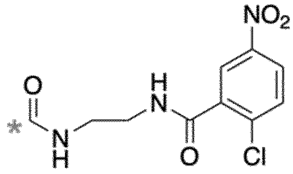,
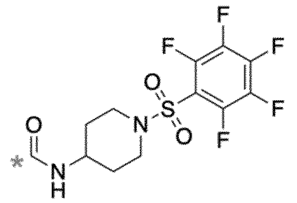,

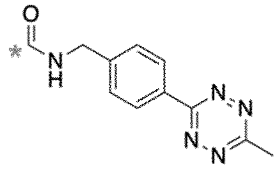,
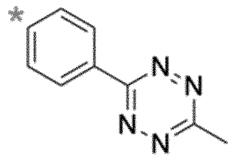

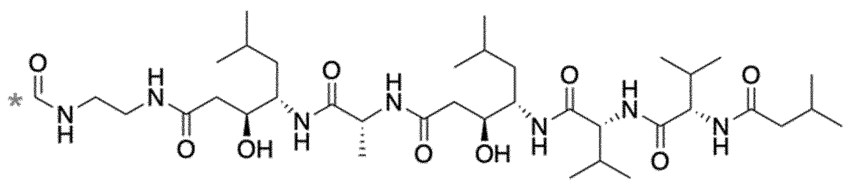

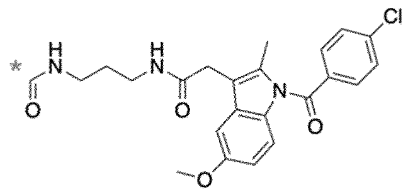,
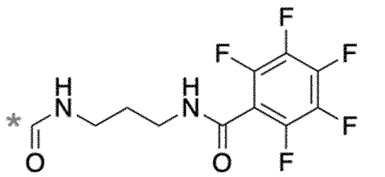,
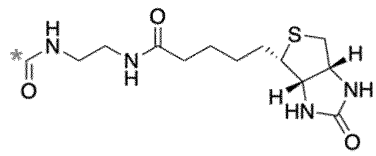,

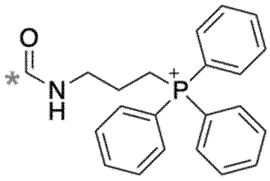,

or any other functional groups used for biological coupling or subcellular localization.

Further, the aza[3.2.1]bridged ring substituted fluorescent dye is selected from at least one of the following formulas IA1-IA3, IIA1-IIA3, IIIA1-IIIA3, IB1-IB3, IC1-IC3, ID1-ID3, IE1-IE3, IF1-IF3, and IG1-IG3:

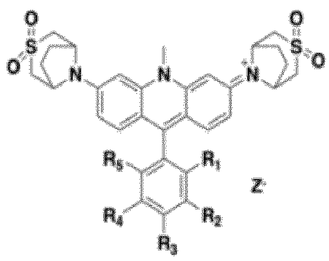

**IA1**

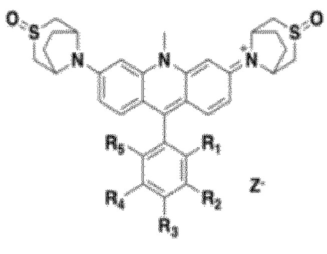

**IA2**

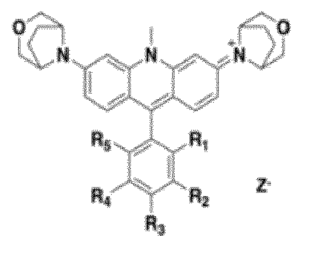

**IA3**

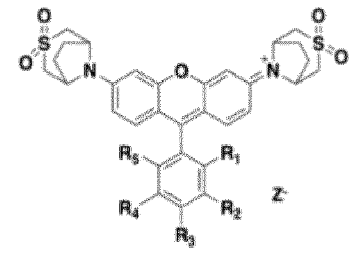

IB1

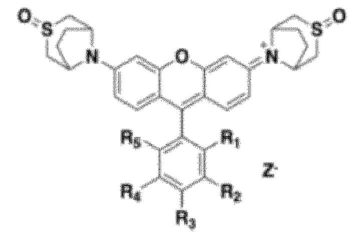

IB2

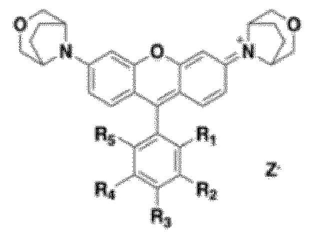

IB3

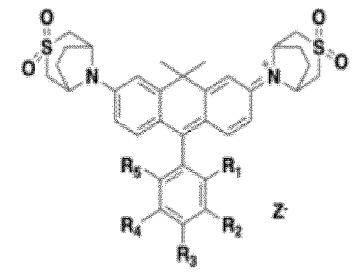

IC1

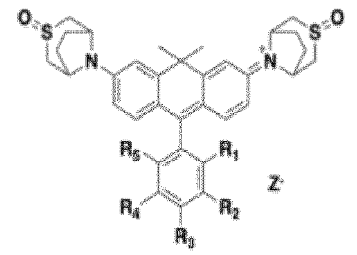

IC2

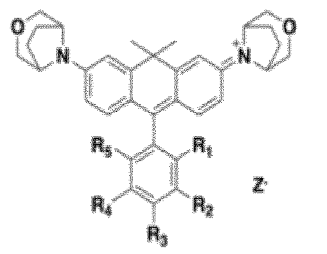

IC3

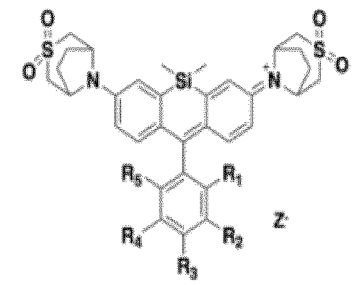

ID1

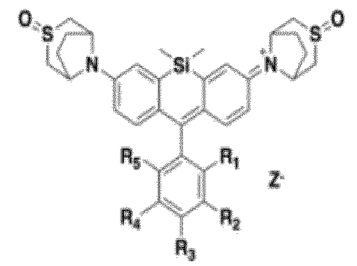

ID2

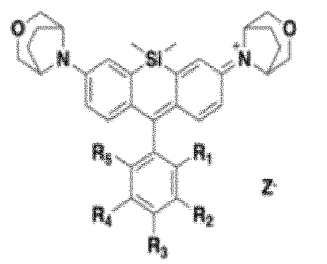

ID3

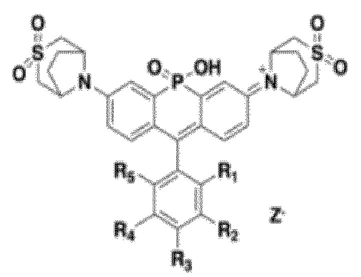

IE1

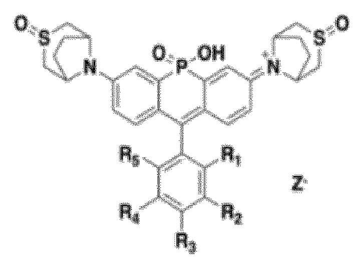

IE2

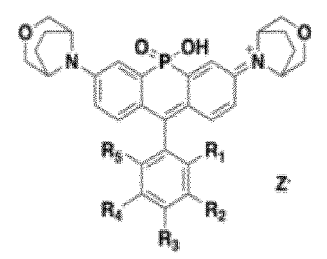

IE3

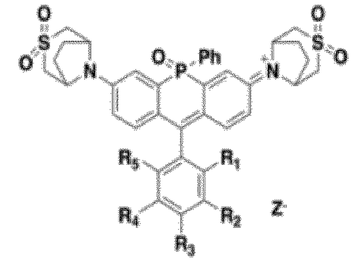

IF1

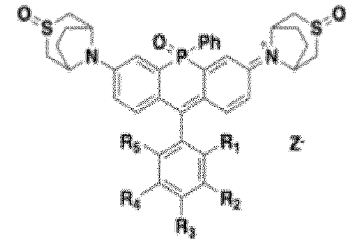

IF2

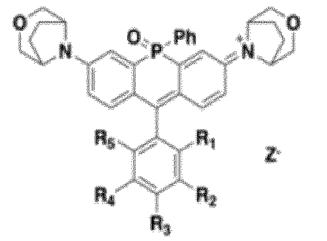

IF3

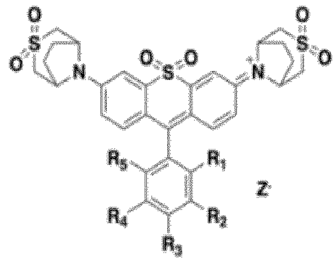

IG1

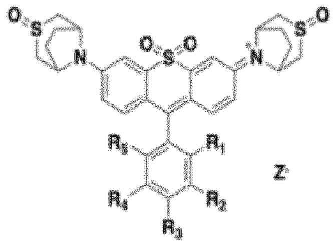

IG2

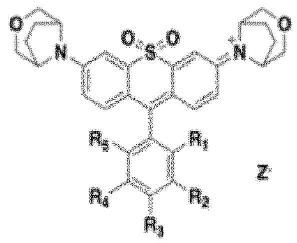

IG3

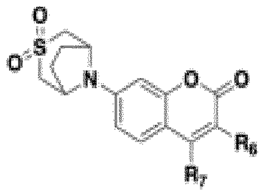

IIA1

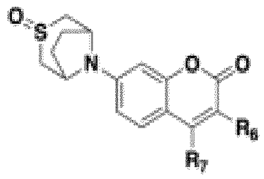

IIA2

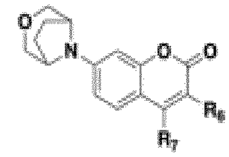

IIA3

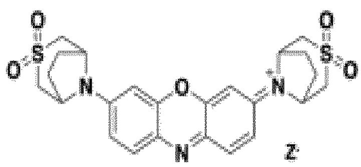

IIIA1

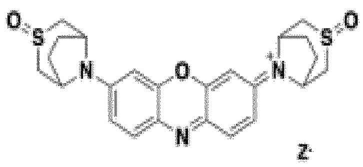

IIIA2

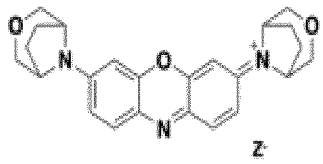

IIIA3

.

Further, the aza[3.2.1]bridged ring substituted fluorescent dye is selected from at least one of the following formulas A528, A532, A555, A586, A591, A620, A634, A640, A655, A720, A586-2Me, A586-2Me-5-COOH, A528-6-COOH, A532-6-COOH, A555-6-COOH, A586-6-COOH, A591-6-COOH, A620-6-COOH, A634-5-COOH, A640-6-COOH, A655-6-COOH, A528-HTL, A532-HTL, A555-HTL, A586-HTL, A620-HTL, A655-HTL, A555-STL, A528-NHS, A620-STL, A655-STL, A634-NHS, A586-NHS, A591-HTL, A640-HTL, A555-TMP3, A620-TMP3, A655-TMP3, A655-4-Hoechst, A591-TMP3, A640-TMP3, A591-STL, A640-STL, A528-NCN-HTL, A555-NCN-HTL, A620-NCN-HTL, B350, B356, C622, and C654, and Me in the following formulas represents methyl:

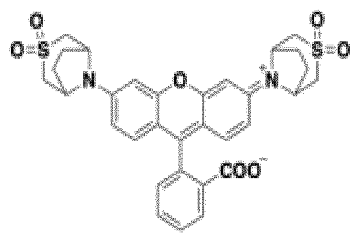

A528

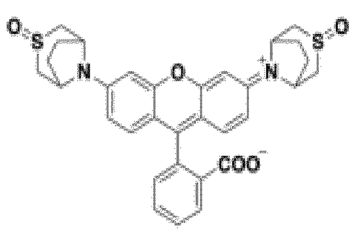

A532

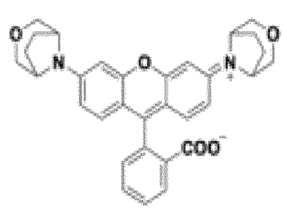

A555

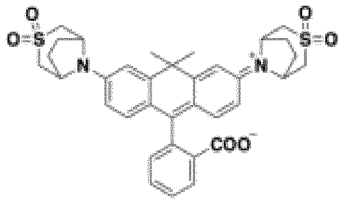

A586

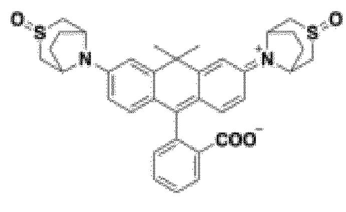

A591

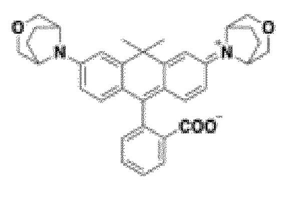

A620

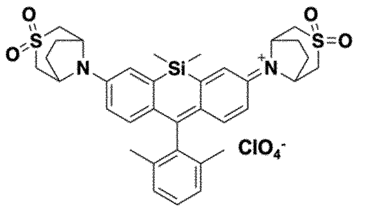

A634

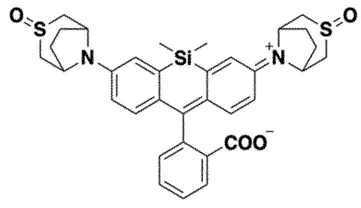

A640

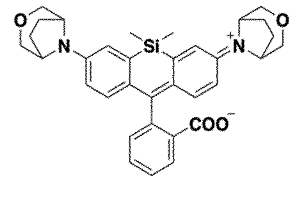

A655

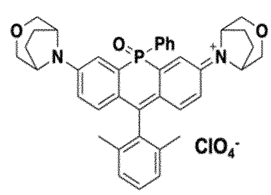

A720

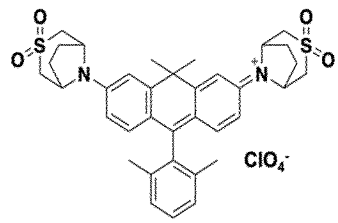

A586-2Me

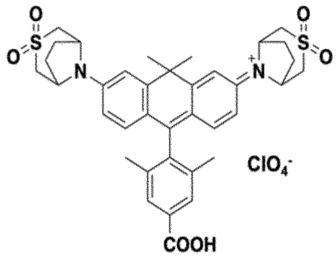

A586-2Me-5-COOH

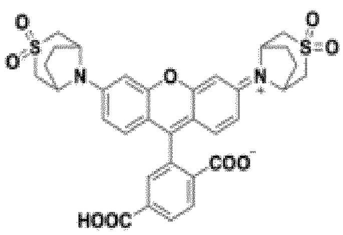

A528-6-COOH

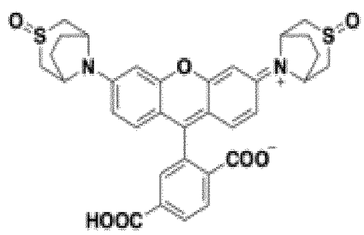

A532-6-COOH

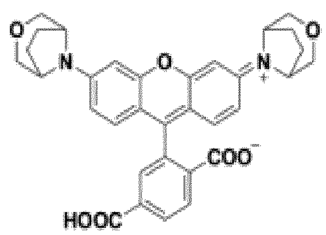

A555-6-COOH

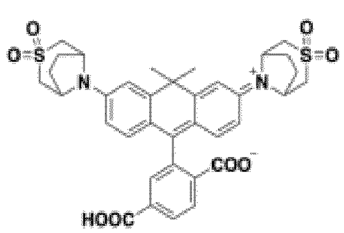

A586-6-COOH

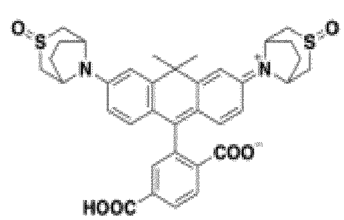

A591-6-COOH

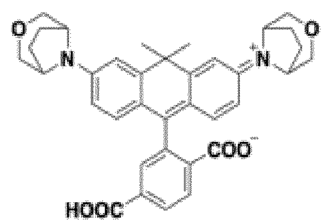

A620-6-COOH

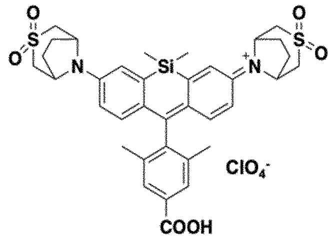

A634-5-COOH

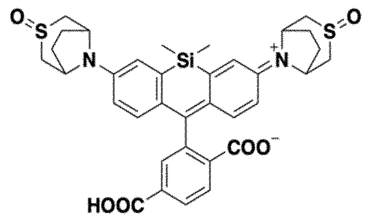

A640-6-COOH

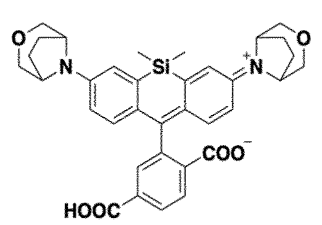

A655-6-COOH

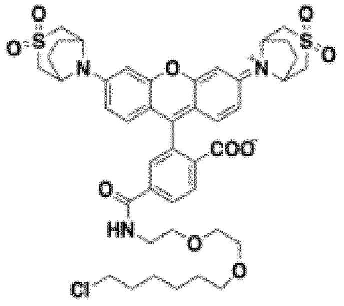

A528-HTL

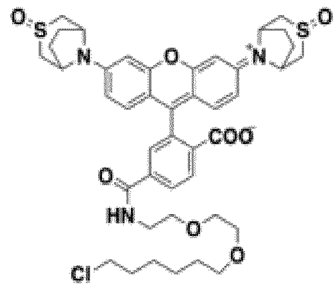

A532-HTL

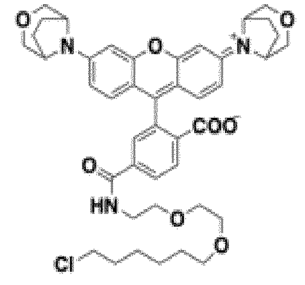

A555-HTL

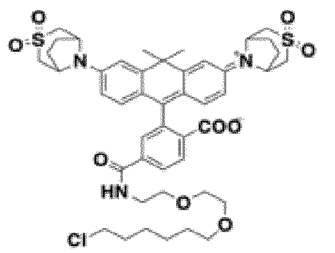

A586-HTL

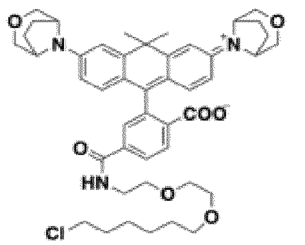

A620-HTL

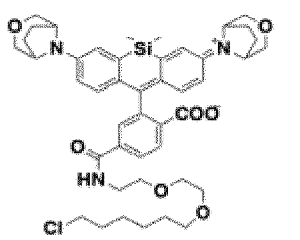

A655-HTL

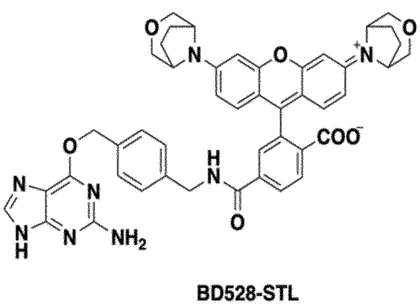

BD528-STL

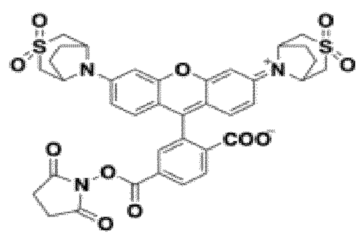

A528-NHS

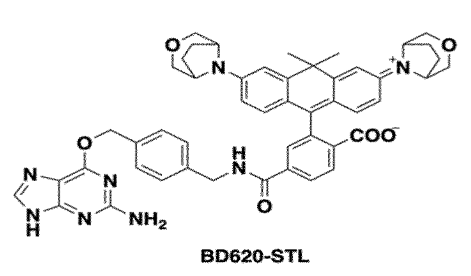

BD620-STL

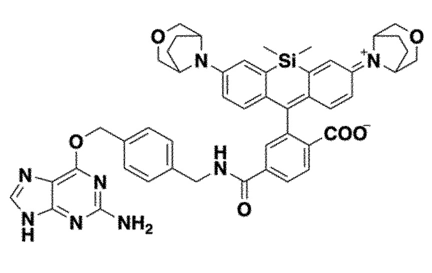

BD655-STL

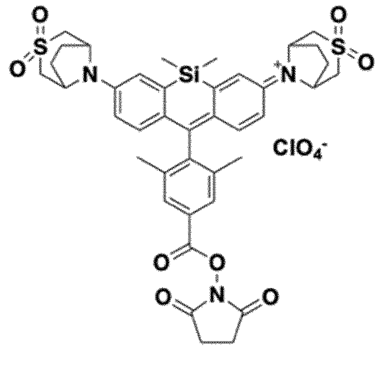

**A634-NHS**

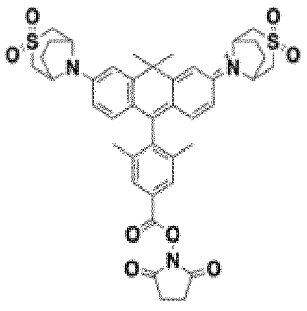

**A586-NHS**

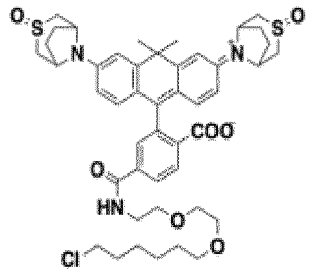

**A591-HTL**

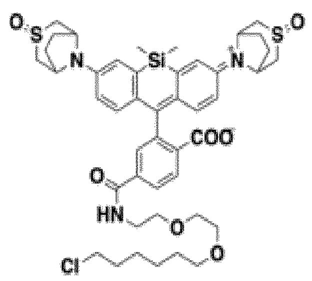

**A640-HTL**

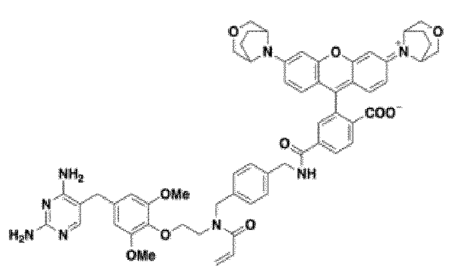

**A555-TMP3**

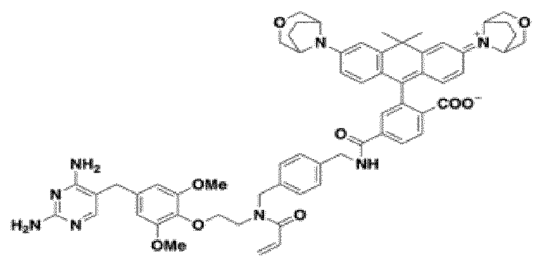

**A620-TMP3**

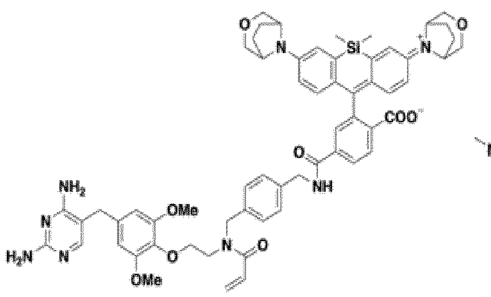

**A655-TMP3**

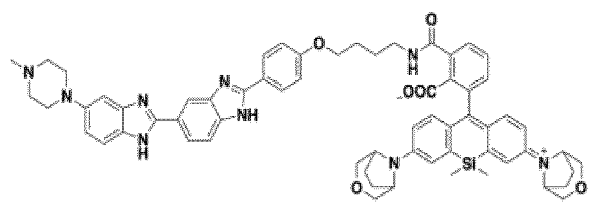

**A655-4-Hoechst**

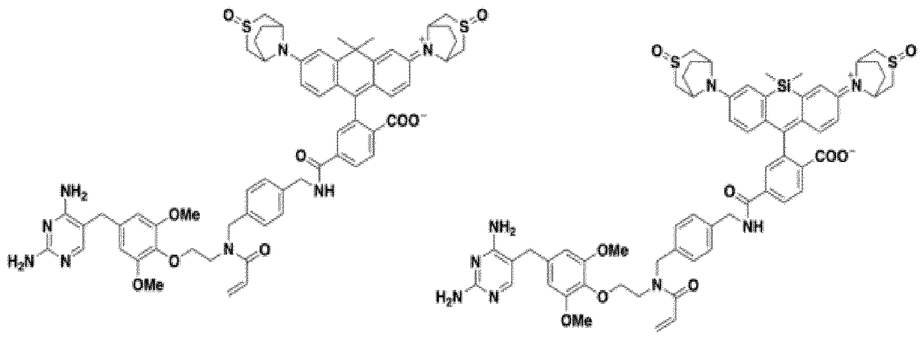

A591-TMP3

A640-TMP3

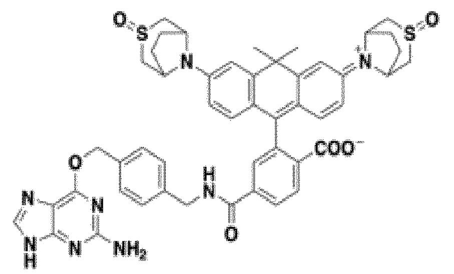

A591-STL

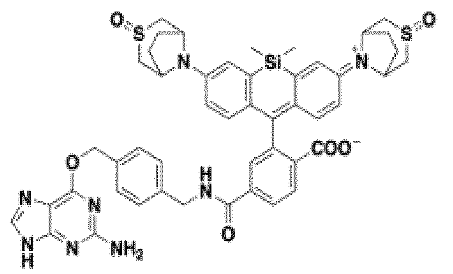

A640-STL

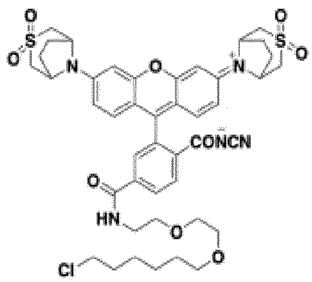

A528-NCN-HTL

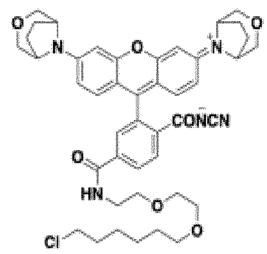

A555-NCN-HTL

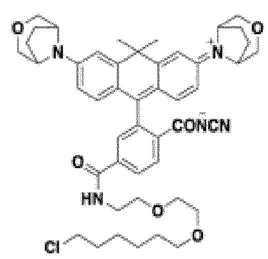

A620-NCN-HTL

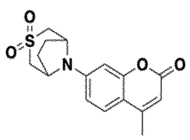

B350

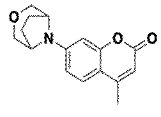

B356

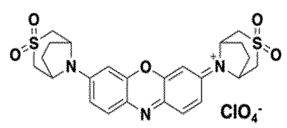

C622

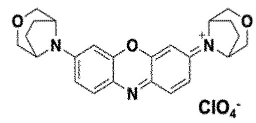

C654

In order to achieve the above purpose, a method for preparing an aza[3.2.1]bridged ring substituted fluorescent dye is provided according to one aspect of the present disclosure. The preparation method includes: step S11, mixing a compound A, an aza[3.2.1]bridged ring compound and a first catalyst in a first reaction solvent to carry out a reaction so as to obtain a compound B; and step S12, mixing the compound B with an HZ aqueous solution to carry out a reaction so as to obtain the aza[3.2.1]bridged ring substituted fluorescent dye shown in Formula (I). The compound A has a structure shown in Formula (V) below, and the aza[3.2.1]bridged ring compound has a structure shown in Formula (VI) below:

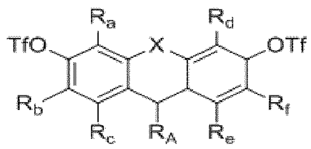 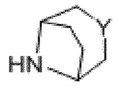

Formula (V) Formula (VI)

where $R_A$, $R_a$, $R_b$, $R_c$, $R_d$, $R_e$, $R_f$, X and Y independently have the same meaning as defined in the first aspect, $Z^-$ in the HZ aqueous solution has the same meaning as defined in the first aspect, and OTf represents a trifluoromethanesulfonate group.

Further, the first catalyst includes a palladium catalyst, a palladium catalyst ligand and an alkaline catalyst.

Further, the palladium catalyst includes at least one of tetrakis(triphenylphosphine)palladium, palladium acetate, tris(dibenzylideneacetone)dipalladium, [(diphenylphosphino)ferrocene]dichloropalladium, dichlorobis(triphenylphosphine)palladium and palladium chloride.

Further, the palladium catalyst includes at least one of tetrakis(triphenylphosphine)palladium, palladium acetate or tris(dibenzylideneacetone)dipalladium.

Further, the palladium catalyst ligand includes at least one of bis(dibenzylideneacetone)palladium, 2,2'-bis(diphenylphosphino)-1,1'-binaphthyl, 2-dicyclohexylphosphino-2',4',6'-triisopropylbiphenyl and 2-dicyclohexylphosphino-2',6'-dimethoxybiphenyl.

Further, the palladium catalyst ligand includes at least one of bis(dibenzylideneacetone)palladium and 2,2'-bis(diphenylphosphino)-1,1'-binaphthyl.

Further, the alkaline catalyst includes at least one of cesium carbonate, sodium tert-butoxide, potassium carbonate, sodium carbonate, sodium methoxide, sodium ethoxide and potassium tert-butoxide.

Further, the alkaline catalyst includes at least one of cesium carbonate, sodium tert-butoxide and potassium tert-butoxide.

Further, the first reaction solvent includes at least one of n-hexane, tetrahydrofuran, 1,4-dioxane, ethyl ether and toluene.

In order to achieve the above purpose, a method for preparing an aza[3.2.1]bridged ring substituted fluorescent dye is provided according to one aspect of the present disclosure. The preparation method includes: mixing a compound C, an aza[3.2.1]bridged ring compound and a second catalyst in a second reaction solvent to carry out a reaction so as to obtain the aza[3.2.1]bridged ring substituted fluorescent dye shown in Formula (II).

The compound C has a structure shown in Formula (VII) below:

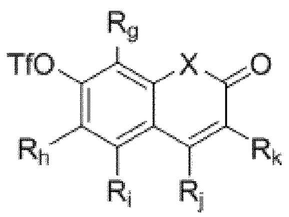

Formula (VII)

where $R_g$, $R_b$, $R_j$, $R_j$, $R_k$ and X independently have the same meaning as defined in the first aspect.

Further, the second catalyst has the same meaning as the first catalyst.

Further, the second reaction solvent has the same meaning as the first reaction solvent.

In order to achieve the above purpose, a method for preparing an aza[3.2.1]bridged ring substituted fluorescent dye is provided according to one aspect of the present disclosure. The preparation method includes: step S31, mixing a compound D, an aza[3.2.1]bridged ring compound and a third catalyst in a third reaction solvent to carry out a reaction so as to obtain a compound E; step S32, mixing the compound E with an oxidizing agent in a fourth reaction solvent to carry out a reaction so as to obtain a compound F; and step S33, mixing the compound F with an HZ aqueous solution to carry out a reaction so as to obtain the aza[3.2.1]bridged ring substituted fluorescent dye shown in Formula (III).

The compound D has a structure shown in Formula (VIII) below:

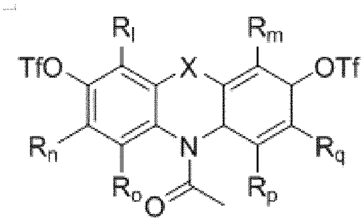

Formula (VIII)

where $R_l$, $R_m$, $R_n$, $R_o$, $R_p$, $R_q$, X and Y independently have the same meaning as defined in the first aspect, and $Z^-$ in the HZ aqueous solution has the same meaning as defined in the first aspect.

Further, the third catalyst has the same meaning as the first catalyst.

Further, the third reaction solvent has the same meaning as the first reaction solvent.

Further, in step S32, the fourth reaction solvent is a mixed solvent of dichloromethane and water, and the volume ratio of the dichloromethane to the water is (10-30):1.

Further, in step S32, the oxidizing agent includes at least one of 2,3-dichloro-5,6-dicyano-1,4-benzoquinone, potassium permanganate, hydrogen peroxide and m-chloroperoxybenzoic acid.

Further, the oxidizing agent is 2,3-dichloro-5,6-dicyano-1,4-benzoquinone.

Further, the molar ratio of the oxidizing agent to the compound E is (1.0-2.0):1.

In order to achieve the above purpose, use of the aza[3.2.1]bridged ring substituted fluorescent dye or the aza [3.2.1]bridged ring substituted fluorescent dye prepared by the method for preparing an aza[3.2.1]bridged ring substituted fluorescent dye in the fields of fluorescence analysis, fluorescence detection and fluorescence imaging as a fluorescent marker is provided according to one aspect of the present disclosure.

Further, use of the aza[3.2.1]bridged ring substituted fluorescent dye in fluorescence analysis, fluorescence detection, biomolecular labeling, immunofluorescence imaging, live-cell imaging, tissue imaging, in vivo imaging, single-molecule imaging and super-resolution fluorescence imaging as a fluorescent marker is provided.

According to use of the aza[3.2.1]bridged ring substituted fluorescent dye provided by the present disclosure in technical schemes of the present disclosure, by adopting an aza[3.2.1]bridged ring as a substituent of the fluorescent dye, not only are the brightness and photostability of the fluorescent dye obviously improved, but also the phenomenon of spectral blue shift caused by irradiation of the dye for a long time is completely avoided. Meanwhile, the water solubility of the fluorescent dye can be effectively improved, and good membrane permeability of the fluorescent dye is maintained.

## Brief Description of the Drawings

Drawings attached to the specification that form a part of the present disclosure are used to provide further understanding of the present disclosure, and schematic embodiments of the present disclosure and description thereof are used to interpret the present disclosure and are not intended to constitute improper limitations to the present disclosure.

Fig.1 shows absorption spectra of aza[3.2.1]bridged ring substituted fluorescent dyes according to Examples 1-4, 6, 7, 9, 18, 20 and 21 of the present disclosure;

Fig. 2 shows emission spectra of the aza[3.2.1]bridged ring substituted fluorescent dyes according to Examples 1-4, 6, 7, 9, 18, 20 and 21 of the present disclosure;

Fig. 3 shows comparison curves of differences of the photostability in orange channel according to Examples 3 and 10 as well as Comparative Examples 1-4 of the present disclosure;

Fig. 4 shows comparison curves of differences of the photostability in a far-red channel according to Example 7 and Comparative Example 5 of the present disclosure;

Fig. 5 shows a single molecule imaging photo of fixed U2OS cells according to Example 27 of the present disclosure;

Fig. 6 shows comparison of the single molecule brightness of fixed U2OS cells according to Example 27 and Comparative Examples 6 and 7 of the present disclosure;

Fig. 7 shows comparison of the photostability of fixed U2OS cells according to Example 27 and Comparative

Examples 6 and 7 of the present disclosure;

Fig. 8 shows a confocal fluorescence imaging photo of labeled TMP-Halo-SNAP-GFP-H2B proteins in living Hela cells according to Example 27 of the present disclosure;

Fig. 9 shows a confocal fluorescence imaging photo of labeled TMP-Halo-SNAP-GFP-H2B proteins in living Hela cells according to Example 31 of the present disclosure;

Fig. 10 shows a confocal fluorescence imaging photo of labeled TMP-Halo-SNAP-GFP-H2B proteins in living Hela cells according to Example 34 of the present disclosure;

Fig. 11 shows comparison of the relative fluorescence intensity of labeled TMP-Halo-SNAP-GFP-H2B proteins in living Hela cells according to Example 27 and Comparative Examples 6 and 8 of the present disclosure;

Fig. 12 shows comparison of the relative fluorescence intensity of labeled TMP-Halo-SNAP-GFP-H2B proteins in living Hela cells according to Example 31 and Comparative Example 9 of the present disclosure;

Fig. 13 shows a confocal fluorescence imaging photo of labeled TMP-Halo-SNAP-GFP-H2B proteins in living Hela cells according to Example 28 of the present disclosure;

Fig. 14 shows comparison of the relative fluorescence intensity of labeled TMP-Halo-SNAP-GFP-H2B proteins in living Hela cells according to Example 28 and Comparative Example 10 of the present disclosure;

Fig. 15 shows a wash-free super-resolution fluorescence imaging photo of labeled Lifeact-Halo proteins in living Hela cells under an STED microscope according to Example 34 of the present disclosure;

Fig. 16 shows comparison of the photostability of labeled Lifeact-Halo proteins in living Hela cells under super-resolution fluorescence imaging conditions of an STED microscope according to Example 34 and Comparative Example 11 of the present disclosure.

## Detailed Description of the Embodiments

Notably, embodiments in the present disclosure and features in the embodiments may be combined with each other without conflict. The present disclosure is described in detail below with reference to the attached drawings and in conjunction with the embodiments.

As described in the background of the present disclosure, by introducing an azacyclobutane group into a matrix of a fluorescent dye in the prior art, the brightness of the fluorescent dye can be improved, but the fluorescent dye has poor photostability and no resistance to photooxidation dealkylation (spectral blue shift). In addition, by adopting a method of introducing azabicyclo[2.2.1]heptane into a matrix of a fluorescent dye to substitute rhodamine, the fluorescence quantum yield, photostability and resistance to photooxidation dealkylation (spectral blue shift) of the dye are improved, but the dye has poor water solubility and membrane permeability, so that use of the dye in living cell imaging is hindered. By introducing a sulfonic acid group into a matrix of a fluorescent dye, the water solubility of the dye can be improved, the antibody labeling efficiency is improved, and non-specific dyeing is reduced, but the dye is also prevented from permeating a cell membrane, so that the sulfonated dye cannot be used in living cells. In order to solve the problems, the present disclosure provides an aza[3.2.1]bridged ring substituted fluorescent dye and a preparation method and use thereof.

In a first typical embodiment of the present disclosure, an aza[3.2.1]bridged ring substituted fluorescent dye is provided. The aza[3.2.1]bridged ring substituted fluorescent dye has a structure shown in Formula (I), Formula (II) or Formula (III) below:

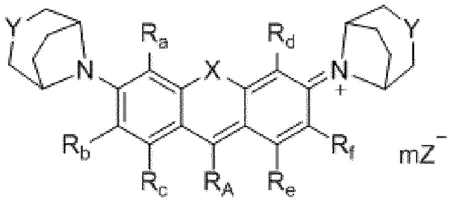

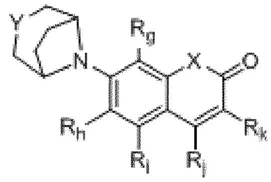

Formula (I)

Formula (II)

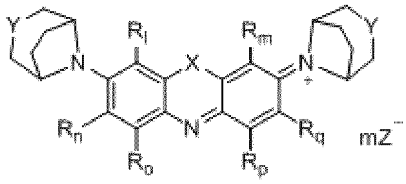

Formula (III)

$R_a$, $R_b$, $R_c$, $R_d$, $R_e$, $R_f$, $R_g$, $R_b$, $R_i$, $R_j$, $R_k$, $R_l$, $R_m$, $R_n$, $R_o$, $R_p$ and $R_q$ are independently selected from hydrogen, halogen, carboxyl, substituted or unsubstituted $C_1$-$C_{20}$ alkyl, substituted or unsubstituted $C_3$-$C_{20}$ cycloalkyl, substituted or unsubstituted $C_4$-$C_{20}$ cycloalkylalkyl, $C_4$-$C_{20}$ alkylcycloalkyl, substituted or unsubstituted $C_1$-$C_{20}$ alkoxyl, substituted or unsubstituted $C_1$-$C_{20}$ ester group, and substituted or unsubstituted $C_1$-$C_{20}$ acylamino;

$R_A$ is selected from substituted or unsubstituted $C_6$-$C_{40}$ aryl, substituted or unsubstituted $C_6$-$C_{40}$ aralkyl, and substituted or unsubstituted $C_5$-$C_{40}$ heteroaryl;

X is selected from oxygen, sulfur, substituted or unsubstituted $C_1$-$C_{20}$ alkylene,

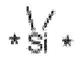 , 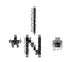 ,

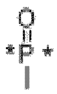 , 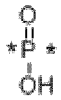 , 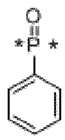 , and 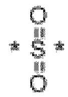 ;

Y is selected from oxygen, sulfur, nitrogen, substituted or unsubstituted $C_1$-$C_{20}$ alkylene, substituted or unsubstituted $C_1$-$C_{20}$ alkylenoxyl, substituted or unsubstituted $C_1$-$C_{20}$ acyl, substituted or unsubstituted $C_1$-$C_{20}$ ester group,

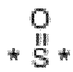 , 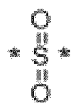 , 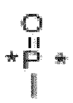 , 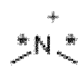 , and 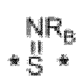 ,

where $R_B$ is selected from substituted or unsubstituted $C_1$-$C_{20}$ alkyl, substituted or unsubstituted $C_1$-$C_{20}$ alkoxyl, substituted or unsubstituted $C_1$-$C_{20}$ acyl, and substituted or unsubstituted $C_1$-$C_{20}$ ester group;

$Z^-$ is selected from $F^-$, $Cl^-$, Br, $I^-$, $OAc^-$, $HSO_4^-$, $H_2PO_4^-$, $ClO_4^-$, $CF_3COO^-$, $CF_3SO_3^-$, $CH_3SO_3^-$, $NO_3^-$, $C_5H_7O_5COO^-$, and $PF_6^-$;

and in the Formula (I), the $Z^-$ may exist in an ionic form and may also be connected with the $R_A$ through a chemical bond, the $Z^-$ may exist in the two forms simultaneously when m is equal to or greater than 2, and the m is an integer

ranged from 1 to 3.

In the present disclosure, the "aryl" refers to a monocyclic ring or fused polycyclic ring derived from an aromatic hydrocarbon, and includes phenyl, biphenyl, bitriphenyl, naphthyl, binaphthyl, phenylnaphthyl, naphthylphenyl, fluorenyl, phenylfluorenyl, benzofluorenyl, dibenzofluorenyl, phenanthryl, phenylphenanthryl, anthryl, indenyl, bitriphenylenyl, pyrenyl, fused tetraphenyl, perylo, boryl, naphthonaphthyl, allenylfluorenyl and the like.

The "heteroaryl" refers to heteroaryl, and the heteroaryl refers to aryl containing at least one heteroatom selected from the group consisting of N, O and S as a main chain atom of a ring, which may be a monocyclic ring, such as furyl, thienyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazyl, pyridyl, pyrazinyl, pyrimidyl or pyridazinyl, or may be a fused ring condensed with at least one benzene ring, such as benzofuryl, benzothienyl, isobenzofuryl, dibenzofuryl, dibenzothienyl, benzoimidazolyl, benzothiazolyl, benzoisothiazolyl, benzoisoxazolyl, quinolyl, isoquinolyl, cinolinyl, quinazoinyl, quinoxalinyl, carbazolyl, phenoxazolyl, phenanthridinyl, benzodiacenaphthenyl or dihydroacridinyl.

The "substituted" in the "substituted or unsubstituted" indicates that a hydrogen atom in a functional group is substituted with another atom or group (namely, substituent). The substituent is independently select from at least one of the following groups: deuterium, halogen, $C_1$-$C_{10}$ alkyl, $C_1$-$C_{10}$ alkoxyl, $C_6$-$C_{10}$ aryl, $C_5$-$C_{10}$ heteroaryl, $C_5$-$C_{10}$ heteroaryl substituted with $C_6$-$C_{10}$ aryl, imidazolyl, benzoimidazolyl, $C_3$-$C_{10}$ cycloalkyl, $C_5$-$C_7$ heterocycloalkyl, tri-($C_1$-$C_{10}$) alkylsilyl, tri-($C_1$-$C_{10}$) arylsilyl, di-($C_1$-$C_{10}$) alkyl-($C_6$-$C_{10}$) arylsilyl, $C_1$-$C_{10}$ alkyl-di-($C_6$-$C_{10}$) arylsilyl, $C_2$-$C_{10}$ alkenyl, $C_2$-$C_{30}$ alkynyl, cyano, di-($C_1$-$C_{30}$) alkylamino, di-($C_6$-$C_{30}$) arylboryl, di-($C_1$-$C_{30}$) alkylboryl, $C_1$-$C_{30}$ alkyl, $C_6$-$C_{10}$ aryl$C_1$-$C_{10}$ alkyl, $C_1$-$C_{10}$ alkyl $C_6$-$C_{10}$ aryl, carboxyl, nitro, hydroxyl,

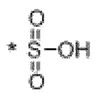, 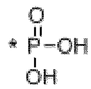,

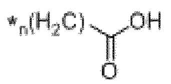, 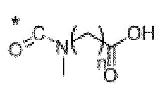, 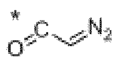, 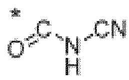, 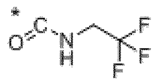, 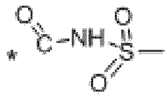,

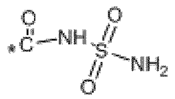, 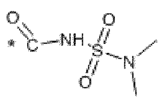, 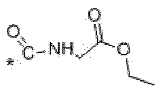, 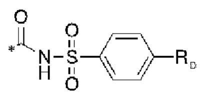, 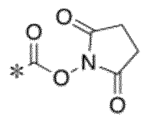, 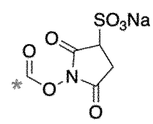,

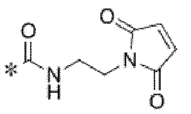, 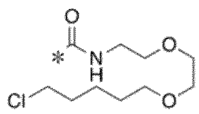, 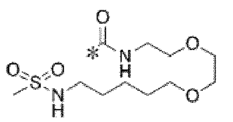, 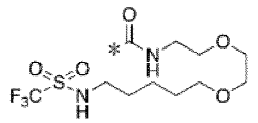,

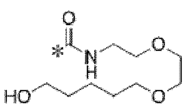, 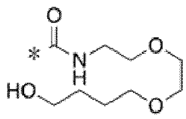, 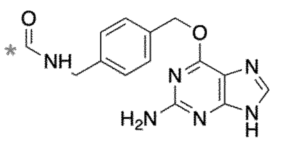, 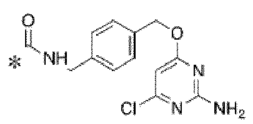,

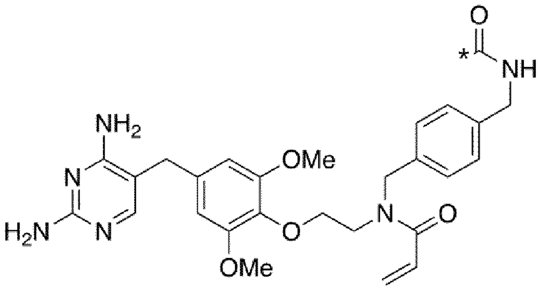 ,

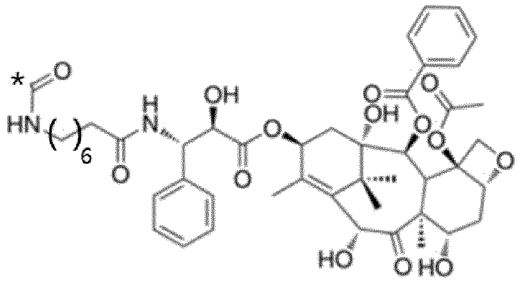 ,

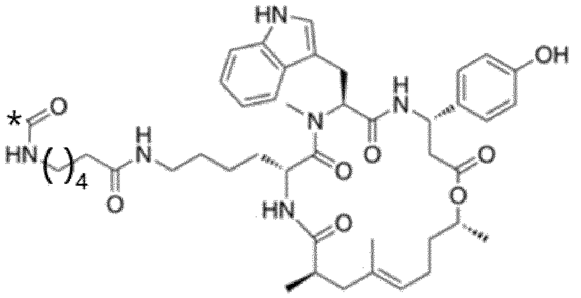 ,

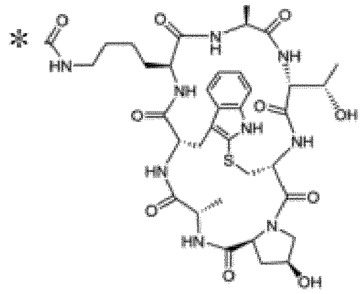 ,

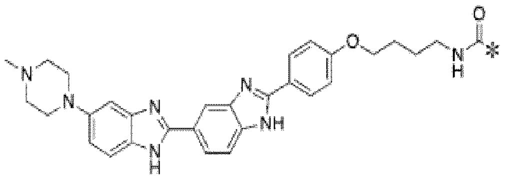 ,

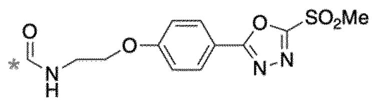 ,

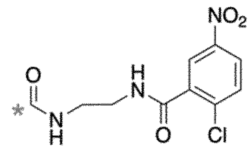 ,

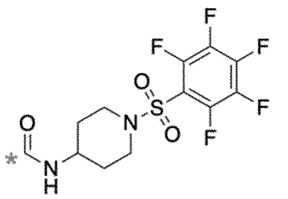 ,

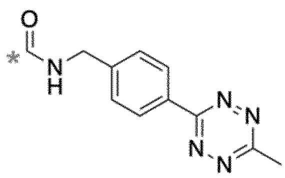 ,

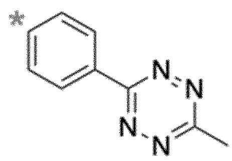 ,

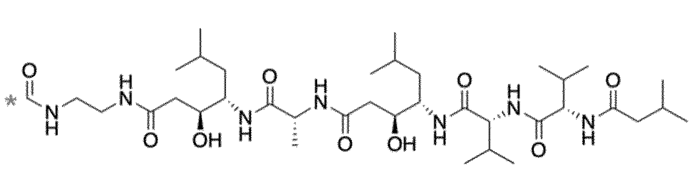 ,

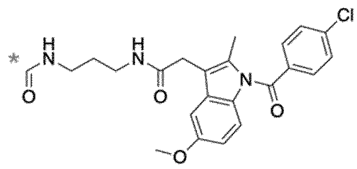 ,

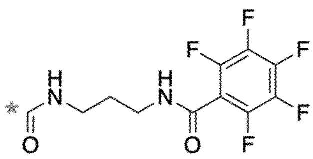 ,

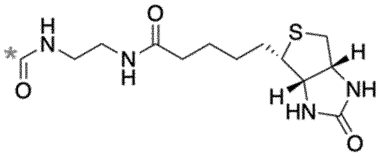 ,

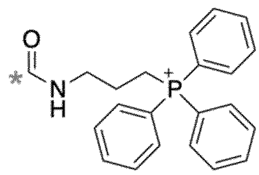 ,

or any other functional groups used for biological coupling or subcellular localization, where $R_D$ is selected from hydrogen, halogen, cyano, substituted or unsubstituted $C_1$-$C_4$ alkyl, and substituted or unsubstituted $C_1$-$C_6$ alkoxyl, and n is a positive integer ranged from 1 to 6. In addition, the number of carbon atoms described herein may be ranged from a lower limit to an upper limit. For example, $C_6$-$C_{10}$ indicates that the number of carbon atoms may be 6, 7, 8, 9 or 10.

In the present disclosure, "*" represents a connecting bond.

According to use of the aza[3.2.1]bridged ring substituted fluorescent dye provided by the present disclosure in technical schemes of the present disclosure, by adopting an aza[3.2.1]bridged ring as a substituent of the fluorescent dye, not only are the brightness and photostability of the fluorescent dye obviously improved, but also the phenomenon of spectral blue shift caused by irradiation of the dye for a long time is completely avoided. Meanwhile, the water solubility of the fluorescent dye can be effectively improved, and good membrane permeability of the fluorescent dye is maintained.

In order to further improve properties of the aza[3.2.1]bridged ring substituted fluorescent dye, preferably, the $R_a$, the $R_b$, the $R_c$, the $R_d$, the $R_e$, the $R_f$, the $R_g$, the $R_h$, the $R_i$, the $R_j$, the $R_k$, the $R_l$, the $R_m$, the $R_n$, the $R_o$, the $R_p$ and the $R_q$ are independently selected from hydrogen, halogen, carboxyl, substituted or unsubstituted $C_1$-$C_{10}$ alkyl, substituted or unsubstituted $C_3$-$C_{10}$ cycloalkyl, substituted or unsubstituted $C_4$-$C_{10}$ cycloalkylalkyl, $C_4$-$C_{10}$ alkylcycloalkyl, substituted or unsubstituted $C_1$-$C_{10}$ alkoxyl, substituted or unsubstituted $C_1$-$C_{10}$ ester group, and substituted or unsubstituted $C_1$-$C_{10}$ acylamino.

In some embodiments, preferably, the $R_A$ is selected from substituted or unsubstituted $C_6$-$C_{30}$ aryl, substituted or unsubstituted $C_6$-$C_{30}$ aralkyl, and substituted or unsubstituted $C_5$-$C_{30}$ heteroaryl.

In other embodiments, preferably, the X is selected from oxygen, sulfur, substituted or unsubstituted $C_1$-$C_{10}$ alkyl,

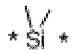, 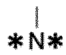, 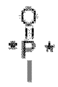, 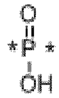, 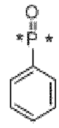, and 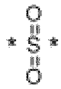;

preferably, the Y is selected from oxygen, sulfur, nitrogen, substituted or unsubstituted $C_1$-$C_{10}$ alkyl, substituted or unsubstituted $C_1$-$C_{10}$ alkoxyl, substituted or unsubstituted $C_1$-$C_{10}$ acyl, substituted or unsubstituted $C_1$-$C_{10}$ ester group,

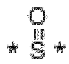, 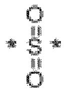, 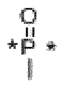, 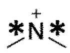, and 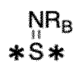,

where the $R_B$ is selected from substituted or unsubstituted $C_1$-$C_{20}$ alkyl, substituted or unsubstituted $C_1$-$C_{20}$ alkoxyl, substituted or unsubstituted $C_1$-$C_{20}$ acyl, and substituted or unsubstituted $C_1$-$C_{20}$ ester group.

In some other embodiments, preferably, the Z- is selected from $F^-$, $Cl^-$, Br, $I^-$, $OAc^-$, $HSO_4^-$, $H_2PO_4^-$, $ClO_4^-$, $CF_3COO^-$, $CF_3SO_3^-$, $CH_3SO_3^-$, $NO_3^-$, $C_5H_7O_5COO^-$, and $PF_6^-$; m is a positive integer ranged from 1 to 3; and $OAc^-$ represents an acetate ion.

In order to further improve properties of the aza[3.2.1]bridged ring substituted fluorescent dye, preferably, the aza [3.2.1]bridged ring substituted fluorescent dye has a structure shown in Formula (IV) below:

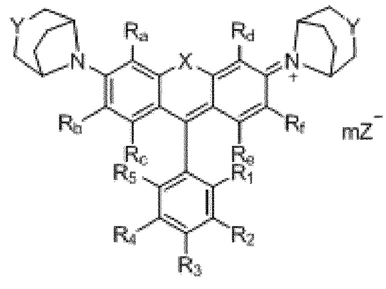

Formula (IV)

$R_1$ and $R_5$ are independently selected from hydrogen, carboxyl, halogen, substituted or unsubstituted $C_1$-$C_6$ alkyl, substituted or unsubstituted $C_1$-$C_6$ alkoxyl, nitro, amino, hydroxyl,

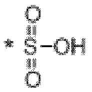, 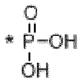, 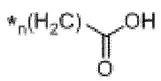, 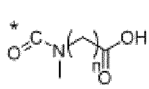, 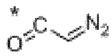, 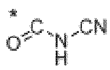, 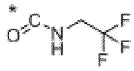, 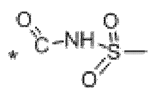, 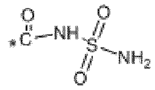, 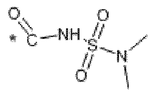, 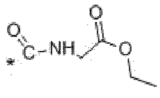, and 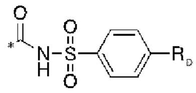, where $R_D$ is selected from hydrogen, halogen, cyano, substituted or unsubstituted $C_1$-$C_4$ alkyl, and substituted or unsubstituted $C_1$-$C_6$ alkoxyl, and n is an integer ranged from 1 to 6.

$R_2$, $R_3$ and $R_4$ are independently selected from hydrogen, halogen, carboxyl, substituted or unsubstituted $C_1$-$C_{10}$ alkyl, substituted or unsubstituted $C_3$-$C_{10}$ cycloalkyl, substituted or unsubstituted $C_4$-$C_{10}$ cycloalkylalkyl, $C_4$-$C_{10}$ alkylcycloalkyl, substituted or unsubstituted $C_1$-$C_{10}$ alkoxyl, substituted or unsubstituted $C_1$-$C_{10}$ ester group,

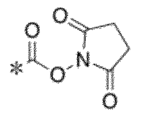, 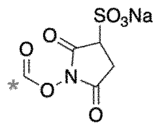,

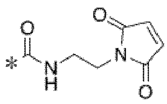, 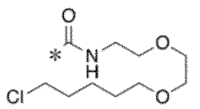, 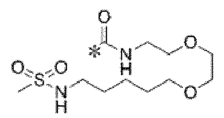, 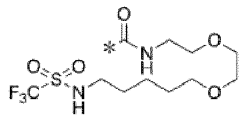,

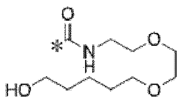, 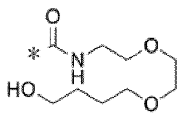, 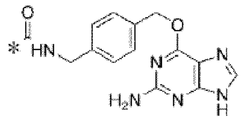, 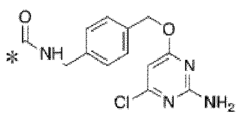,

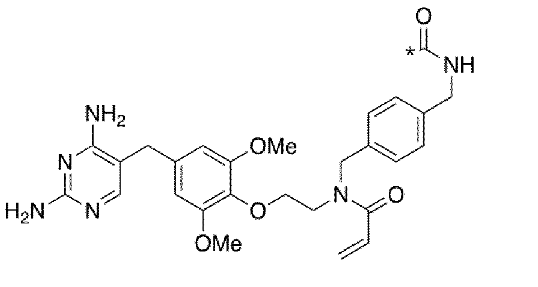, 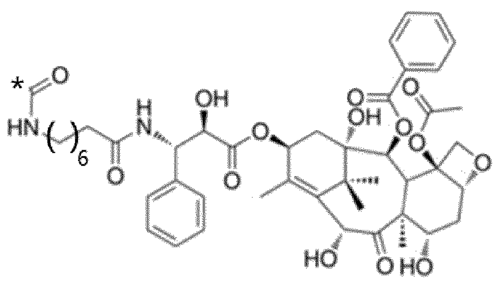,

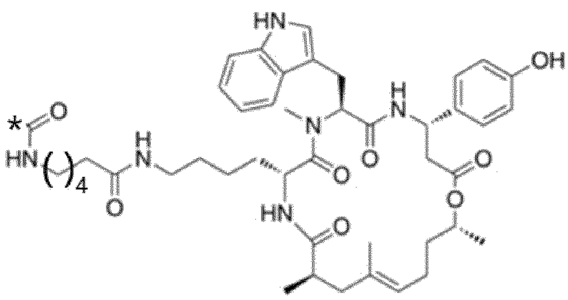, 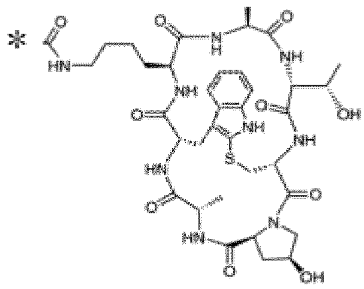,

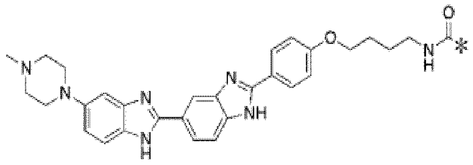,

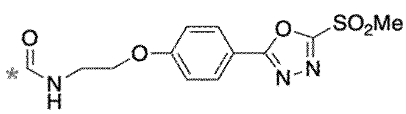,

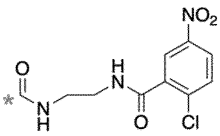,

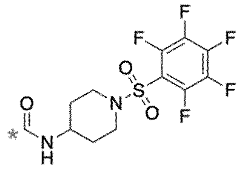,

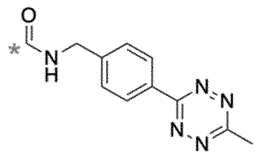,

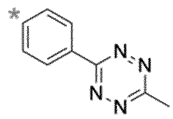,

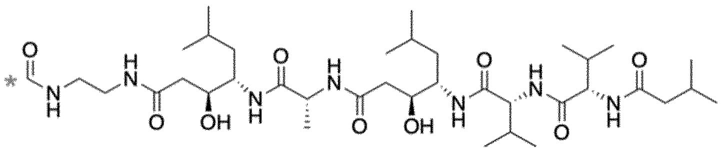,

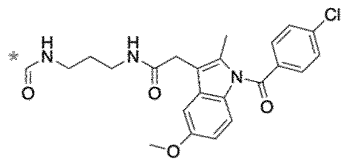,

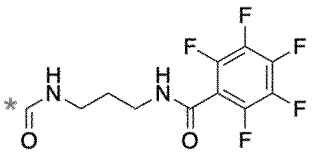,

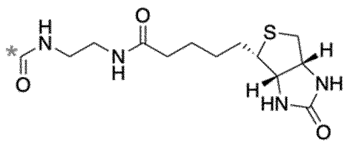,

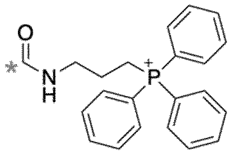, or any other functional groups used for biological coupling or subcellular localization.

In some specific embodiments of the present disclosure, the aza[3.2.1]bridged ring substituted fluorescent dye is selected from at least one of the following formulas IA1-IA3, IIA1-IIA3, IIIA1-IIIA3, IB1-IB3, IC1-IC3, ID1-ID3, IE1-IE3, IF1-IF3, and IG1-IG3, where Ph is phenyl:

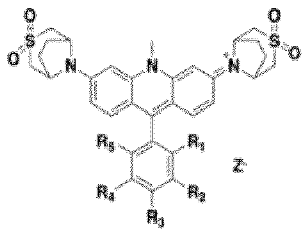

**IA1**

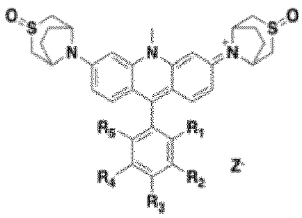

**IA2**

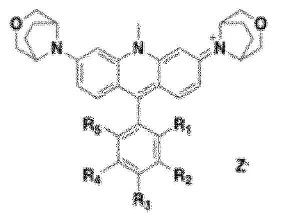

**IA3**

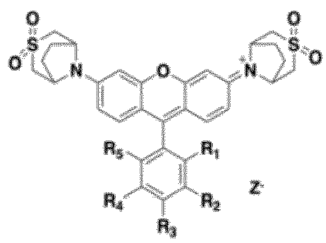

**IB1**

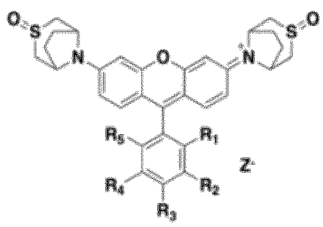

**IB2**

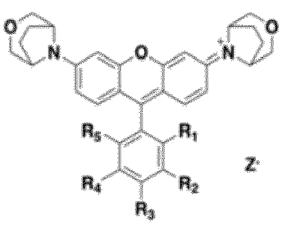

**IB3**

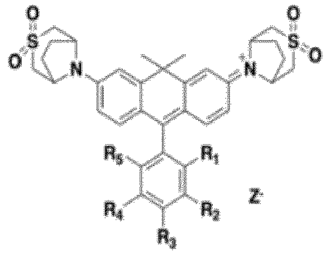

IC1

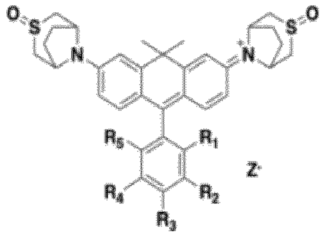

IC2

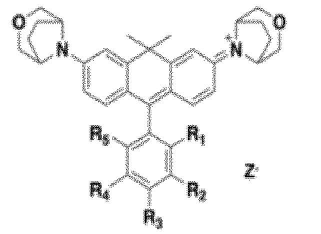

IC3

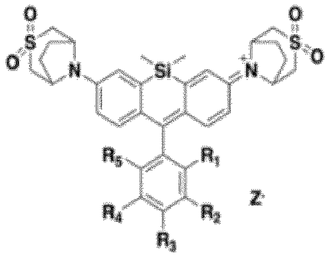

ID1

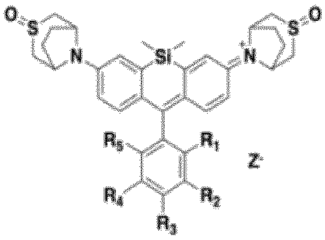

ID2

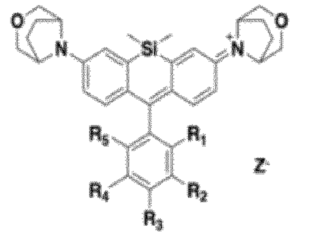

ID3

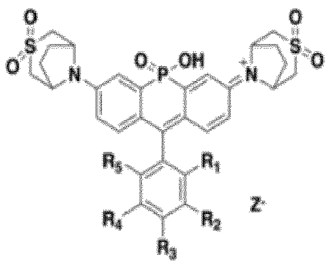

IE1

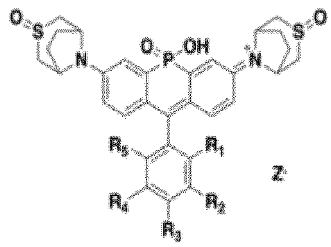

IE2

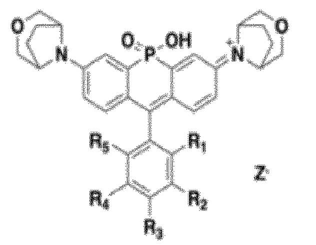

IE3

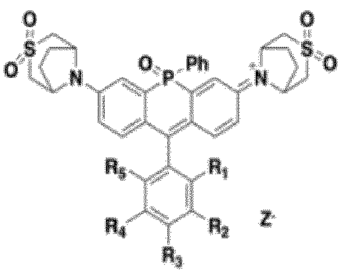

IF1

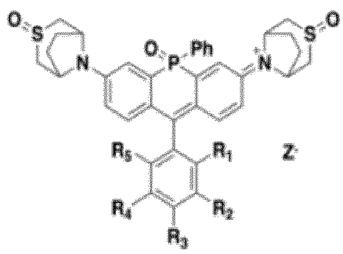

IF2

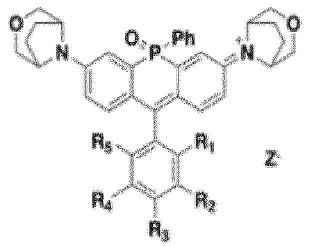

IF3

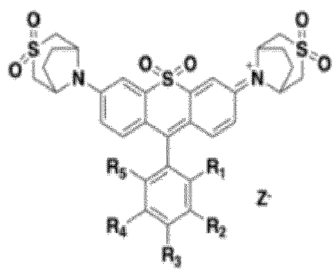

IG1

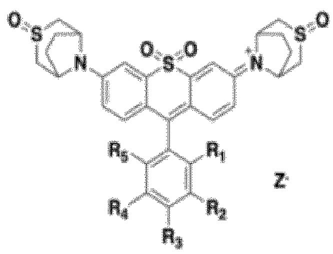

IG2

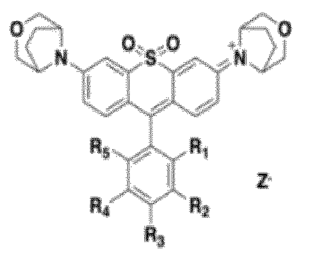

IG3

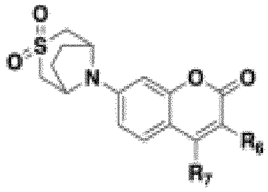

IIA1

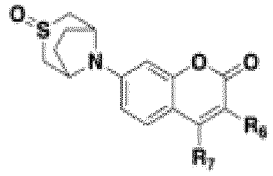

IIA2

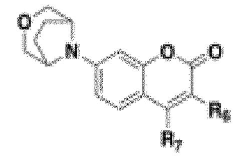

IIA3

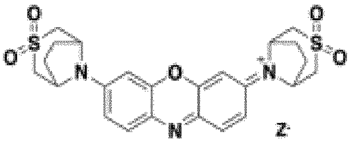

IIIA1

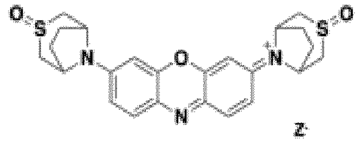

IIIA2

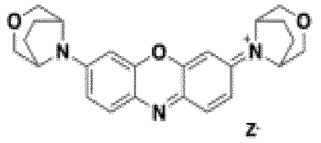

IIIA3

.

In other specific embodiments of the present disclosure, the aza[3.2.1]bridged ring substituted fluorescent dye is selected from at least one of the following formulas A528, A532, A555, A586, A591, A620, A634, A640, A655, A720, A586-2Me, A586-2Me-5-COOH, A528-6-COOH, A532-6-COOH, A555-6-COOH, A586-6-COOH, A591-6-COOH, A620-6-COOH, A634-5-COOH, A640-6-COOH, A655-6-COOH, A528-HTL, A532-HTL, A555-HTL, A586-HTL, A620-HTL, A655-HTL, A555-STL, A528-NHS, A620-STL, A655-STL, A634-NHS, A586-NHS, A591-HTL, A640-HTL, A555-TMP3, A620-TMP3, A655-TMP3, A655-4-Hoechst, A591-TMP3, A640-TMP3, A591-STL, A640-STL, A528-NCN-HTL, A555-NCN-HTL, A620-NCN-HTL, B350, B356, C622, and C654:

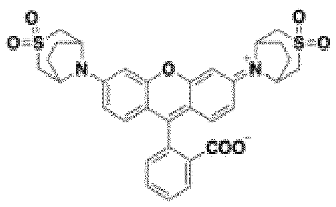

A528

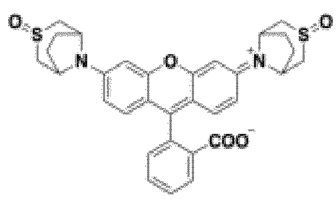

A532

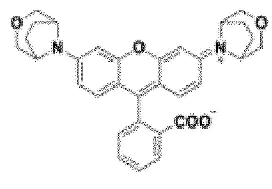

A555

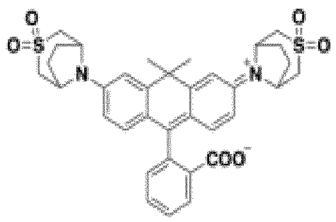

A586

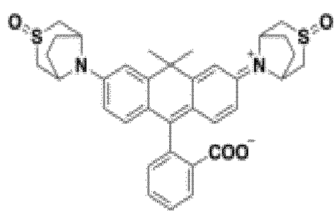

A591

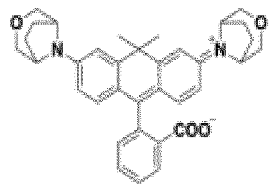

A620

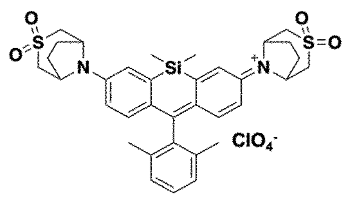

A634

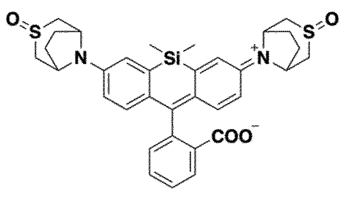

A640

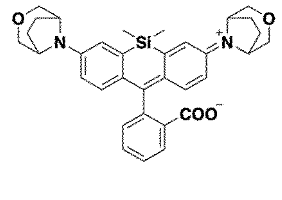

A655

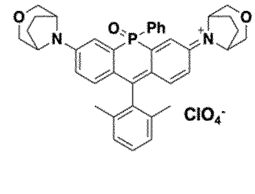

A720

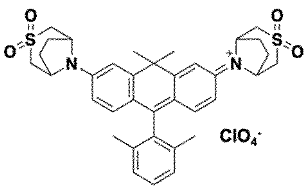

A586-2Me

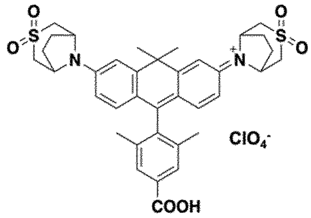

A586-2Me-5-COOH

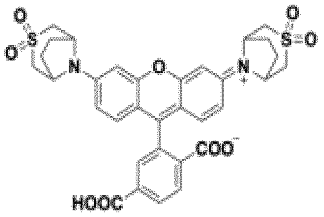

A528-6-COOH

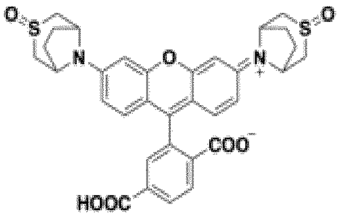

A532-6-COOH

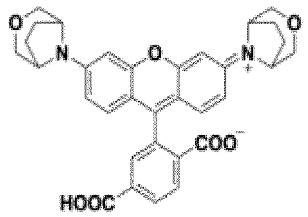

A555-6-COOH

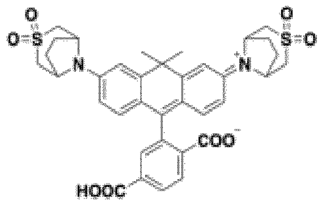

A586-6-COOH

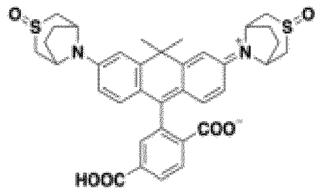

A591-6-COOH

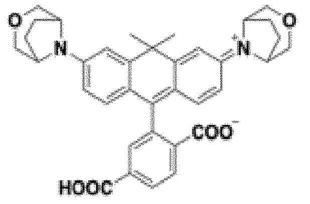

A620-6-COOH

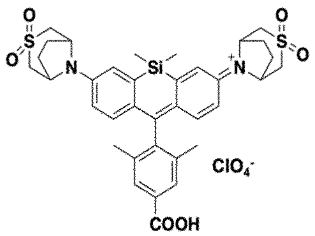

A634-5-COOH

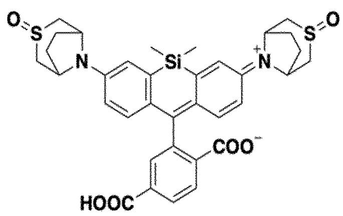

A640-6-COOH

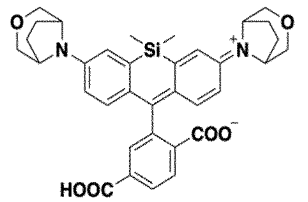

A655-6-COOH

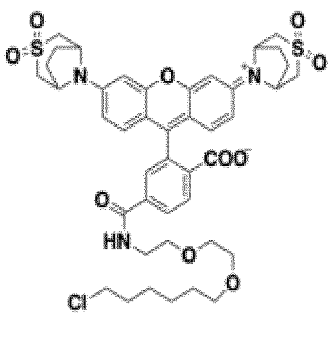

A528-HTL

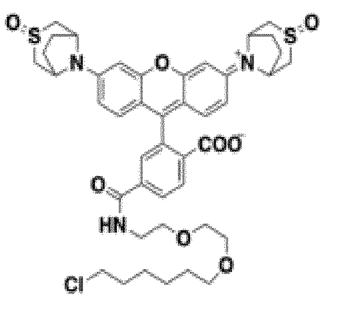

A532-HTL

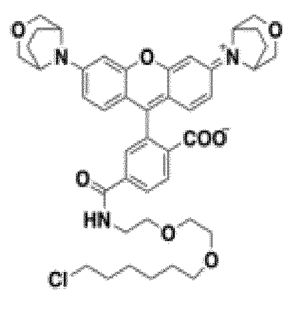

A555-HTL

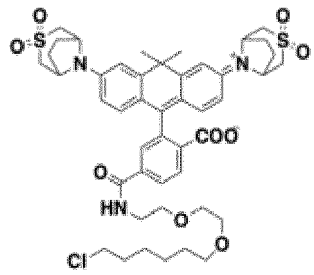

**A586-HTL**

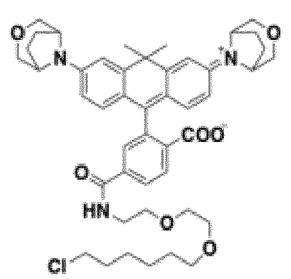

**A620-HTL**

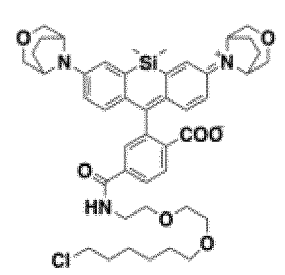

**A655-HTL**

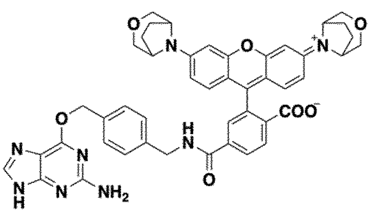

**BD528-STL**

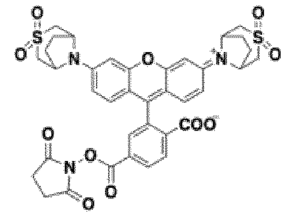

**A528-NHS**

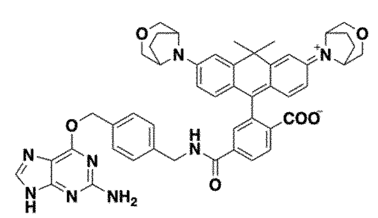

**BD620-STL**

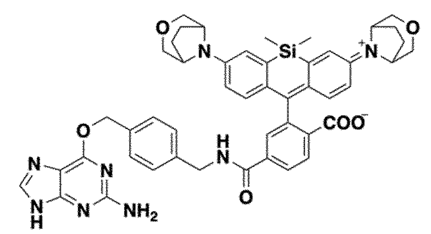

**BD655-STL**

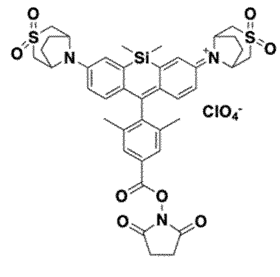

**A634-NHS**

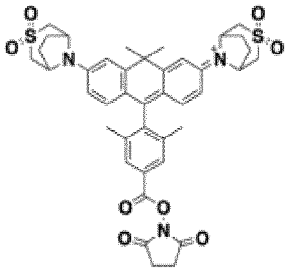

**A586-NHS**

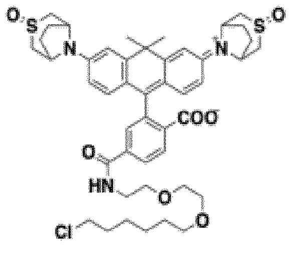

**A591-HTL**

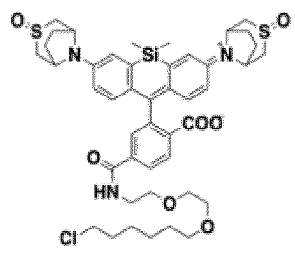

**A640-HTL**

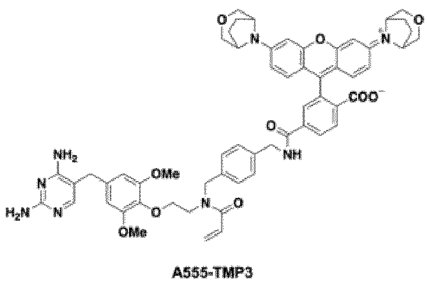

**A555-TMP3**

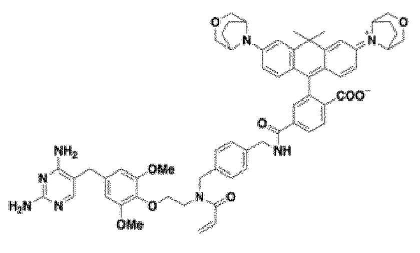

**A620-TMP3**

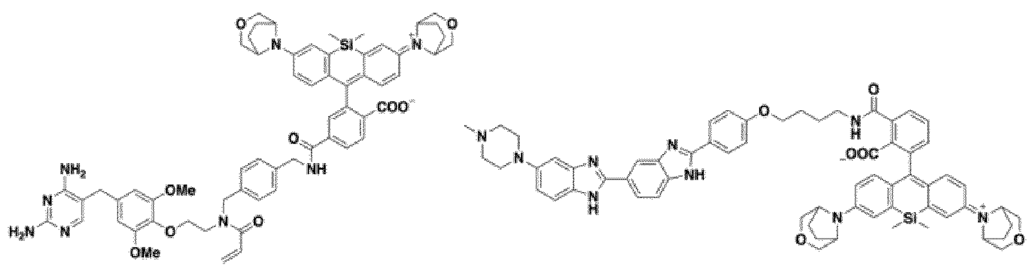

A655-TMP3 A655-4-Hoechst

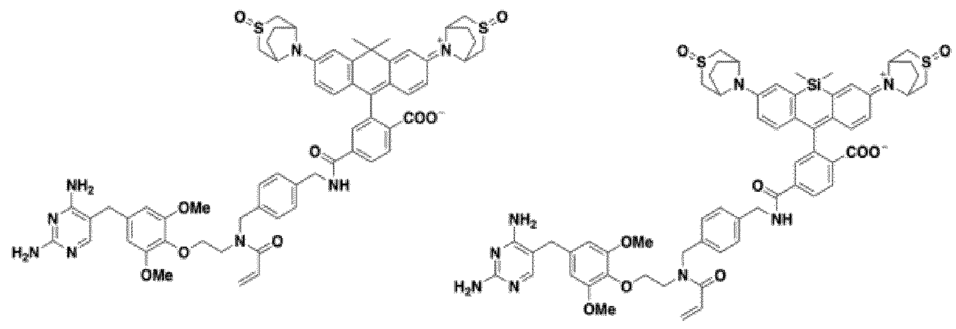

A591-TMP3 A640-TMP3

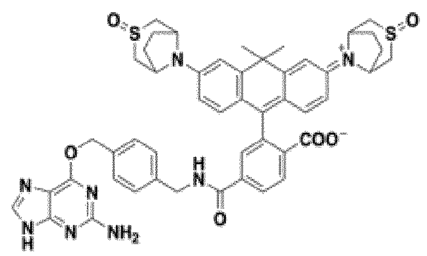

A591-STL

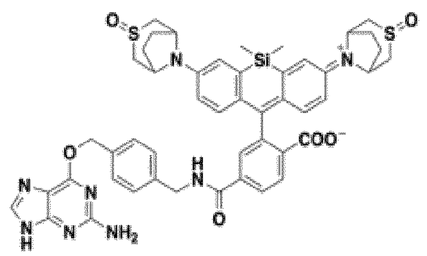

A640-STL

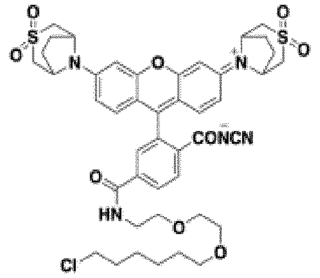

A528-NCN-HTL

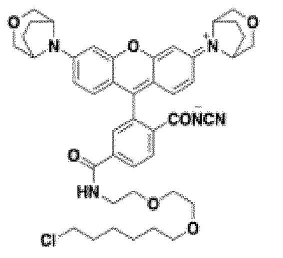

A555-NCN-HTL

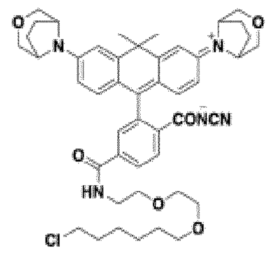

A620-NCN-HTL

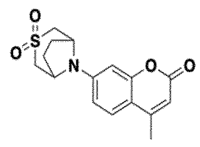

B350

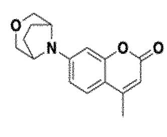

B356

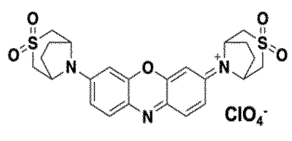

C622

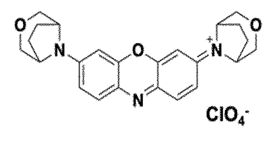

C654

In a second typical embodiment of the present disclosure, a method for preparing the aza[3.2.1]bridged ring substituted fluorescent dye shown in Formula (I) is further provided. The preparation method includes: step S11, mixing a compound A, an aza[3.2.1]bridged ring compound and a first catalyst in a first reaction solvent to carry out a reaction so as to obtain a compound B; and step S12, mixing the compound B with an HZ aqueous solution to carry out a reaction so as to

obtain the aza[3.2.1]bridged ring substituted fluorescent dye shown in Formula (I).

The compound A has a structure shown in Formula (V) below, and the aza[3.2.1]bridged ring compound has a structure shown in Formula (VI) below:

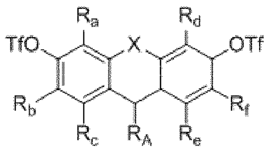

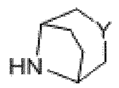

Formula (V) Formula (VI)

$R_A$, $R_a$, $R_b$, $R_c$, $R_d$, $R_e$, $R_f$, X and Y have the same meaning as the $R_A$, the $R_a$, the $R_b$, the $R_c$, the $R_d$, the $R_e$, the $R_f$, the X and the Y in the first typical embodiment, and $Z^-$ in the HZ aqueous solution has the same meaning as the $Z^-$ in the first typical embodiment, which are not repeated herein.

In some embodiments, a reaction diagram is shown in Formula (IX) below:

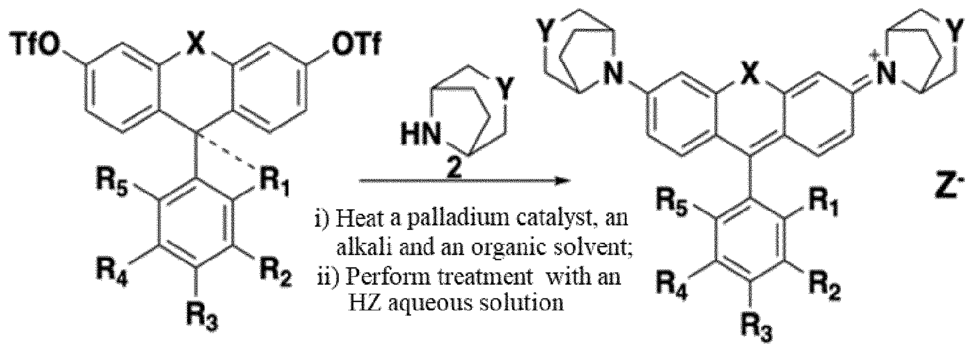


1 I

Formula (IX)

In order to further improve the reaction efficiency, in step S11, the first catalyst includes a palladium catalyst, a palladium catalyst ligand and an alkaline catalyst, where the palladium catalyst includes at least one of tetrakis(triphenylphosphine)palladium, palladium acetate, tris(dibenzylideneacetone)dipalladium, [(diphenylphosphino)ferrocene]dichloropalladium, dichlorobis(triphenylphosphine)palladium and palladium chloride, preferably at least one of tetrakis(triphenylphosphine)palladium, palladium acetate or tris(dibenzylideneacetone)dipalladium.

In the present disclosure, a catalytic reaction is mainly carried out using the palladium catalyst and the palladium ligand. Different palladium catalyst ligands contain different electron-rich substituents, which have a great impact on the reaction yield. In order to further improve the reaction yield, preferably, the palladium catalyst ligand includes at least one of bis(dibenzylideneacetone)palladium, 2,2'-bis(diphenylphosphino)-1,1'-binaphthyl, 2-dicyclohexylphosphino-2',4',6'-triisopropylbiphenyl and 2-dicyclohexylphosphino-2',6'-dimethoxybiphenyl, further preferably at least one of bis(dibenzylideneacetone)palladium and 2,2'-bis(diphenylphosphino)-1,1'-binaphthyl.

Specific types of the alkaline catalyst are not limited. The alkaline catalyst commonly used in the field includes at least one of cesium carbonate, sodium tert-butoxide, potassium carbonate, sodium carbonate, sodium methoxide, sodium ethoxide and potassium tert-butoxide, preferably at least one of cesium carbonate, sodium tert-butoxide and potassium tert-butoxide. The alkaline catalyst can not only improve the deprotonation ability, but also improve the compatibility of functional groups in reaction compounds, thus improving the reaction efficiency.

In step S11, in order to further improve the yield of the aza[3.2.1]bridged ring substituted fluorescent dye, preferably, the reaction is carried out under anhydrous and anaerobic conditions, air in a reaction vessel is replaced by an inert gas, and the inert gas includes, but is not limited to, at least one of nitrogen, helium and argon.

In step S11, in order to further improve the preparation efficiency of the aza[3.2.1]bridged ring substituted fluorescent dye, preferably, the molar ratio of the palladium catalyst to the compound A is (0.1-1.0):1 (such as 0.1:1, 0.2:1, 0.4:1, 0.6:1, 0.8:1, 1:1 or any ranges composed of two values); preferably, the molar ratio of the palladium catalyst ligand to the compound A is (0.3-1.2):1 (such as 0.3:1, 0.6:1, 0.9:1, 1.2:1 or any ranges composed of two values); and preferably, the

molar ratio of the alkaline catalyst to the compound A is (1.2-2.0):1 (such as 1.2:1, 1.4:1, 1.6:1, 1.8:1, 2:1 or any ranges composed of two values). The palladium catalyst and the palladium catalyst ligand are precious metal catalysts with high prices. When the added amounts of the palladium catalyst and the palladium catalyst ligand are too large, the reaction cost is increased. When the added amounts of the palladium catalyst and the palladium catalyst ligand are too small, the effect of catalyzing a reaction cannot be achieved, thus reducing the reaction yield. Therefore, the palladium catalyst and the palladium catalyst ligand used within the above ranges can reduce the reaction cost under the condition of a sufficient reaction. The reaction yield is reduced when the added amount of the alkaline catalyst is too small, and more complicated post-treatment is caused when the added amount of the alkaline catalyst is too large. Thus, the reaction efficiency can be further improved when the alkaline catalyst is within the range.

Specific types of the first reaction solvent are not particularly limited to any reaction solvents commonly used in the field, including but not limited to: at least one of n-hexane, tetrahydrofuran, 1,4-dioxane, ethyl ether and toluene, which can make a reactant completely dissolved, thereby improving the reaction efficiency.

In some embodiments, toluene is usually used as the first reaction solvent when the reactant is a benzene ring compound, and 1,4-dioxane is usually used as the first reaction solvent when the reactant is a heterocyclic ring compound. An appropriate first reaction solvent is selected according to the solubility of the reactant in the organic solvent, so as to improve the reaction efficiency.

In step S11, the reaction is carried out at a temperature of 60-120°C (such as 60°C, 70°C, 80°C, 90°C, 100°C, 110°C, 120°C or any ranges composed of two values). When the reaction temperature is too high, a large number of by-products are produced, which may lead to decomposition of an intermediate. When the reaction temperature is too low, the reaction rate is low. When the reaction temperature is in the range, the reaction yield and the reaction efficiency can be further improved. The reaction is carried out for 4-18 h. When the reaction is carried out to a certain extent, not only is the reaction yield not increased by prolonging the reaction time, but also a large number of by-products are produced, so that the reaction yield is reduced. Moreover, when the reaction time is too short, the reaction is incomplete, so that the reaction yield is reduced. When the time is in the range, the reaction yield can be improved, and the reaction time can be shortened.

In some embodiments, in step S12, the reaction is carried out at a temperature of 20-30°C. The reaction temperature is low, so that the reaction has simple operation and a high reaction yield.

In step S12, in order to avoid release of a large amount of heat from a violent reaction, in some embodiments, the HZ aqueous solution is slowly dropped into the reaction system in step S11 to neutralize a positive charge carried by an intermediate, so that the obtained aza[3.2.1]bridged ring substituted fluorescent dye is electrically neutral. The HZ aqueous solution has a molar concentration of 1-6 mol/L, preferably 1-3 mol/L. When the molar concentration of the HZ aqueous solution is too high, a violent reaction is caused, the reaction rate is too high, and the reaction is not easy to control. When the molar concentration of the HZ aqueous solution is too low, a large amount of the HZ aqueous solution needs to be added. When the molar concentration is within the range, the reaction rate can be effectively improved.

In some embodiments, in order to further improve the purity of the aza[3.2.1]bridged ring substituted fluorescent dye, post-treatment is performed after the reaction is completed, where the post-treatment includes extraction, drying, separation by column chromatography and other common post-treatment processes.

In a third typical embodiment of the present disclosure, a method for preparing the aza[3.2.1]bridged ring substituted fluorescent dye shown in Formula (II) is further provided. The preparation method includes: mixing a compound C, an aza[3.2.1]bridged ring compound and a second catalyst in a second reaction solvent to carry out a reaction so as to obtain the aza[3.2.1]bridged ring substituted fluorescent dye shown in Formula (II).

The compound C has a structure shown in Formula (VII) below:

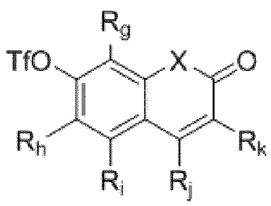

Formula (VII)

$R_g$, $R_h$, $R_i$, $R_j$, $R_k$ and X have the same meaning as the $R_g$, the $R_h$, the $R_i$, the $R_j$, the $R_k$ and the X in the first typical embodiment, and $Z^-$ in the HZ aqueous solution has the same meaning as the Z in the first typical embodiment, which are not repeated herein.

The second catalyst has the same meaning as the first catalyst, and the second reaction solvent has the same meaning as the first reaction solvent, which are not repeated herein.

In some embodiments, a reaction diagram is shown in Formula (X) below:

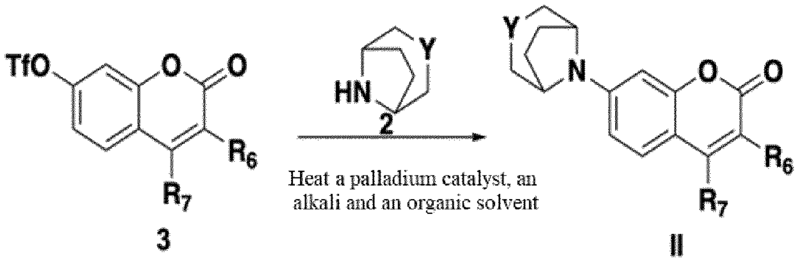


Formula (X)

In addition to different reaction compounds, the third typical embodiment of the present disclosure has same catalysts, same ratios of the catalysts to compounds, same solvents, same reaction process parameters and same post-treatment processes compared with the second typical embodiment, which are not repeated herein.

In a fourth typical embodiment of the present disclosure, a method for preparing the aza[3.2.1]bridged ring substituted fluorescent dye shown in Formula (III) is further provided. The preparation method includes: step S31, mixing a compound D, an aza[3.2.1]bridged ring compound and a third catalyst in a third reaction solvent to carry out a reaction so as to obtain a compound E; step S32, mixing the compound E with an oxidizing agent in a fourth reaction solvent to carry out a reaction so as to obtain a compound F; and step S33, mixing the compound F with an HZ aqueous solution to carry out a reaction so as to obtain the aza[3.2.1]bridged ring substituted fluorescent dye shown in Formula (III).

The compound D has a structure shown in Formula (VIII) below:

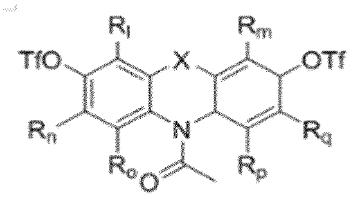

Formula (VIII)

$R_l$, $R_m$, $R_n$, $R_o$, $R_p$, $R_q$, X and Y have the same meaning as the $R_l$, the $R_m$, the $R_n$, the $R_o$, the $R_p$, the $R_q$, the X and the Y in the first typical embodiment, and $Z^-$ in the HZ aqueous solution has the same meaning as the $Z^-$ in the first typical embodiment, which are not repeated herein.

The third catalyst has the same meaning as the first catalyst, and the third reaction solvent has the same meaning as the first reaction solvent, which are not repeated herein.

In some embodiments, a reaction diagram is shown in Formula (XI) below:

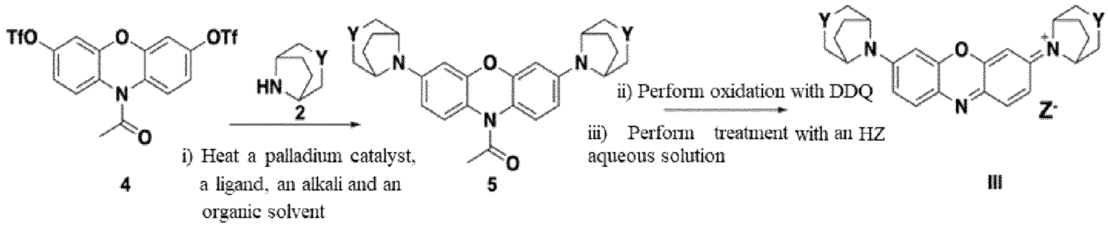


Formula (XI).

Compared with step S11 and step S12 in the second typical embodiment, in addition to different reaction compounds, step S31 and step S33 in the fourth typical embodiment of the present disclosure have same catalysts, same ratios of the catalysts to compounds, same solvents, same reaction process parameters and same post-treatment processes, which are not repeated herein.

In step S32, the oxidizing agent includes at least one of 2,3-dichloro-5,6-dicyano-1,4-benzoquinone, potassium permanganate, hydrogen peroxide (30% aqueous solution) and m-chloroperoxybenzoic acid, preferably 2,3-dichloro-5,6-dicyano-1,4-benzoquinone; and the molar ratio of the oxidizing agent to the compound D is (1.0-2.0):1 (such as 1:1, 1.2:1, 1.4:1, 1.6:1, 1.8:1, 2:1 or any ranges composed of two values).

In order to further improve the reaction yield, in step S32, preferably, the fourth reaction solvent is a mixed solvent of dichloromethane and water, where the volume ratio of the dichloromethane to the water is (10-30):1.

In step S32, the reaction is carried out at a temperature of 40-120°C for 2-24 h.

In a fifth typical embodiment of the present disclosure, use of an aza[3.2.1]bridged ring substituted fluorescent dye in the fields of fluorescence analysis, fluorescence detection and fluorescence imaging as a fluorescent marker is further provided, where the aza[3.2.1]bridged ring substituted fluorescent dye is the aza[3.2.1]bridged ring substituted fluorescent dye provided in the first typical embodiment.

Use of the aza[3.2.1]bridged ring substituted fluorescent dye in fluorescence analysis, fluorescence detection, biological macromolecular labeling, immunofluorescence imaging, live cell imaging, tissue imaging, biological living imaging, single molecule imaging and super-resolution fluorescence imaging as a fluorescent marker is provided.

The beneficial effects of the present disclosure are further described below in combination with examples and comparative examples.

Example 1 Compound A528

The compound A528 was prepared in the following steps.

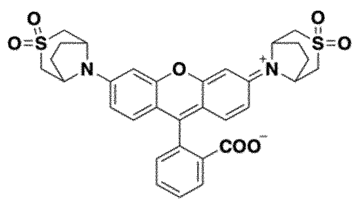

**A528**

(1) A compound 1 (1 mol) and a compound 2 (3 mol) were added into a toluene solvent under anhydrous and anaerobic conditions, and palladium acetate (0.2 mol), 2,2'-bis(diphenylphosphino)-1,1'-binaphthyl (0.3 mol) and cesium carbonate (1.5 mol) were sequentially added to carry out a reaction at a temperature of 100°C for 18 h so as to obtain a compound 3.

(2) A 2 mol/L hydrochloric acid (0.5 mL) aqueous solution was slowly dropped into the reaction system containing the compound 3 to carry out a reaction at a temperature of 25°C for 0.5 h, followed by extraction, drying and separation by column chromatography to obtain a red solid product, namely, the compound A528, with a yield of 33%.

$^1H$ NMR (400 MHz, $CDCl_3$) δ 8.03 (d, *J* = 7.6 Hz, 1H), 7.71 (td, *J* = 7.5, 1.2 Hz, 1H), 7.63 (td, *J* = 7.4, 1.2 Hz, 1H), 7.28 (d, *J* = 7.6 Hz, 1H), 6.72 (d, *J* = 8.7 Hz, 2H), 6.55 (d, *J* = 2.4 Hz, 2H), 6.44 (dd,

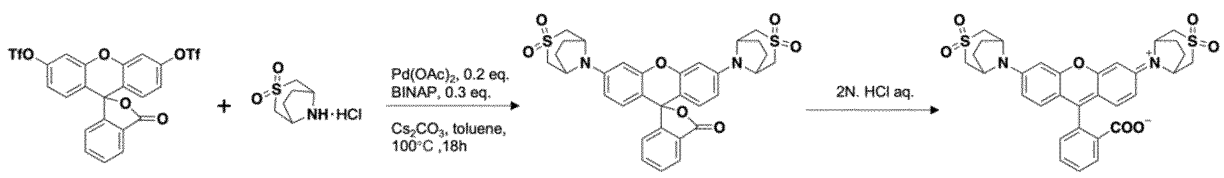


1 2 3 A528

*J* = 8.8, 2.4 Hz, 2H), 4.71-4.54 (m, 4H), 3.57-3.36 (m, 4H), 3.17-3.00 (m, 4H), 2.64-2.48 (m, 4H), 2.34-2.15 (m, 4H).

$^{13}C$ NMR (101 MHz, DMSO-$d_6$) δ 168.71, 152.42, 152.13, 145.56, 135.57, 130.13, 129.27, 126.41, 124.64, 124.19, 111.48, 108.54, 101.37, 83.27, 54.68, 54.64, 52.98, 52.96, 26.42.

An HRMS (ESI) theoretical value of $C_{32}H_{30}N_2O_7S_2$ $[M+H]^+$ was 619.1562, and an actual value was 619.1573.

Example 2 Compound A532

A preparation method in the present example was the same as that in Example 1, and a red solid product with a yield of 19% was obtained.

$^1H$ NMR (400 MHz, Methanol-$d_4$) δ 8.37 (dd, *J* = 7.7, 1.5 Hz, 1H), 7.89 (td, *J* = 7.5, 1.5 Hz, 1H), 7.84 (td, *J* = 7.6, 1.5

Hz, 1H), 7.46 (dd, *J* = 7.4, 1.4 Hz, 1H), 7.33-7.28 (m, 4H), 7.25 (dd, *J* = 9.3, 2.3 Hz, 2H), 5.10-4.99 (m, 4H), 3.91-3.79 (m, 4H), 2.82-2.72 (m, 4H), 2.40-2.27 (m, 4H), 2.14-2.05 (m, 4H).

$^{13}C$ NMR (101 MHz, DMSO-$d_6$) δ 168.82, 153.05, 146.97, 131.54, 130.18, 129.90, 125.76, 125.19, 124.48, 112.57, 108.34, 102.34, 70.38, 53.35, 52.79, 44.70, 29.63, 28.52.

An HRMS (ESI) theoretical value of $C_{32}H_{30}N_2O_5S_2$ $[M+H]^+$ was 587.1663, and an actual value was 587.1632.

Example 3 Compound A555

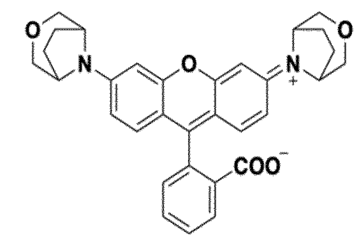

A555

A preparation method in the present example was the same as that in Example 1, and a purple-red solid product with a yield of 40% was obtained.

$^1H$ NMR (400 MHz, $CDCl_3$) δ 8.03 (d, J = 7.6 Hz, 1H), 7.66 (td, J = 7.5, 1.3 Hz, 1H), 7.60 (td, J = 7.4, 1.1 Hz, 1H), 7.21 (d, J = 7.6 Hz, 1H), 6.64 (d, J = 8.8 Hz, 2H), 6.56 (d, J = 2.4 Hz, 2H), 6.45 (dd, J = 8.8, 2.4 Hz, 2H), 4.12-4.03 (m, 4H), 3.92-3.84 (m, 4H), 3.57-3.49 (m, 4H), 2.16-1.97 (m, 8H).

$^{13}C$ NMR (101 MHz, DMSO-$d_6$) δ 166.66, 152.21, 148.26, 137.07, 130.83, 130.24, 129.41, 129.18, 120.15, 115.01, 112.97, 108.34, 72.30, 70.24, 57.22, 34.94, 27.20.

An HRMS (ESI) theoretical value of $C_{32}H_{30}N_2O_5$ $[M+H]^+$ was 523.2222, and an actual value was 523.2256.

Example 4 Compound A586

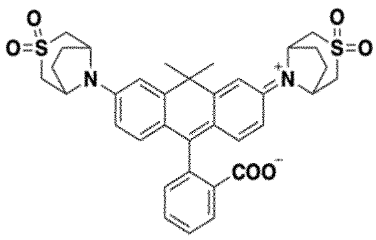

A586

A preparation method in the present example was the same as that in Example 1, and a white solid product with a yield of 12% was obtained.

$^1H$ NMR (400 MHz, $CDCl_3$) δ 8.01 (d, J = 7.6 Hz, 1H), 7.65 (td, J = 7.5, 1.2 Hz, 1H), 7.58 (td, J = 7.4, 1.2 Hz, 1H), 7.13 (d, J = 7.6 Hz, 1H), 6.91 (d, J = 2.5 Hz, 2H), 6.70 (d, J = 8.7 Hz, 2H), 6.55 (dd, J = 8.8, 2.4 Hz, 2H), 4.77-4.63 (m, 4H), 3.54-3.35 (m, 4H), 3.16-2.98 (m, 4H), 2.62-2.49 (m, 4H), 2.30-2.18 (m, 4H), 1.83 (s, 3H), 1.75 (s, 3H).

$^{13}C$ NMR (101 MHz, $CDCl_3$) δ 170.77, 155.28, 147.06, 143.81, 135.04, 130.29, 129.31, 126.47, 125.06, 124.23, 122.10, 113.98, 111.66, 86.47, 54.70, 54.63, 53.46, 53.44, 38.41, 35.31, 33.49, 27.06, 27.04.

An HRMS (ESI) theoretical value of $C_{35}H_{36}N_2O_6S_2$ $[M+H]^+$ was 645.2082, and an actual value was 645.2048.

Example 5 Compound A591

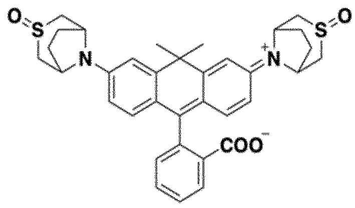

A591

A preparation method in the present example was the same as that in Example 1, and a bluish violet solid product with a yield of 10% was obtained.

An MS (ESI) theoretical value of $C_{35}H_{36}N_2O_4S_2$ $[M+H]^+$ was 613.21, and an actual value was 613.21.

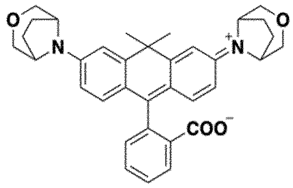

A620

Example 6 Compound A620

A preparation method in the present example was the same as that in Example 1, and a blue solid product with a yield of 18% was obtained.

$^1$H NMR (400 MHz, $CDCl_3$) δ 7.99 (dd, J = 7.0, 1.4 Hz, 1H), 7.60 (td, J = 7.4, 1.4 Hz, 1H), 7.55 (td, J = 7.4, 1.2 Hz, 1H), 7.08 (d, J = 7.6 Hz, 1H), 6.97 (d, J = 2.3 Hz, 2H), 6.60 (d, J = 8.7 Hz, 2H), 6.54 (dd, J = 8.8, 2.4 Hz, 2H), 4.11-4.06 (m, 4H), 3.93-3.86 (m, 4H), 3.56-3.50 (m, 4H), 2.12-1.96 (m, 8H), 1.82 (s, 3H), 1.73 (s, 3H).

$^{13}$C NMR (101 MHz, $CDCl_3$) δ 170.83, 155.31, 147.80, 147.15, 134.58, 129.49, 129.02, 127.14, 125.00, 124.00, 121.34, 114.35, 112.26, 87.70, 69.79, 69.77, 57.06, 38.52, 35.58, 32.60, 26.88.

An HRMS (ESI) theoretical value of $C_{35}H_{36}N_2O_4$ $[M+H]^+$ was 549.2742, and an actual value was 549.2748.

Example 7 Compound A634

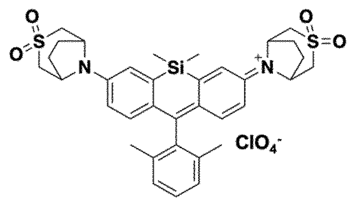

A634

A preparation method in the present example was the same as that in Example 1, and a blue solid product with a total yield of 8% was obtained.

An HRMS (ESI) theoretical value of $C_{35}H_{41}N_2O_4S_2Si^+$ $[M]^+$ was 645.2272, and an actual value was 645.2305.

Example 8 Compound A640

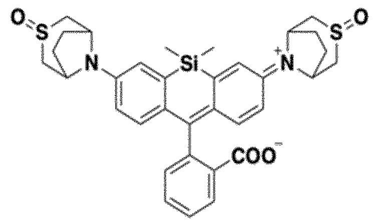

A640

A preparation method in the present example was the same as that in Example 1, and a white solid product with a yield of 5% was obtained.
An MS (ESI) theoretical value of $C_{34}H_{36}N_2O_4S_2Si$ $[M+H]^+$ was 629.10, and an actual value was 629.19.

Example 9 Compound A655

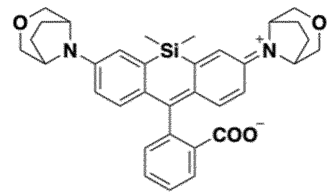

A655

A preparation method in the present example was the same as that in Example 1, and a blue solid product with a yield of 14% was obtained.
$^1$H NMR (400 MHz, Methanol-*d*$_4$) δ 8.22 (d, *J* = 7.9 Hz, 1H), 7.77 (td, *J* = 7.5, 1.4 Hz, 1H), 7.70 (td, *J* = 7.6, 1.3 Hz, 1H), 7.36 (d, *J* = 2.7 Hz, 2H), 7.31 (dd, *J* = 7.6, 1.3 Hz, 1H), 6.96 (d, *J* = 9.4 Hz, 2H), 6.77 (dd, *J* = 9.5, 2.7 Hz, 2H), 4.62 (s, 4H), 3.72 (q, *J* = 11.0 Hz, 8H), 2.26 - 2.01 (m, 7H), 0.63 (s, 3H), 0.58 (s, 3H).
An HRMS (ESI) theoretical value of $C_{34}H_{36}N_2O_4Si$ $[M+H]^+$ was 565.2517, and an actual value was 565.2528.

Example 10 Compound A528-6-COOH

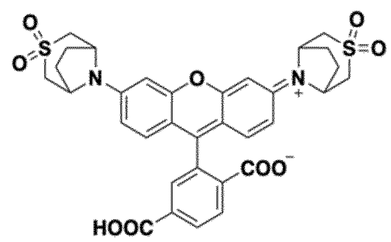

A528-6-COOH

A preparation method in the present example was the same as that in Example 1, and a red solid product with a yield of 28% was obtained.
$^1$H NMR (400 MHz, DMSO-*d*$_6$) δ 8.27 (d, J = 7.6, 1H), 8.20 (d, J = 8.4 Hz, 1H), 7.80 (s, 1H), 6.66 - 7.12 (m, 6H), 4.93 (s, 4H), 3.20 - 3.45 (m, 8H), 2.36 (d, J = 7.2 Hz, 4H), 2.07 - 2.22 (m, 4H).
$^{13}$C NMR (101 MHz, Methanol-*d*$_4$)δ167.48, 159.29, 158.56, 158.20, 155.14, 148.82, 137.04, 131.38, 130.79, 120.54, 117.64, 114.73, 113.92, 100.79, 82.72, 56.31, 53.88, 28.09.
An HRMS (ESI) theoretical value of $C_{33}H_{30}N_2O_9S_2$ $[M+H]^+$ was 663.1465, and an actual value was 663.1467.

Example 11 Compound A555-6-COOH

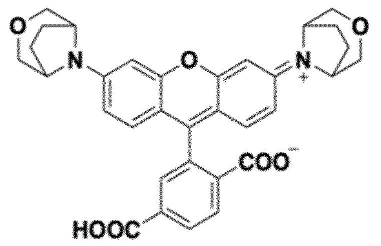

A555-6-COOH

A preparation method in the present example was the same as that in Example 1, and a purple-red solid product with a yield of 37% was obtained.

$^1$H NMR (400 MHz, Methanol-$d_4$) δ 8.44 - 8.37 (m, 2H), 7.99 (s, 1H), 7.16 - 7.07 (m, 6H), 4.68 - 4.57 (m, 4H), 3.82 - 3.66 (m, 8H), 2.26 - 2.07 (m, 8H).

$^{13}$C NMR (101 MHz, Methanol-$d_4$) δ 167.67, 167.38, 159.74, 159.65, 154.68, 135.99, 135.98, 135.36, 132.85, 132.70, 132.36, 132.32, 116.89, 115.43, 98.98, 72.75, 59.30, 27.45.

An HRMS (ESI) theoretical value of $C_{37}H_{30}N_2O_7$ $[M+H]^+$ was 567.2126, and an actual value was 567.2148.

Example 12 Compound A586-6-COOH

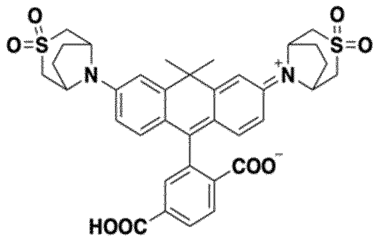

A586-6-COOH

A preparation method in the present example was the same as that in Example 1, and a white solid product with a yield of 16% was obtained.

$^1$H NMR (400 MHz, DMSO-$d_6$) δ 8.18 (dd, *J* = 8.0, 1.3 Hz, 1H), 8.11 (d, *J* = 8.0 Hz, 1H), 7.49 (s, 1H), 7.20 (d, *J* = 2.5 Hz, 2H), 6.77 (dd, *J* = 8.9, 2.4 Hz, 2H), 6.55 (d, *J* = 8.7 Hz, 2H), 4.97 - 4.78 (m, 4H), 3.32 - 3.18 (m, 8H), 2.37 - 2.25 (m, 4H), 2.18 - 2.00 (m, 4H), 1.86 (s, 3H), 1.76 (s, 3H).

$^{13}$C NMR (101 MHz, DMSO-$d_6$) δ 169.41, 166.48, 155.59, 147.59, 144.62, 137.63, 130.85, 129.51, 129.36, 125.79, 124.40, 120.28, 114.78, 112.69, 55.01, 53.21, 53.17,38.68, 34.56, 33.62, 26.88.

An HRMS (ESI) theoretical value of $C_{36}H_{36}N_2O_8S_2$ $[M+H]^+$ was 689.1986, and an actual value was 689.1990.

Example 13 Compound A591-6-COOH

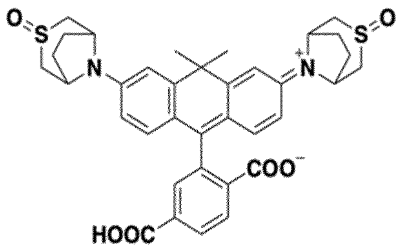

A591-6-COOH

A preparation method in the present example was the same as that in Example 1, and a blue solid product with a yield of 8% was obtained.

An MS (ESI) theoretical value of $C_{36}H_{36}N_2O_6S_2$ $[M+H]^+$ was 657.21, and an actual value was 657.32.

Example 14 Compound A620-6-COOH

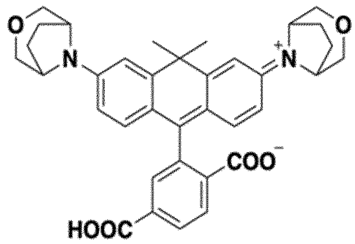

A620-6-COOH

A preparation method in the present example was the same as that in Example 1, and a blue solid product with a yield of 16% was obtained.

$^1$H NMR (400 MHz, DMSO-$d_6$) δ 8.28 - 8.13 (m, 2H), 7.68 (s, 1H), 7.40 - 7.19 (m, 2H), 6.91 - 6.61 (m, 4H), 4.77 - 4.49 (m, 4H), 3.73-3.53 (m, 8H), 2.12 - 1.86 (m, 8H).

$^{13}$C NMR (101 MHz, DMSO-$d_6$) δ 169.99, 164.41, 155.36, 147.78, 146.98, 137.76, 130.05, 129.93, 129.38, 124.97, 124.80, 120.51, 114.38, 112.27, 82.34, 56.99, 56.97, 38.36, 35.24, 33.02, 26.80.

An HRMS (ESI) theoretical value of $C_{36}H_{36}N_2O_8S_2$ $[M+H]^+$ was 593.2646, and an actual value was 593.2641.

Example 15 Compound A634-5-COOH

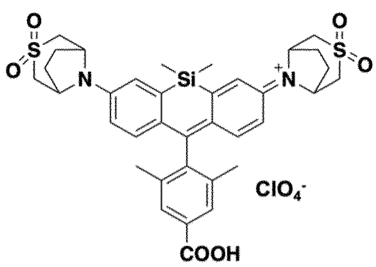

A634-5-COOH

A preparation method in the present example was the same as that in Example 1, and a blue solid product with a total yield of 2% was obtained.

An MS (ESI) theoretical value of $C_{36}H_{41}N_2O_6S_2Si^+$ [M]+ was 689.22, and an actual value was 689.42.

Example 16 Compound A640-6-COOH

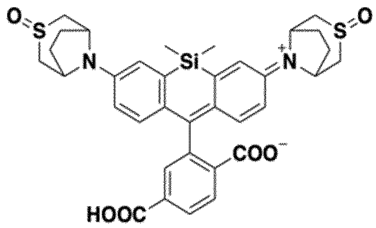

A640-6-COOH

A preparation method in the present example was the same as that in Example 1, and a blue solid product with a yield of 6% was obtained.

An MS (ESI) theoretical value of $C_{35}H_{36}N_2O_6S_2Si$ [M+H]+ was 673.19, and an actual value was 673.40.

Example 17 Compound A655-6-COOH

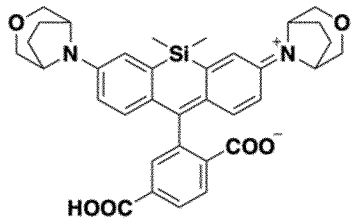

A655-6-COOH

A preparation method in the present example was the same as that in Example 1, and a blue solid product with a yield of 14% was obtained.

$^1H$ NMR (400 MHz, Methanol-$d_4$) δ 8.28 (dd, J = 8.1, 1.5 Hz, 1H), 8.22 (d, J = 8.1 Hz, 1H), 7.87 (d, J = 1.4 Hz, 1H), 7.32 (d, J = 2.6 Hz, 2H), 6.89 (d, J = 9.2 Hz, 2H), 6.76 (dd, J = 9.4, 2.7 Hz, 2H), 4.53 - 4.48 (m, 4H), 3.85 - 3.73 (m, 4H), 3.72 - 3.59 (m, 4H), 2.18 -1.95 (m, 8H), 0.65 (s, 3H), 0.57 (s, 3H).

An HRMS (ESI) theoretical value of $C_{35}H_{36}N_2O_6Si$ [M+H]+ was 609.2415, and an actual value was 609.2420.

Example 18 Compound A720

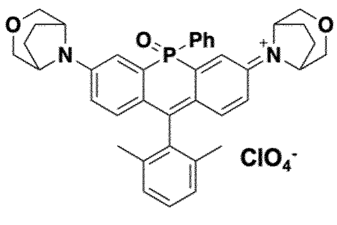

A720

A preparation method in the present example was the same as that in Example 1, and a green solid product with a

total yield of 8% was obtained.
An MS (ESI) theoretical value of $C_{39}H_{4o}N_2O_3P^+$ $[M]^+$ was 615.28, and an actual value was 615.57.

Example 19 Compound A586-2Me

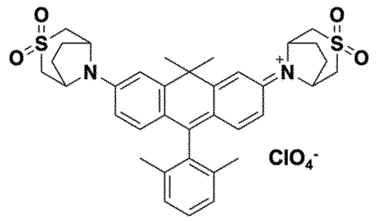

**A586-2Me**

A preparation method in the present example was the same as that in Example 1, and a purple-red solid product with a total yield of 18% was obtained.
An MS (ESI) theoretical value of $C_{36}H_{41}N_2O_4S_2^+$ $[M]^+$ was 629.25, and an actual value was 629.38.

Example 20 Compound B350

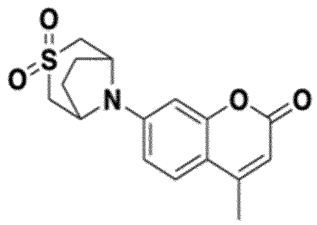

**B350**

The compound B350 was prepared in the following steps.

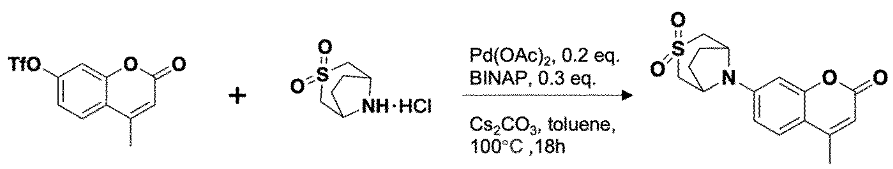


**4** **5** **B350**

A compound 4 (1 mol) and a compound 5 (3 mol) were added into a toluene solvent under anhydrous and anaerobic conditions, and palladium acetate (0.2 mol), 2,2'-bis(diphenylphosphino)-1,1'-binaphthyl (0.3 mol) and cesium carbonate (1.5 mol) were sequentially added to carry out a reaction at a temperature of 100°C for 18h, followed by extraction, drying and separation by column chromatography to obtain a colorless solid product, namely, the compound B350, with a yield of 40%.
$^1H$ NMR (400 MHz, DMSO-$d_6$) δ 7.63 (d, J = 8.8 Hz, 1H), 6.98 (dd, J = 8.8, 2.5 Hz, 1H), 6.94 (d, J = 2.4 Hz, 1H), 6.09 (q, J = 1.3 Hz, 1H), 4.93 - 4.87 (m, 2H), 3.31 - 3.28 (m, 2H), 3.27 - 3.20 (m, 2H), 2.37 (d, J = 1.2 Hz, 3H), 2.36 - 2.33 (m, 2H), 2.18-2.13 (m, 2H).
$^{13}C$ NMR (101 MHz, DMSO-$d_6$) δ 160.97, 155.93, 154.05, 147.47, 127.44, 112.21, 111.52, 110.38, 101.78, 55.61, 53.69, 40.74, 40.54, 40.33, 40.12, 39.91, 39.70, 39.49, 26.94, 18.59.
An HRMS (ESI) theoretical value of $C_{16}H_{17}NO_4S$ $[M+H]^+$ was 320.0957, and an actual value was 320.0962.

Example 21 Compound B356

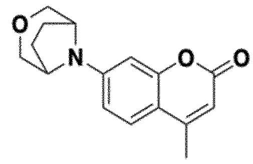

B356

A preparation method in the present example was the same as that in Example 20, and a colorless solid product with a yield of 70% was obtained.

$^1$H NMR (400 MHz, $CDCl_3$) δ 7.43 (d, J = 8.8 Hz, 1H), 6.68 (dd, J = 8.8, 2.4 Hz, 1H), 6.59 (d, J = 2.4 Hz, 1H), 6.02 (q, J = 1.2 Hz, 1H), 4.18 - 4.11 (m, 2H), 3.86 (d, J = 10.9 Hz, 2H), 3.56 (dt, J = 11.1, 1.1 Hz, 2H), 2.36 (d, J = 1.2 Hz, 3H), 2.20-2.03 (m, 4H).

$^{13}$C NMR (101 MHz, $CDCl_3$) δ 162.00, 156.04, 152.90, 149.79, 126.10, 111.69, 110.95, 110.23, 101.57, 77.48, 77.16, 76.84, 69.60, 56.97, 27.08, 18.62.

An HRMS (ESI) theoretical value of $C_{16}H_{17}NO_3$ $[M+H]^+$ was 272.1287, and an actual value was 272.1280.

Example 22 Compound C622

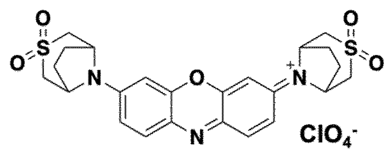

C622

The compound C622 was prepared in the following steps.

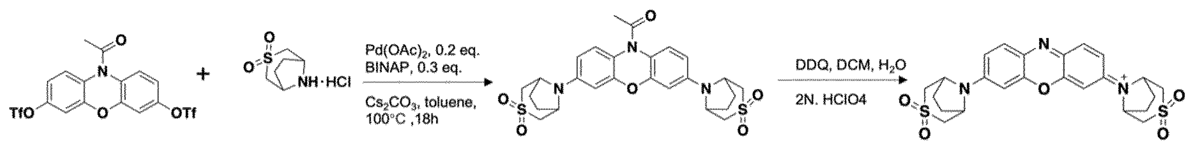


6 7 8

( 1 ) A compound 6 (1 mol) and a compound 7 (3 mol) were added into a toluene solvent under anhydrous and anaerobic conditions, and palladium acetate (0.2 mol), 2,2'-bis(diphenylphosphino)-1,1'-binaphthyl (0.3 mol) and cesium carbonate (1.5 mol) were sequentially added to carry out a reaction at a temperature of 100°C for 18 h so as to obtain a compound 8.

( 2 ) The compound 8 was dissolved in a mixed solvent (100 mL) of dichloromethane and water at a ratio of 20:1, and 2,3-dichloro-5,6-dicyano-1,4-benzoquinone (1.6 mol) was added to carry out a reaction at a temperature of 25°C for 3 h.

( 3 ) A 2 mol/L hypochlorous acid (5 mL) aqueous solution was slowly dropped into the reaction system containing the compound 7 to carry out a reaction at a temperature of 25°C for 0.5 h, followed by extraction, drying and separation by column chromatography to obtain a blue solid product, namely, the compound C622, with a total yield of 35%.

An HRMS (ESI) theoretical value of $C_{24}H_{26}N_3O_5S_2^+$ $[M]^+$ was 500.13, and an actual value was 500.14.

Example 23 Compound C654

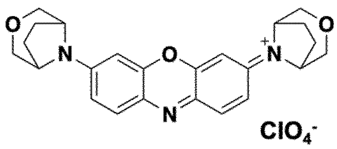

C654

A preparation method in the present example was the same as that in Example 22, and a blue solid product with a total yield of 52% was obtained.

An HRMS (ESI) theoretical value of $C_{24}H_{26}N_3O_3^+$ $[M]^+$ was 404.20, and an actual value was 404.19.

Compounds listed below are functional derivatives of fluorescent matrices above, which are synthesized by dissolving corresponding dye matrices containing carboxyl and functional groups containing amino in dimethylformamide (DMF), adding 1.2 equivalent amount of a Castros condensing agent and 2.0 equivalent amount of diisopropylethylamine (DIPEA), performing stirring at normal temperature for 1 h, and performing separation and purification by high performance liquid chromatography to obtain final products.

Example 24 Compound A528-HTL

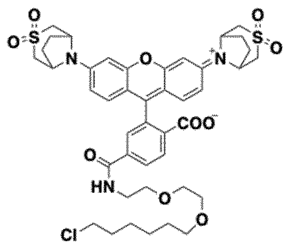

A528-HTL

The compound A528-HTL was obtained by condensation of the A528-6-COOH in Example 10 and HaloTag($O_2$) amines, and a red solid product with a yield of 78% was obtained.

An HRMS (ESI) theoretical value of $C_{43}H_{50}ClN_3O_{10}S_2$ $[M+H]^+$ was 868.2699, and an actual value was 868.2720.

Example 25 Compound A528-NCN-HTL

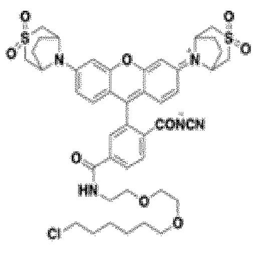

A528-NCN-HTL

The compound A528-NCN-HTL was obtained by condensation of the compound A528-NCN-6-COOH and HaloTag(Oz)amines, and a red solid product with a yield of 81% was obtained.

An MS (ESI) theoretical value of $C_{44}H_{54}ClN_5O_9S_2$ $[M+H]^+$ was 896.31, and an actual value was 896.70.

Example 26 Compound A532-HTL

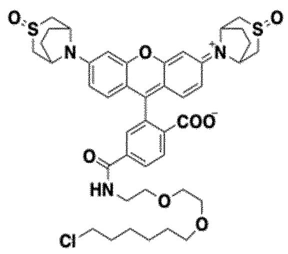

A532-HTL

The compound A532-HTL was obtained by condensation of the compound A532-6-COOH and HaloTag(Oz) amines, and a red solid product with a yield of 72% was obtained.

An HRMS (ESI) theoretical value of $C_{43}H_{50}ClN_3O_8S_2$ $[M+H]^+$ was 836.2801, and an actual value was 836.2830.

Example 27 Compound A555-HTL

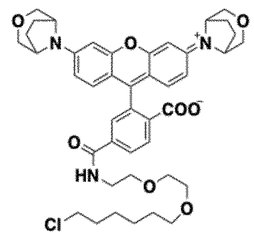

A555-HTL

The compound A555-HTL was obtained by condensation of the A555-6-COOH in Example 11 and HaloTag($O_2$) amines, and a purple-red solid product with a yield of 82% was obtained.

An HRMS (ESI) theoretical value of $C_{43}H_{50}ClN_3O_8$ $[M+H]^+$ was 772.3359, and an actual value was 772.3383.

Example 28 Compound A555-STL

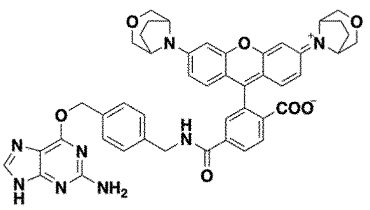

BD528-STL

The compound A555-STL was obtained by condensation of the A555-6-COOH in Example 11 and BG-$NH_2$, and a purple-red solid product with a yield of 65% was obtained.

An MS (ESI) theoretical value of $C_{46}H_{42}N_8O_7$ $[M+H]^+$ was 819.32, and an actual value was 819.68.

Example 29 Compound A555-TMP3

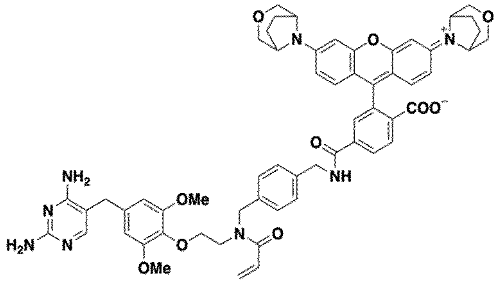

A555-TMP3

The compound A555-TMP3 was obtained by condensation of the A555-6-COOH in Example 11 and TMP3-$NH_2$, and a purple-red solid product with a yield of 74% was obtained.

An MS (ESI) theoretical value of $C_{59}H_{60}N_8O_{10}$ $[M+2H]^{2+}$ was 521.23, and an actual value was 521.70.

Example 30 Compound A586-HTL

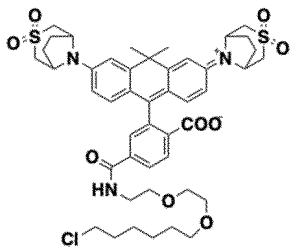

A586-HTL

The compound A586-HTL was obtained by condensation of the A586-6-COOH in Example 12 and HaloTag($O_2$) amines, and a white solid product with a yield of 52% was obtained.

An HRMS (ESI) theoretical value of $C_{46}H_{56}ClN_3O_9S_2$ $[M+H]^+$ was 894.3219, and an actual value was 894.3216.

Example 31 Compound A620-HTL

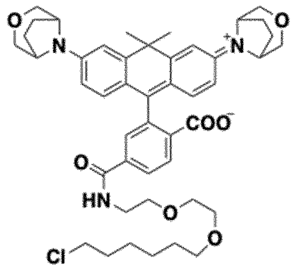

A620-HTL

The compound A620-HTL was obtained by condensation of the A620-6-COOH in Example 14 and HaloTag($O_2$) amines, and a blue solid product with a yield of 62% was obtained.

An HRMS (ESI) theoretical value of $C_{46}H_{56}ClN_3O_7$ $[M+H]^+$ was 798.3880, and an actual value was 798.3872.

Example 32 Compound A620-STL

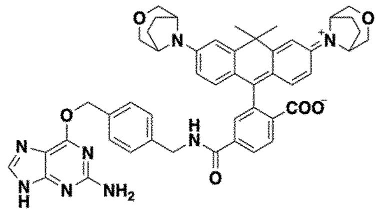

BD620-STL

The compound A620-STL was obtained by condensation of the A620-6-COOH in Example 14 and BG-$NH_2$, and a blue solid product with a yield of 51% was obtained.

An HRMS (ESI) theoretical value of $C_{49}H_{48}N_8O_6$ was $[M+H]^+$ 845.3770, and an actual value was 845.3776.

Example 33 Compound A620-TMP3

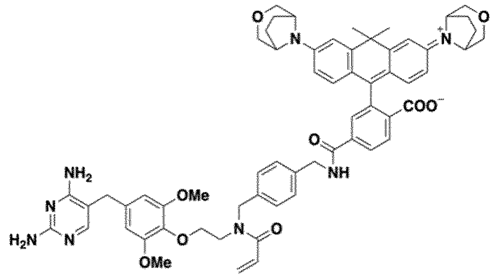

A620-TMP3

The compound A620-TMP3 was obtained by condensation of the A620-6-COOH in Example 14 and TMP3-$NH_2$, and a blue solid product with a yield of 42% was obtained.

An MS (ESI) theoretical value of $C_{62}H_{66}N_8O_9$ $[M+2H]^{2+}$ was 534.26, and an actual value was 534.42.

Example 34 Compound A655-HTL

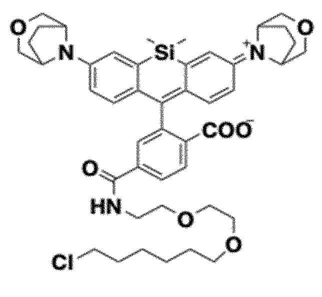

A655-HTL

The compound A655-HTL was obtained by condensation of the A655-6-COOH in Example 17 and HaloTag(Oz) amines, and a blue solid product with a yield of 86% was obtained.

An HRMS (ESI) theoretical value of $C_{45}H_{56}ClN_3O_7Si$ $[M+H]^+$ was 814.3649, and an actual value was 814.3650.

Example 35 Compound A655-STL

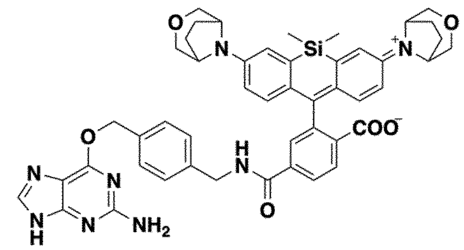

BD655-STL

The compound A655-STL was obtained by condensation of the A655-6-COOH in Example 17 and BG-$NH_2$, and a blue solid product with a yield of 60% was obtained.

An MS (ESI) theoretical value of $C_{48}H_{48}N_8O_6Si$ $[M+H]^+$ was 861.35, and an actual value was 861.49.

Example 36 Compound A655-TMP3

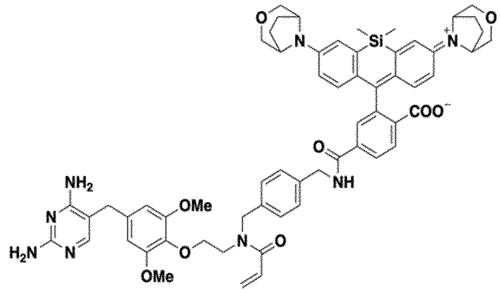

A655-TMP3

The compound A655-TMP3 was obtained by condensation of the A655-6-COOH in Example 17 and TMP3-$NH_2$, and a blue solid product with a yield of 57% was obtained.

An MS (ESI) theoretical value of $C_{61}H_{66}N_8O_9Si$ $[M+2H]^{2+}$ was 542.24, and an actual value was 542.68.

Example 37 Compound A655-4-Hoechst

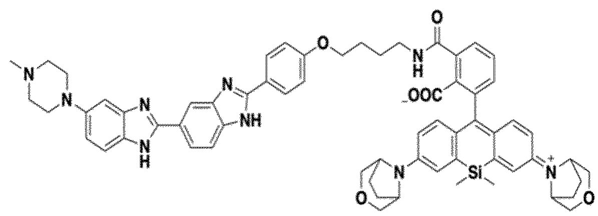

A655-4-Hoechst

The compound A655-4-Hoechst was obtained by condensation of the A655-6-COOH in Example 17 and Hoechst-$NH_2$, and a green solid product with a yield of 32% was obtained.

An MS (ESI) theoretical value of $C_{64}H_{67}N_9O_6Si$ $[M+H]^+$ was 1086.51, and an actual value was 1086.22.

Comparative Example 1 Compound JF549

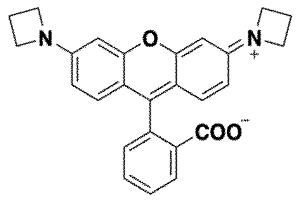

JF549

Comparative Example 2 Compound TMRE

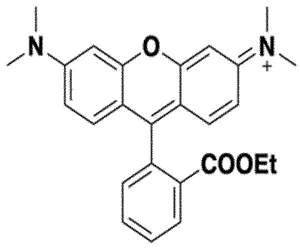

TMRE

Comparative Example 3 Compound Rhodamine B

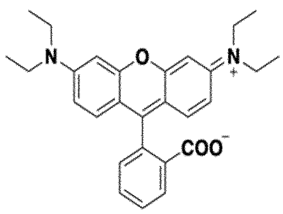

Rhodamine B

Comparative Example 4 Compound Cy3

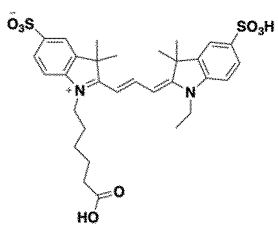

Cy3

Comparative Example 5 Compound Atto647N

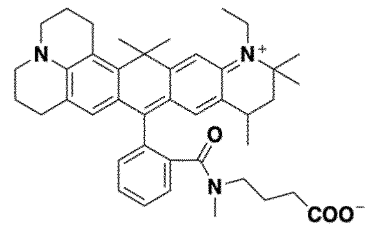

**Atto 647N**

Comparative Example 6 Compound TMR-HTL

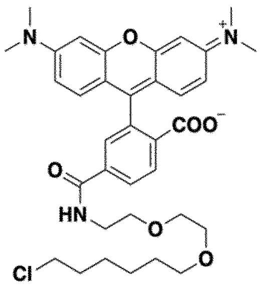

**TMR-HTL**

Comparative Example 7 Compound JF549-HTL

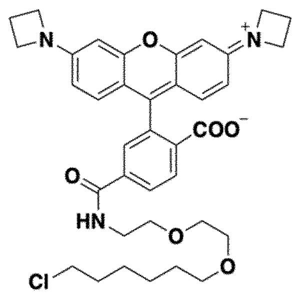

**JF549-HTL**

Comparative Example 8 Compound JFX554-HTL

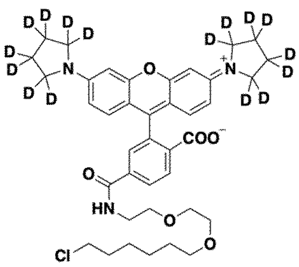

**JFX554-HTL**

Comparative Example 9 Compound CPY-HTL

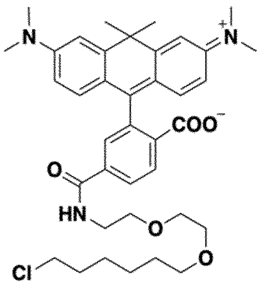

CPY-HTL

Comparative Example 10 Compound TMR-STL

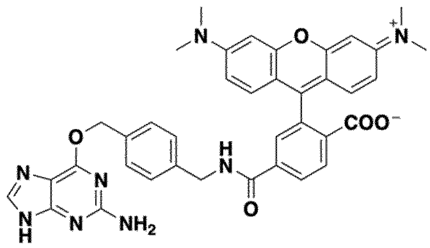

TMR-STL

Comparative Example 11 Compound SiR-HTL

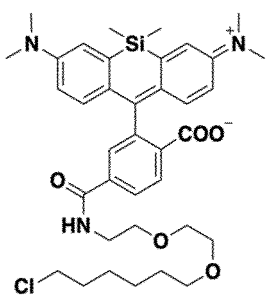

SiR-HTL

Test Example 1

Determination of basic photophysical properties of fluorescent dyes

Basic photophysical properties of the fluorescent dyes in Example 1 (A528), Example 2 (A532), Example 3 (A555), Example 4 (A586), Example 6 (A620), Example 7 (A634), Example 9 (A655), Example 18 (A720), Example 20 (B350) and Example 21 (B356) were determined, as shown in Fig. 1 and Fig. 2.

Method for determining basic photophysical properties of a fluorescent dye: A fluorescent dye was prepared into a solution with a concentration of 1 μmol/L, where a PBS buffer solution (phosphate buffer solution) with a pH value of 7.4 was used as a solvent; and then, the solution was tested in a Duetta fluorescence and absorption spectrometer at 25°C for 3 times.

Method for determining the fluorescence quantum yield of a fluorescent dye: The fluorescence quantum yield was determined by a reference method, a fluorescent dye to be tested or a reference fluorescent dye was prepared into a solution with a concentration of 1 μmol/L, where a PBS buffer solution (phosphate buffer solution) with a pH value of 7.4 was used as a solvent; and then, the solution was scanned in a Duetta fluorescence and absorption spectrometer at 25°C to obtain an absorption spectrum and a fluorescence spectrum, and the fluorescence spectrum was integrated, where the fluorescence quantum yield of the dye was obtained based on a ratio of fluorescence spectrum integrals of the fluorescent dye to be tested and the reference fluorescent dye. A formula for calculating the fluorescence quantum yield of a fluorescent dye is as follows:

$$\frac{\Phi_{f,sample}}{\Phi_{f,ref}} = \frac{OD_{ref} \cdot I_{sample} \cdot d_{sample}^2}{OD_{sample} \cdot I_{ref} \cdot d_{ref}^2}$$

where $\Phi_{sample}$ and $\Phi_{ref}$ represent a fluorescence quantum yield of the fluorescent dye to be tested and the reference fluorescent dye, respectively;

$OD_{sample}$ and $OD_{ref}$ represent an absorption value of the fluorescent dye to be tested and the reference fluorescent dye at an excitation wavelength, respectively;

$I_{sample}$ and $I_{ref}$ represent an integral value of an excitation spectrum of the fluorescent dye to be tested and the reference fluorescent dye, respectively;

$d_{sample}$ and $d_{ref}$ represent a refractive index of a test solvent of the fluorescent dye to be tested and the reference fluorescent dye, respectively.

As can be seen from Table 1, a class of aza[3.2.1]bridged ring substituted fluorescent dyes obtained in the present disclosure have an emission wavelength within ultraviolet and visible light regions, and the brightness of the fluorescent dyes of various colors is 1.2-16 times higher than that of traditional dimethylamino or diethylamino substituted fluorescent dyes. The dyes with emissions in ultraviolet, orange and red light regions have quantum yields similar to theoretical limits.

Table 1

| Matrix | Substituen t $NR_2$= | Compound name | $\lambda_{Abs}$ (nm) | $\lambda_{Em}$ (nm) | Φ |
|---|---|---|---|---|---|
| 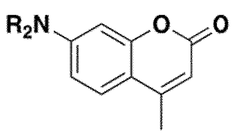 | 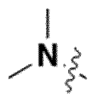 | Dimethylcouma rin | 372 | 470 | 0.19 |
| | 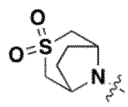 | B350 (Example 20) | 350 | 440 | 0.96 |
| | 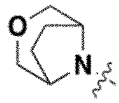 | B356 (Example 21) | 356 | 472 | 0.72 |
| 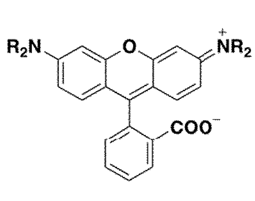 | 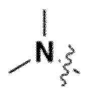 | TMR | 548 | 572 | 0.41 |
| | 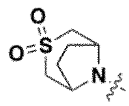 | A528 (Example 1) | 528 | 552 | 0.94 |
| | 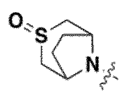 | A532 (Example 2) | 532 | 558 | 0.92 |
| | 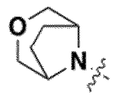 | A555 (Example 3) | 555 | 583 | 0.78 |

(continued)

| Matrix | Substituen t $NR_2$= | Compound name | $\lambda_{Abs}$ (nm) | $\lambda_{Em}$ (nm) | $\Phi$ |
|---|---|---|---|---|---|
| 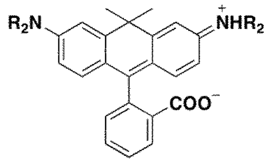 | 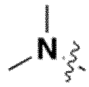 | CPY | 606 | 626 | 0.52 |
| | 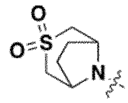 | A586 (Example 4) | 588 | 613 | 0.92 |
| | 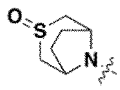 | A591 (Example 5) | 593 | 620 | 0.87 |
| | 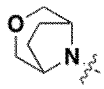 | A620 (Example 6) | 615 | 645 | 0.64 |
| 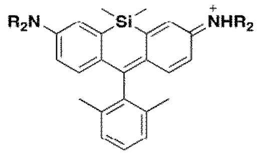 | 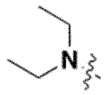 | SiRhodB | 655 | 674 | 0.21 |
| | 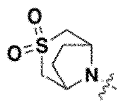 | A634 (Example 7) | 634 | 652 | 0.78 |
| 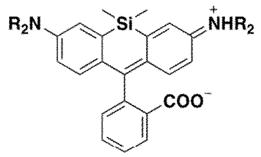 | 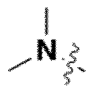 | SiR | 643 | 662 | 0.41 |
| | 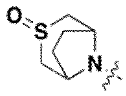 | A640 (Example 8) | 640 | 660 | 0.72 |
| | 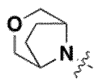 | A655 (Example 9) | 657 | 680 | 0.31 |
| 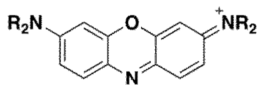 | 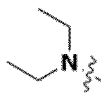 | oxazine | 655 | 669 | 0.07 |
| | 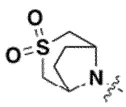 | C622 (Example 22) | 622 | 646 | 0.47 |
| | 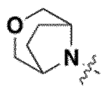 | C654 (Example 23) | 654 | 691 | 0.21 |
| 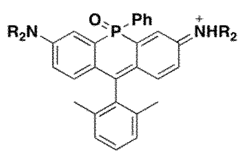 | 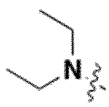 | PRhodB | 708 | 734 | 0.22 |
| | 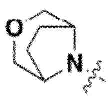 | A720 (Example 18) | 714 | 747 | 0.15 |

Test Example 2 Determination of the photostability of fluorescent dyes

The photostability of the fluorescent dyes in Example 3 (A555), Example 10 (A528-6-COOH), Example 7 (A634), Comparative Example 2 (TMRE), Comparative Example 1 (JF549), Comparative Example 3 (Rhodamine B), Comparative Example 4 (Cy3) and Comparative Example 5 (Atto647N) was determined, as shown in Fig. 3 and Fig. 4.
Method for determining the photostability of a fluorescent dye: A fluorescent dye with a concentration of 10 mol/L was dissolved in a PBS buffer solution (phosphate buffer solution) with a pH value of 7.4; and then, the dye solution was irradiated with an LED lamp (light-emitting diode) having an excitation wavelength matched with that of the fluorescent dye, and measured once after the irradiation for 10 min to obtain an absorption spectrum, where the measurement was performed for a total of 30 min.
As can be seen from Fig.3, in an orange channel, the stability in Example 3 and Example 10 is improved by 3.3-7.6 times compared with that in Comparative Example 2, Comparative Example 1, Comparative Example 3 and Comparative Example 4. In addition, the fluorescent dyes in Comparative Example 2, Comparative Example 1, Comparative Example 3 and Comparative Example 4 have the phenomenon of blue shift of absorption spectra in 12-22 nm, while the fluorescent dyes in Example 3 and Example 10 of the present disclosure have no phenomenon of spectral blue shift.
As can be seen from Fig. 4, in a far-red channel, the photostability in Example 7 of the present disclosure is improved by 1 time compared with that in Comparative Example 5, and the phenomenon of spectral blue shift is not observed.
By combining Fig. 3 and Fig. 4, it is shown that the photostability of the aza[3.2.1]bridged ring substituted fluorescent dye prepared in the present disclosure is greatly improved, and a photooxidation dealkylation (spectral blue shift) process is completely avoided.

Test Example 3

Determination of functionalized derivatives of fluorescent dyes in a single molecule experiment of fixed cells

The functionalized derivatives of fluorescent dyes in Example 27 (A555-HTL), Comparative Example 6 (TMR-HTL) and Comparative Example 7 (JF549-HTL) were determined in a single molecule experiment of fixed cells, as shown in Fig. 5, Fig. 6 and Fig. 7.
Method for determining a functionalized derivative of a fluorescent dye in a single molecule experiment of fixed cells: 5 pM of a fluorescent dye and U2OS cells were co-incubated for 30 min; then, the cells were washed with a PBS buffer solution with a pH value of 7.4 for three times, and fixed with 4% paraformaldehyde; and a single molecule experiment was carried out on a total internal reflection fluorescence microscope (100 mW, excitation at 561 nm, and excitation intensity: 6%).
As can be seen from Fig. 6, the average single molecule fluorescence intensity in Comparative Example 6, Comparative Example 7 and Example 27 is 4,354, 5,466 and 8,176, respectively, and the photostability in Example 27 is improved by one time compared with that in Comparative Example 7.
By combining Fig. 5, Fig. 6 and Fig. 7, it is shown that the aza[3.2.1]bridged ring substituted fluorescent dye prepared in the present disclosure can not only be used in a single molecule imaging experiment, but also has good performance in brightness and photostability.

Test Example 4

Determination of functionalized derivatives of fluorescent dyes in a confocal microscopy imaging experiment of living cells

The functionalized derivatives of fluorescent dyes in Example 27 (A555-HTL), Example 31 (A620-HTL), Example 34 (A655-HTL), Example 28 (A555-STL), Comparative Example 6 (TMR-HTL), Comparative Example 8 (JFX554-HTL), Comparative Example 9 (CPY-HTL) and Comparative Example 10 (TMR-STL) were determined in a confocal microscopy imaging experiment of living cells, as shown in Fig. 8, Fig. 9, Fig. 10, Fig. 11, Fig. 12, Fig. 13 and Fig. 14.
Method for determining a functionalized derivative of a fluorescent dye in a confocal microscopy imaging experiment of living cells: A 200 nM fluorescent dye was used to perform confocal fluorescence imaging on TMP-Halo-SNAP-GFP-H2B proteins in living Hela cells, and compared with a traditional dye of the same color in brightness.
As can be seen from Fig. 11, the brightness in Example 27 is 50% higher than that of a high-brightness dye reported in a document JACS Au. 2021, 1:690-696., namely, in Comparative Example 8, and is 158% higher than that in Comparative Example 6 (confocal imaging at an excitation wavelength of 561 nm and an excitation intensity of 10%, a detector at 566 nm, and no gate).
As can be seen from Fig. 12, the brightness in Example 31 is 350% higher than that of a high-brightness dye

reported in a document Angew. Chem. Int. Ed. 2016, 55: 3290-3294, namely, in Comparative Example 9 (confocal imaging at an excitation wavelength of 640 nm and an excitation intensity of 5%, a detector at 664 nm, and no gate).

As can be seen from Fig. 14, the brightness in Example 28 is 223% higher than that in Comparative Example 10 (confocal imaging at an excitation wavelength of 561 nm and an excitation intensity of 10%, a detector at 566 nm, and no gate).

Test Example 5

Determination of functional derivatives of fluorescent dyes in a super-resolution microscopy imaging experiment of living cells

The functional derivatives of fluorescent dyes in Example 34 (A655-HTL) and Comparative Example 11 (SiR-HTL) were determined in a super-resolution microscopy imaging experiment of living cells, as shown in Fig. 15 and Fig. 16.

Method for determining a functional derivative of a fluorescent dye in a super-resolution microscopy imaging experiment of living cells: A 1 μM functional derivative of a fluorescent dye was used to perform wash-free super-resolution imaging on Lifeact-Halo proteins in living Hela cells (STED imaging at an excitation wavelength of 640 nm, an excitation intensity of 2% and a depletion light intensity of 30%, a detector at 650 nm, and no gate).

By combining Fig. 15 and Fig. 16, it can be seen that compared with a far-red wash-free dye reported in a document Nature Chemistry. 2013, 5: 132-139., namely, in Comparative Example 11, the dye in Example 34 has higher photostability on STED.

As can be seen from the above descriptions, the above embodiments of the present disclosure have the following technical effects. According to the aza[3.2.1]bridged ring substituted fluorescent dye provided by the present disclosure, by adopting an aza[3.2.1]bridged ring as a substituent of the fluorescent dye, not only can the brightness of the fluorescent dye be improved, but also the photostability of the fluorescent dye is improved while spectral blue shift of the dye is avoided. Meanwhile, the water solubility of the fluorescent dye can be effectively improved, and good membrane permeability of the fluorescent dye is maintained. The fluorescence quantum yield, photostability, resistance to photooxidation dealkylation (spectral blue shift), water solubility and membrane permeability of the fluorescent dye can be improved simultaneously.

The above descriptions are only preferred embodiments of the present disclosure and are not intended to limit the present disclosure. For persons skilled in the art, various alterations and changes of the present disclosure may be made. Any modifications, equivalent substitutions, improvements and the like made within the spirit and principles of the present disclosure shall be included in the scope of protection of the present disclosure.

## Claims

**1.** An aza[3.2.1]bridged ring substituted fluorescent dye, wherein the aza[3.2.1]bridged ring substituted fluorescent dye has a structure shown in Formula (I), Formula (II) or Formula (III) below:

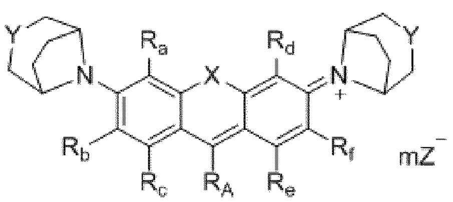

Formula (I)

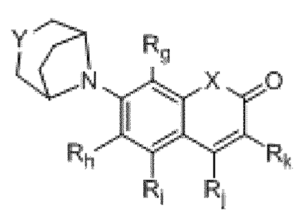

Formula (II)

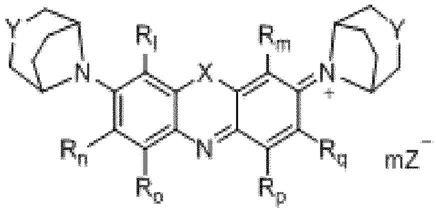

Formula (III)

$R_a$, $R_b$, $R_c$, $R_d$, $R_e$, $R_f$, $R_g$, $R_h$, $R_i$, $R_j$, $R_k$, $R_l$, $R_m$, $R_n$, $R_o$, $R_p$ and $R_q$ are independently selected from hydrogen, halogen, carboxyl, substituted or unsubstituted $C_1$-$C_{20}$ alkyl, substituted or unsubstituted $C_3$-$C_{20}$ cycloalkyl, substituted or unsubstituted $C_4$-$C_{20}$ cycloalkylalkyl, $C_4$-$C_{20}$ alkylcycloalkyl, substituted or unsubstituted $C_1$-$C_{20}$ alkoxyl, substituted or unsubstituted $C_1$-$C_{20}$ ester group, and substituted or unsubstituted $C_1$-$C_{20}$ acylamino;
$R_A$ is selected from substituted or unsubstituted $C_6$-$C_{40}$ aryl, substituted or unsubstituted $C_6$-$C_{40}$ aralkyl, and substituted or unsubstituted $C_5$-$C_{40}$ heteroaryl;
X is selected from oxygen, sulfur, substituted or unsubstituted $C_1$-$C_{20}$ alkylene,

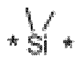, 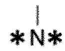,

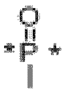, 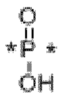, 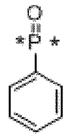, and 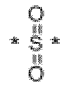;

Y is selected from oxygen, sulfur, nitrogen, substituted or unsubstituted $C_1$-$C_{20}$ alkylene, substituted or unsubstituted $C_1$-$C_{20}$ alkylenoxyl, substituted or unsubstituted $C_1$-$C_{20}$ acyl, substituted or unsubstituted $C_1$-$C_{20}$ ester group,

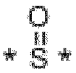, 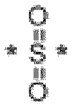, 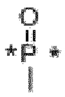, 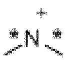, and 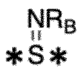,

wherein $R_B$ is selected from substituted or unsubstituted $C_1$-$C_{20}$ alkyl, substituted or unsubstituted $C_1$-$C_{20}$ alkoxyl, substituted or unsubstituted $C_1$-$C_{20}$ acyl, and substituted or unsubstituted $C_1$-$C_{20}$ ester group;
$Z^-$ is selected from $F^-$, $Cl^-$, Br, $I^-$, $OAc^-$, $HSO_4^-$, $H_2PO_4^-$, $ClO_4^-$, $CF_3COO^-$, $CF_3SO_3^-$, $CH_3SO_3^-$, $NO_3^-$, $C_5H_7O_5COO^-$; and $PF_6^-$; and m is an integer ranged from 1 to 3.

**2.** The aza[3.2.1]bridged ring substituted fluorescent dye according to claim 1, wherein the $R_a$, the $R_b$, the $R_c$, the $R_d$, the $R_e$, the $R_f$, the $R_g$, the $R_h$, the $R_i$, the $R_j$, the $R_k$, the $R_l$, the $R_m$, the $R_n$, the $R_o$, the $R_p$ and the $R_q$ are independently selected from hydrogen, halogen, carboxyl, substituted or unsubstituted $C_1$-$C_{10}$ alkyl, substituted or unsubstituted $C_3$-$C_{10}$ cycloalkyl, substituted or unsubstituted $C_4$-$C_{10}$ cycloalkylalkyl, $C_4$-$C_{10}$ alkylcycloalkyl, substituted or unsubstituted $C_1$-$C_{10}$ alkoxyl, substituted or unsubstituted $C_1$-$C_{10}$ ester group, and substituted or unsubstituted $C_1$-$C_{10}$ acylamino;

and/or, the $R_A$ is selected from substituted or unsubstituted $C_6$-$C_{30}$ aryl, substituted or unsubstituted $C_6$-$C_{30}$ aralkyl, and substituted or unsubstituted $C_5$-$C_{30}$ heteroaryl;
and/or, the X is selected from oxygen, sulfur, substituted or unsubstituted $C_1$-$C_{10}$ alkyl,

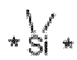,

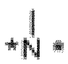, 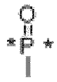, 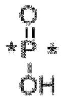, 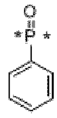, and 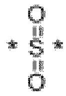;

and/or, the Y is selected from oxygen, sulfur, nitrogen, substituted or unsubstituted $C_1$-$C_{10}$ alkyl, substituted or unsubstituted $C_1$-$C_{10}$ alkoxyl, substituted or unsubstituted $C_1$-$C_{10}$ acyl, substituted or unsubstituted $C_1$-$C_{10}$ ester group,

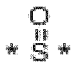, 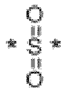, 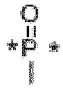, 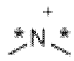, and 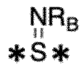,

wherein the $R_B$ is selected from substituted or unsubstituted $C_1$-$C_{20}$ alkyl, substituted or unsubstituted $C_1$-$C_{20}$ alkoxyl, substituted or unsubstituted $C_1$-$C_{20}$ acyl, and substituted or unsubstituted $C_1$-$C_{20}$ ester group;
and/or, the $Z^-$ is selected from $F^-$, $Cl^-$, Br, $I^-$, $OAc^-$, $HSO_4^-$, $H_2PO_4^-$, $ClO_4^-$, $CF_3COO^-$, $CF_3SO_3^-$, $CH_3SO_3^-$, $NO_3^-$, $C_5H_7O_5COO^-$, and $PF_6^-$;
and/or, the m is an integer ranged from 1 to 3.

**3.** The aza[3.2.1]bridged ring substituted fluorescent dye according to claim 1 or 2, wherein the aza[3.2.1]bridged ring substituted fluorescent dye shown in Formula (I) has a structure shown in Formula (IV) below:

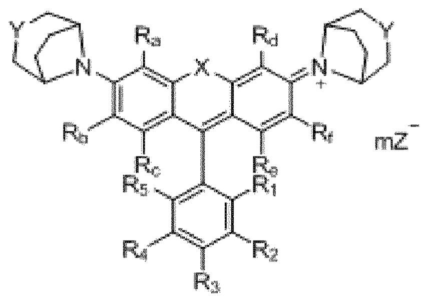

Formula (IV)

$R_1$ and $R_5$ are independently selected from hydrogen, carboxyl, halogen, substituted or unsubstituted $C_1$-$C_6$ alkyl, substituted or unsubstituted $C_1$-$C_6$ alkoxyl, nitro, amino, hydroxyl,

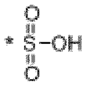, 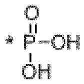, 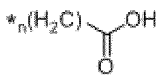, 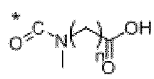, 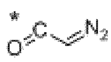, 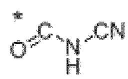, 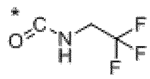,

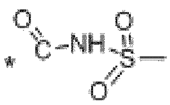, 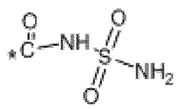, 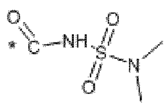, 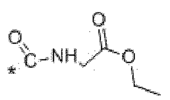, and 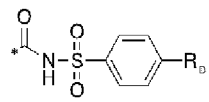,

wherein $R_D$ is selected from hydrogen, halogen, cyano, substituted or unsubstituted $C_1$-$C_6$ alkyl, and substituted or unsubstituted $C_1$-$C_6$ alkoxyl, and n is an integer ranged from 1 to 6; and $R_2$, $R_3$ and $R_4$ are independently selected from hydrogen, halogen, carboxyl, substituted or unsubstituted $C_1$-$C_{10}$ alkyl, substituted or unsubstituted $C_3$-$C_{10}$ cycloalkyl, substituted or unsubstituted $C_4$-$C_{10}$ cycloalkylalkyl, $C_4$-$C_{10}$ alkylcycloalkyl, substituted or unsubstituted $C_1$-$C_{10}$ alkoxyl, substituted or unsubstituted $C_1$-$C_{10}$ ester group,

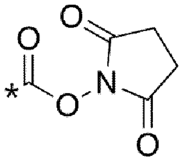, 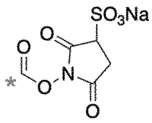, 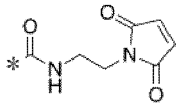, 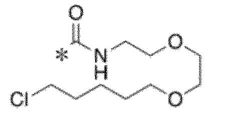,

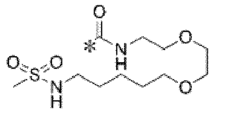, 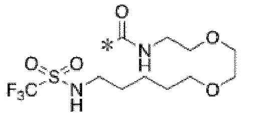, 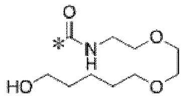, 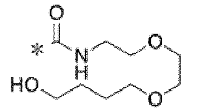,

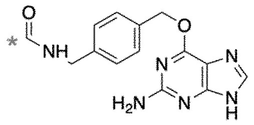, 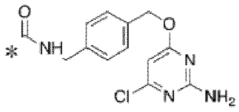, 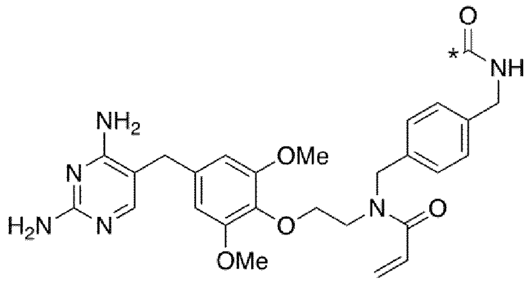,

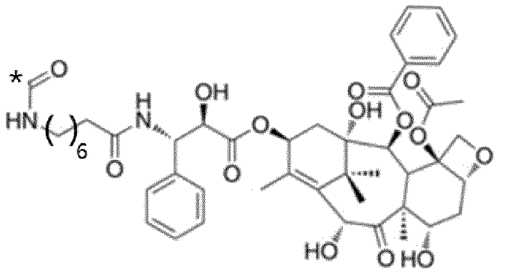, 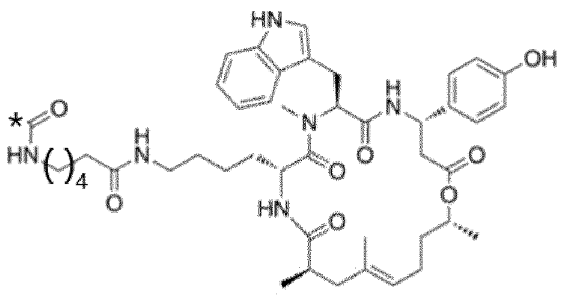,

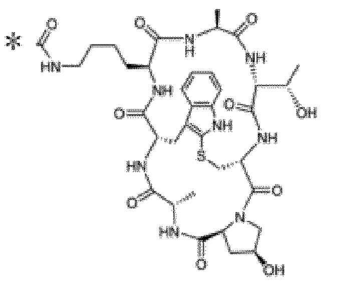, 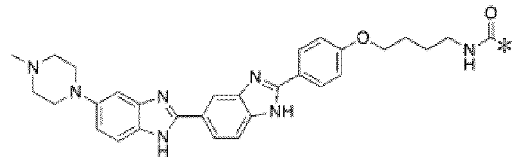

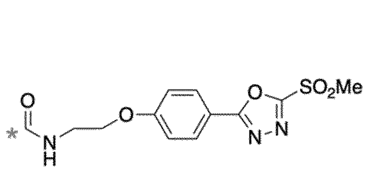, 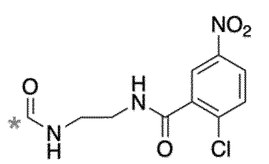, 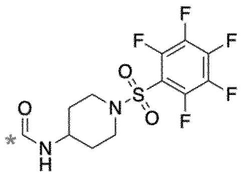,

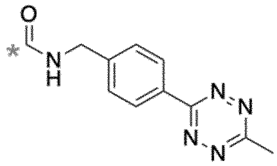

,

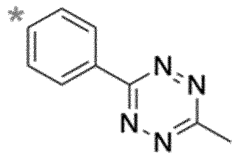

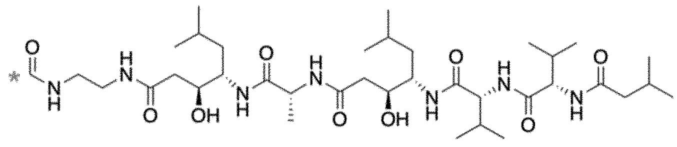

,

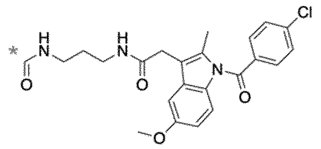

,

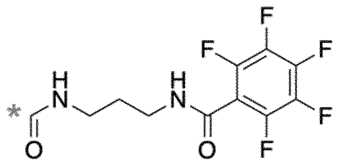

,

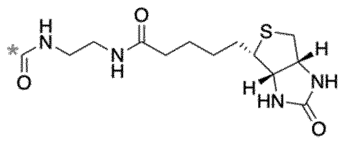

, and

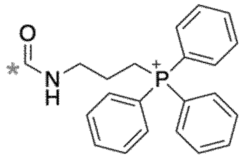

.

**4.** The aza[3.2.1]bridged ring substituted fluorescent dye according to claim 3, wherein the aza[3.2.1]bridged ring substituted fluorescent dye is selected from at least one of the following formulas IA1-IA3, IIA1-IIA3, IIIA1-IIIA3, IB1-IB3, IC1-IC3, ID1-ID3, IE1-IE3, IF1-IF3, and IG1-IG3:

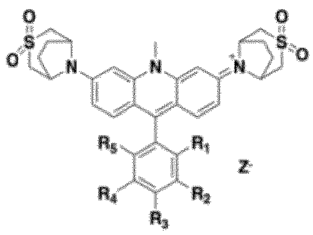

**IA1**

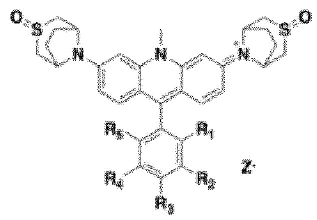

**IA2**

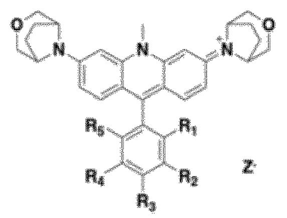

**IA3**

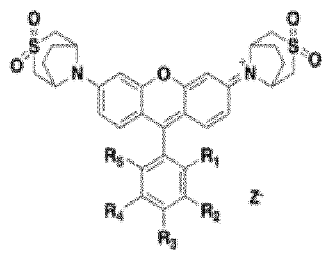

**IB1**

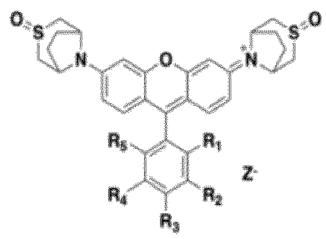

**IB2**

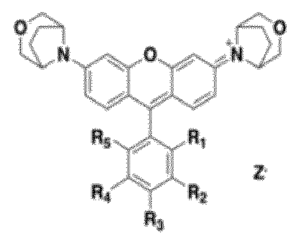

**IB3**

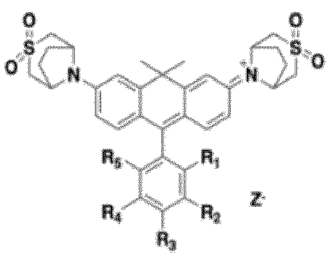

**IC1**

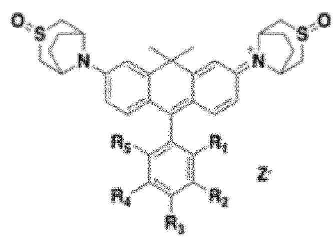

**IC2**

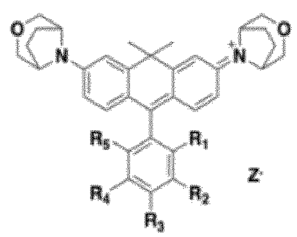

**IC3**

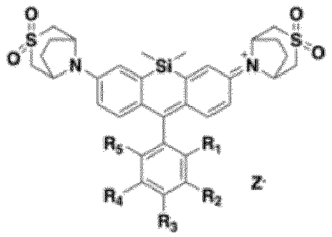

ID1

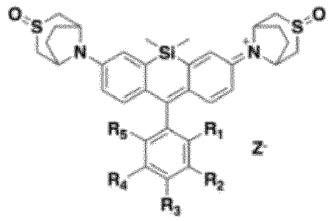

ID2

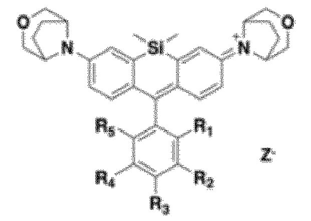

ID3

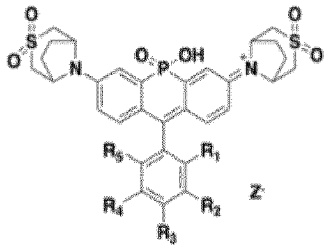

IE1

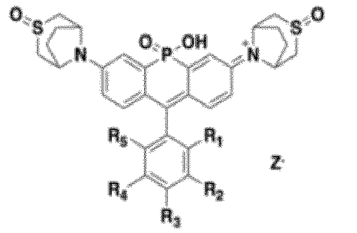

IE2

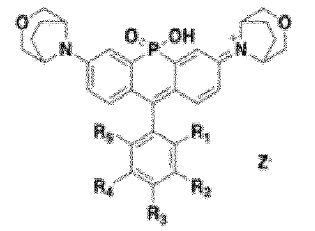

IE3

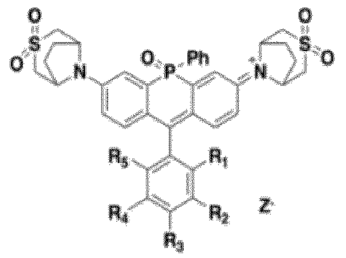

IF1

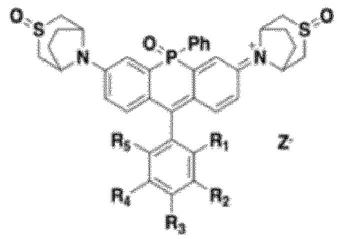

IF2

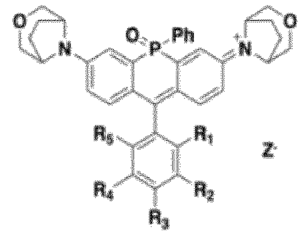

IF3

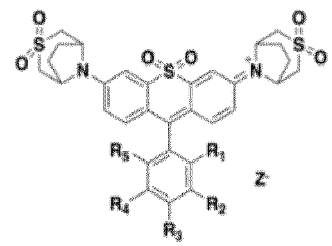

IG1

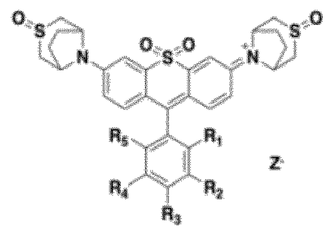

IG2

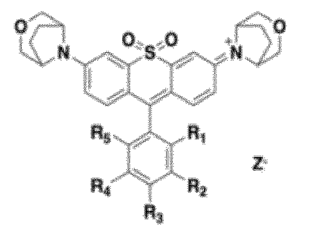

IG3

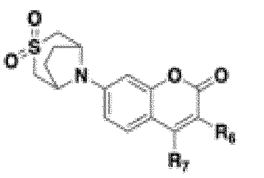

IIA1

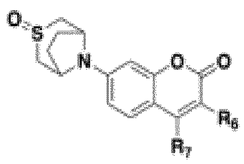

IIA2

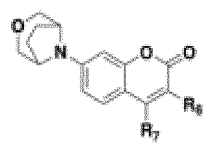

IIA3

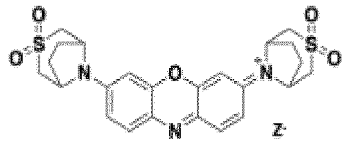

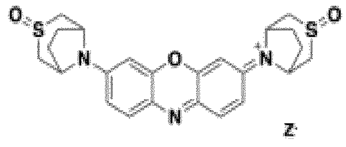

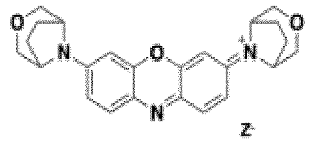

IIIA1 IIIA2 IIIA3

.

**5.** The aza[3.2.1]bridged ring substituted fluorescent dye according to any one of claims 1 to 4, wherein the aza[3.2.1] bridged ring substituted fluorescent dye is selected from at least one of the following formulas A528, A532, A555, A586, A591, A620, A634, A640, A655, A720, A586-2Me, A586-2Me-5-COOH, A528-6-COOH, A532-6-COOH, A555-6-COOH, A586-6-COOH, A591-6-COOH, A620-6-COOH, A634-5-COOH, A640-6-COOH, A655-6-COOH, A528-HTL, A532-HTL, A555-HTL, A586-HTL, A620-HTL, A655-HTL, A555-STL, A528-NHS, A620-STL, A655-STL, A634-NHS, A586-NHS, A591-HTL, A640-HTL, A555-TMP3, A620-TMP3, A655-TMP3, A655-4-Hoechst, A591-TMP3, A640-TMP3, A591-STL, A640-STL, A528-NCN-HTL, A555-NCN-HTL, A620-NCN-HTL, B350, B356, C622, and C654:

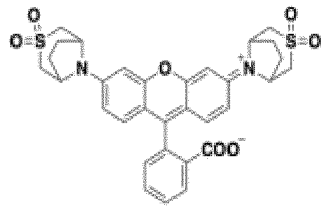

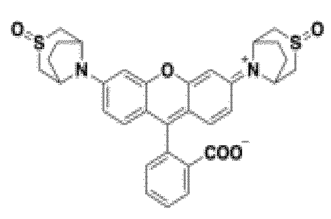

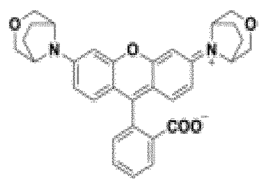

A528 A532 A555

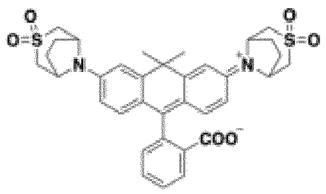

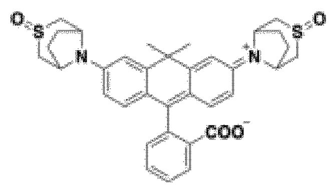

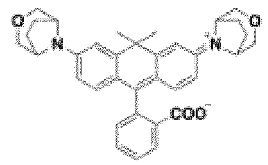

A586 A591 A620

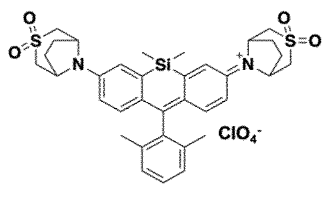

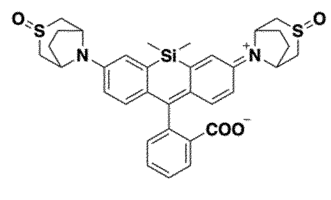

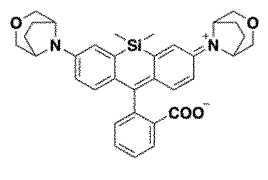

A634 A640 A655

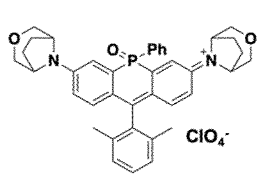

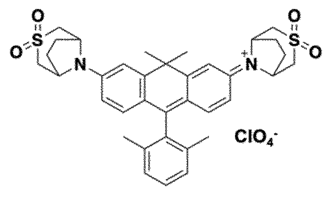

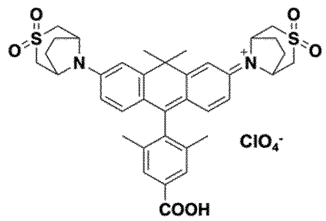

A720 A586-2Me A586-2Me-5-COOH

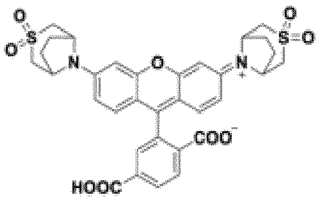

A528-6-COOH

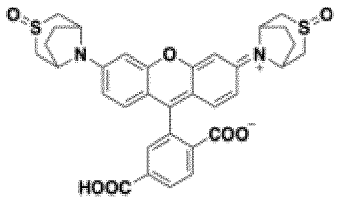

A532-6-COOH

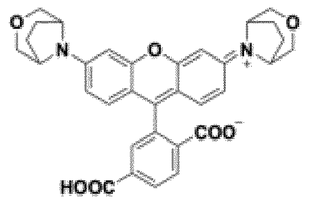

A555-6-COOH

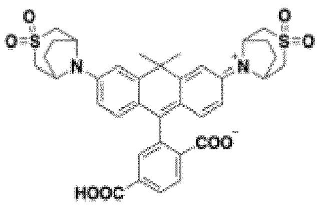

A586-6-COOH

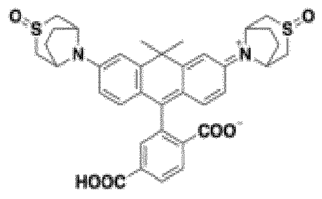

A591-6-COOH

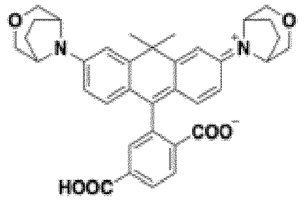

A620-6-COOH

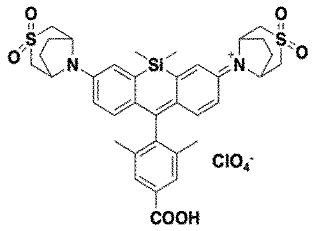

A634-5-COOH

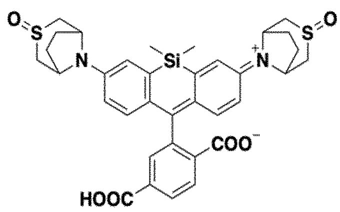

A640-6-COOH

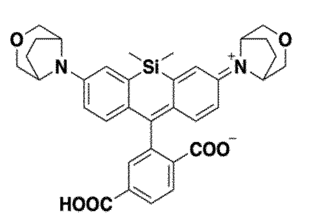

A655-6-COOH

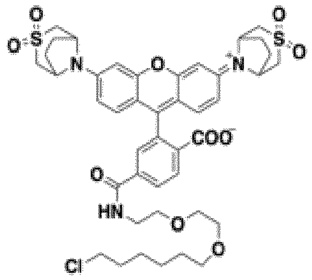

A528-HTL

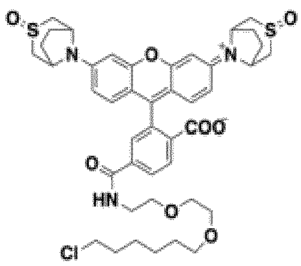

A532-HTL

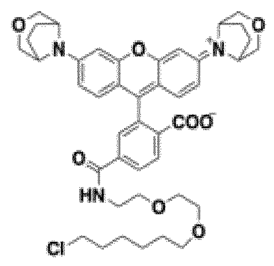

A555-HTL

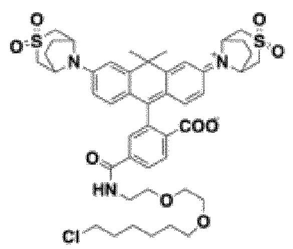

A586-HTL

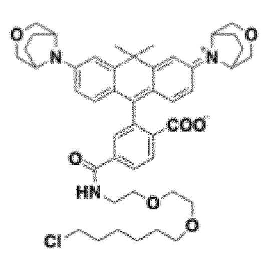

A620-HTL

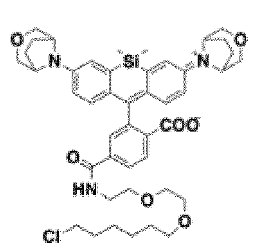

A655-HTL

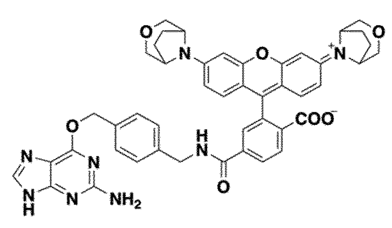

BD528-STL

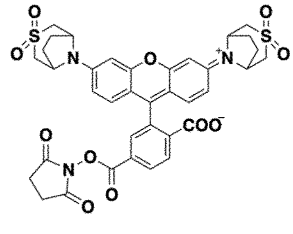

BD528-NHS

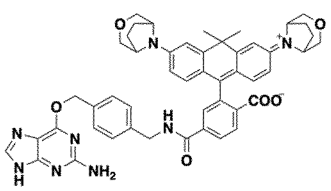

BD620-STL

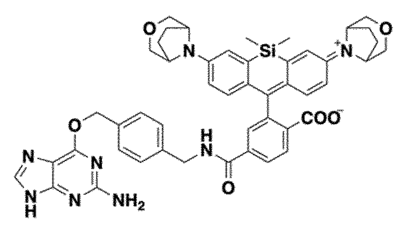

BD655-STL

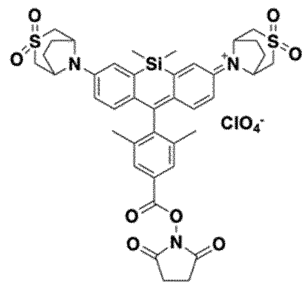

A634-NHS

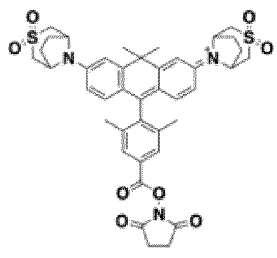

A586-NHS

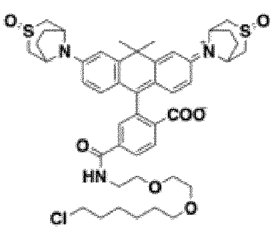

A591-HTL

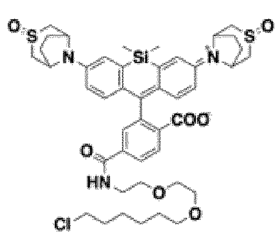

A640-HTL

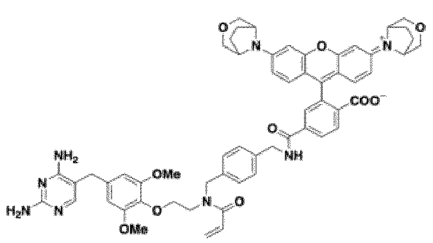

A555-TMP3

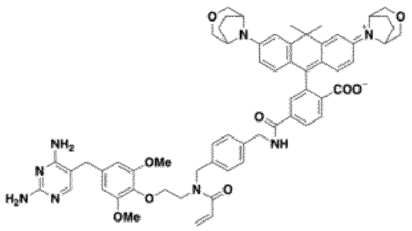

A620-TMP3

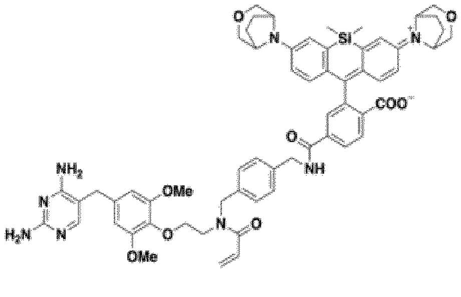

A655-TMP3

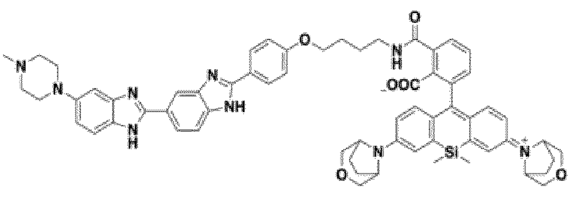

A655-4-Hoechst

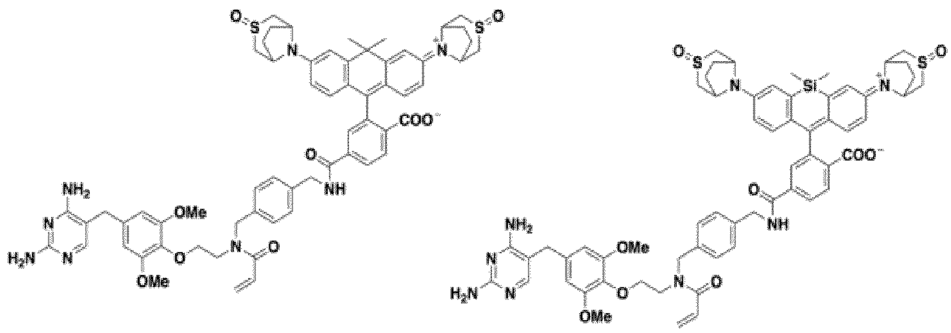

A591-TMP3 A640-TMP3

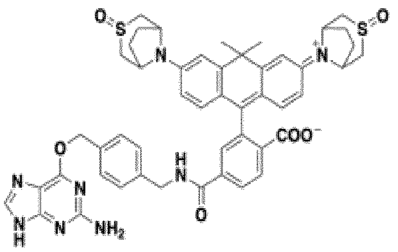

A591-STL

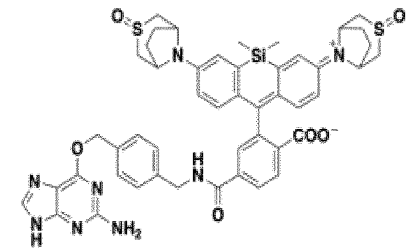

A640-STL

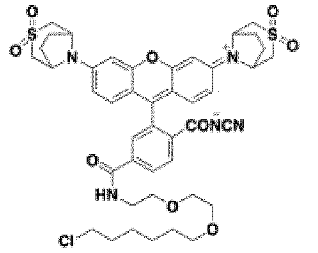

A528-NCN-HTL

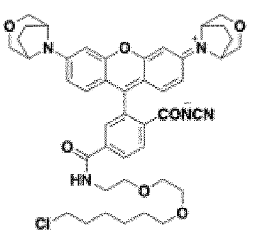

A555-NCN-HTL

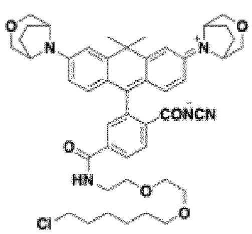

A620-NCN-HTL

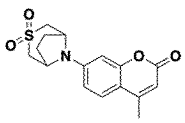

B350

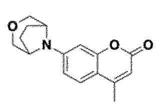

B356

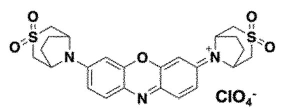

C622

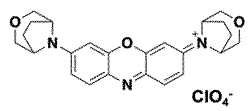

C654

.

**6.** A method for preparing an aza[3.2.1]bridged ring substituted fluorescent dye, wherein the preparation method comprises:

step S11, mixing a compound A, an aza[3.2.1]bridged ring compound and a first catalyst in a first reaction solvent to carry out a reaction so as to obtain a compound B; and

step S12, mixing the compound B with an HZ aqueous solution to carry out a reaction so as to obtain the aza[3.2.1]bridged ring substituted fluorescent dye shown in Formula (I);

the compound A has a structure shown in Formula (V) below, and the aza[3.2.1]bridged ring compound has a structure shown in Formula (VI) below:

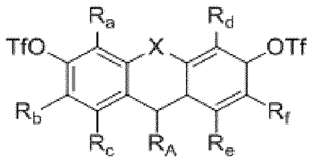

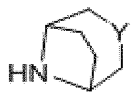

Formula (V) Formula (VI)

wherein $R_A$, $R_a$, $R_b$, $R_c$, $R_d$, $R_e$, $R_f$, X and Y independently have the same meaning as defined in any one of claims 1 to 5, and $Z^-$ in the HZ aqueous solution has the same meaning as defined in any one of claims 1 to 5;
preferably, the first catalyst comprises a palladium catalyst, a palladium catalyst ligand and an alkaline catalyst;
preferably, the palladium catalyst comprises at least one of tetrakis(triphenylphosphine)palladium, palladium acetate, tris(dibenzylideneacetone)dipalladium, [(diphenylphosphino)ferrocene]dichloropalladium, dichlorobis(triphenylphosphine) palladium and palladium chloride, preferably at least one of tetrakis(triphenylphosphine)palladium, palladium acetate or tris(dibenzylideneacetone)dipalladium;
preferably, the palladium catalyst ligand comprises at least one of bis(dibenzylideneacetone)palladium, 2,2'-bis(diphenylphosphino)-1,1'-binaphthyl, 2-dicyclohexylphosphino-2',4',6'-triisopropylbiphenyl and 2-dicyclohexylphosphino-2',6'-dimethoxybiphenyl, preferably at least one of bis(dibenzylideneacetone)palladium and 2,2'-bis(diphenylphosphino)-1,1'-binaphthyl;
preferably, the alkaline catalyst comprises at least one of cesium carbonate, sodium tert-butoxide, potassium carbonate, sodium carbonate, sodium methoxide, sodium ethoxide and potassium tert-butoxide, preferably at least one of cesium carbonate, sodium tert-butoxide and potassium tert-butoxide;
and preferably, the first reaction solvent comprises at least one of n-hexane, tetrahydrofuran, 1,4-dioxane, ethyl ether and toluene.

**7.** A method for preparing an aza[3.2.1]bridged ring substituted fluorescent dye, wherein the preparation method comprises:

mixing a compound C, an aza[3.2.1]bridged ring compound and a second catalyst in a second reaction solvent to carry out a reaction so as to obtain the aza[3.2.1]bridged ring substituted fluorescent dye shown in Formula (II);
the compound C has a structure shown in Formula (VII) below:

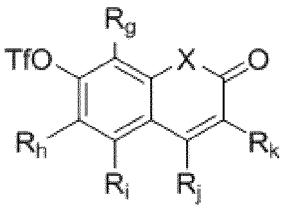

Formula (VII)

wherein $R_g$, $R_h$, $R_i$, $R_j$, $R_k$ and X independently have the same meaning as defined in any one of claims 1 to 5;
preferably, the second catalyst has the same meaning as the first catalyst;
and preferably, the second reaction solvent has the same meaning as the first reaction solvent.

**8.** A method for preparing an aza[3.2.1]bridged ring substituted fluorescent dye, wherein the preparation method comprises:

step S31, mixing a compound D, an aza[3.2.1]bridged ring compound and a third catalyst in a third reaction solvent to carry out a reaction so as to obtain a compound E;
step S32, mixing the compound E with an oxidizing agent in a fourth reaction solvent to carry out a reaction so as to obtain a compound F; and
step S33, mixing the compound F with an HZ aqueous solution to carry out a reaction so as to obtain the aza[3.2.1]bridged ring substituted fluorescent dye shown in Formula (III);
the compound D has a structure shown in Formula (VIII) below:

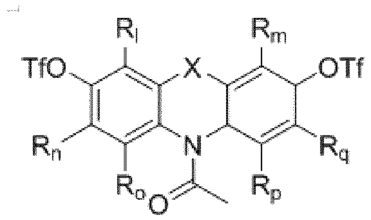

Formula (VIII)

wherein $R_l$, $R_m$, $R_n$, $R_o$, $R_p$, $R_q$, X and Y independently have the same meaning as defined in any one of claims 1 to 5, and $Z^-$ in the HZ aqueous solution has the same meaning as defined in any one of claims 1 to 5;
preferably, the third catalyst has the same meaning as the first catalyst;
preferably, the third reaction solvent has the same meaning as the first reaction solvent; preferably, in step S32, the fourth reaction solvent is a mixed solvent of dichloromethane and water, and the volume ratio of the dichloromethane to the water is (10-30):1;
preferably, in step S32, the oxidizing agent comprises at least one of 2,3-dichloro-5,6-dicyano-1,4-benzoquinone, potassium permanganate, hydrogen peroxide and m-chloroperoxybenzoic acid, preferably 2,3-dichloro-5,6-dicyano-1,4-benzoquinone;
and preferably, the molar ratio of the oxidizing agent to the compound E is (1.0-2.0):1.

**9.** Use of the aza[3.2.1]bridged ring substituted fluorescent dye according to any one of claims 1 to 5 or the aza[3.2.1] bridged ring substituted fluorescent dye prepared by the method for preparing an aza[3.2.1]bridged ring substituted fluorescent dye according to any one of claims 6 to 8 in the fields of fluorescence analysis, fluorescence detection and fluorescence imaging as a fluorescent marker.

**10.** The use of the aza[3.2.1]bridged ring substituted fluorescent dye according to claim 9, wherein the aza[3.2.1]bridged ring substituted fluorescent dye is used in fluorescence analysis, fluorescence detection, biomolecular labeling, immunofluorescence imaging, live-cell imaging, tissue imaging, in vivo imaging, single-molecule imaging and super-resolution fluorescence imaging as a fluorescent marker.

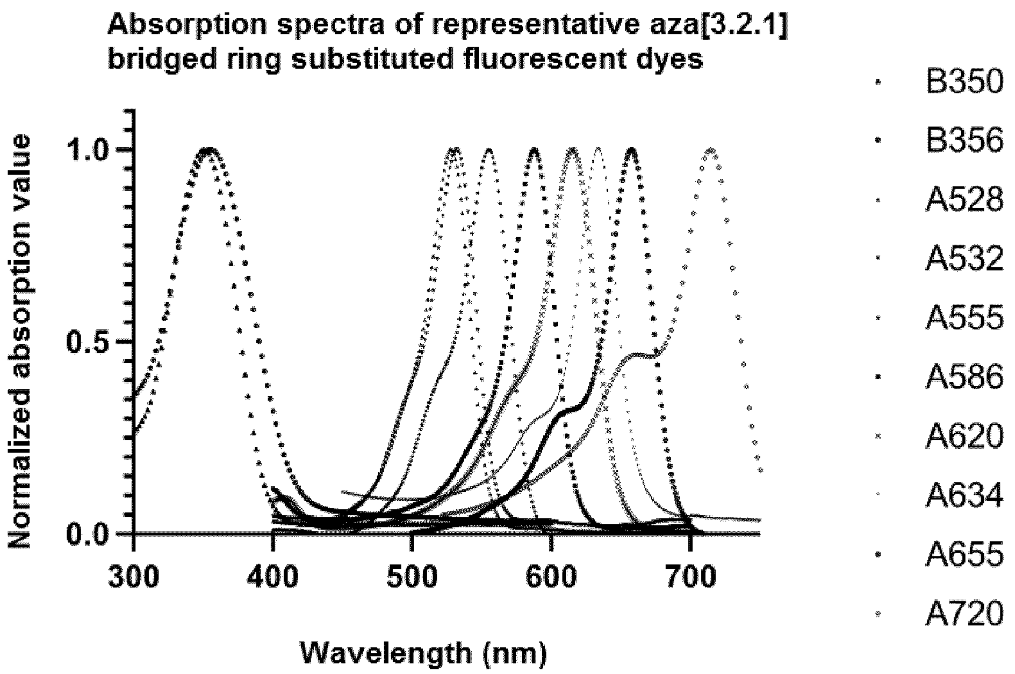
Absorption spectra of representative aza[3.2.1] bridged ring substituted fluorescent dyes
Normalized absorption value
1.0
0.5
0.0
300
400
500
600
700
Wavelength (nm)
B350
B356
A528
A532
A555
A586
A620
A634
A655
A720

Fig. 1

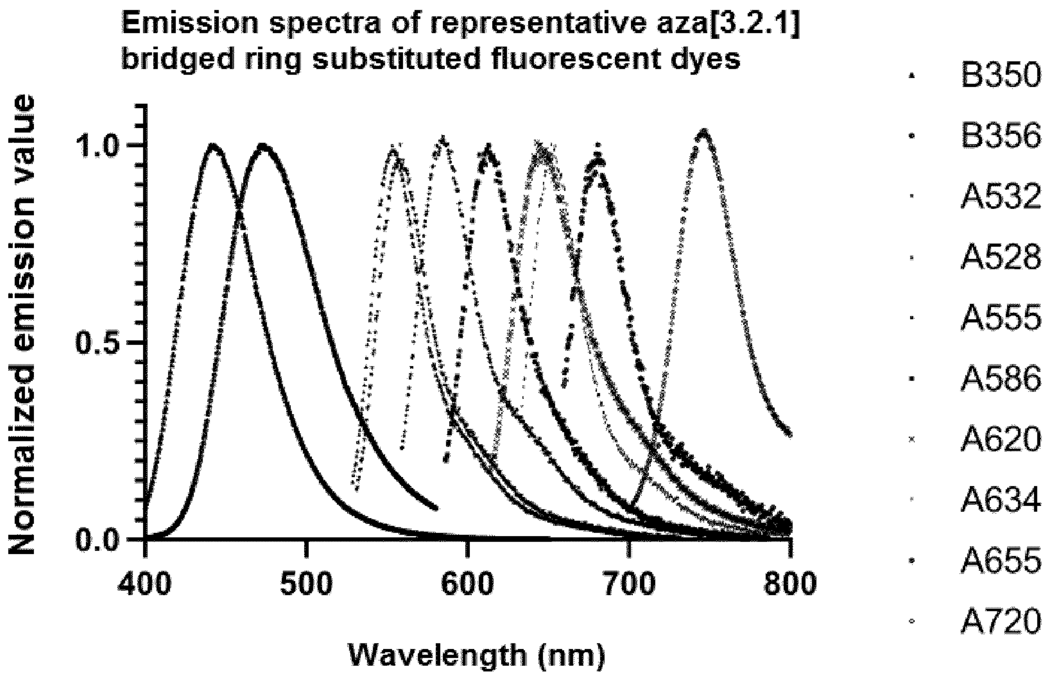
Emission spectra of representative aza[3.2.1] bridged ring substituted fluorescent dyes
Normalized emission value
1.0
0.5
0.0
400
500
600
700
800
Wavelength (nm)
B350
B356
A532
A528
A555
A586
A620
A634
A655
A720

Fig. 2

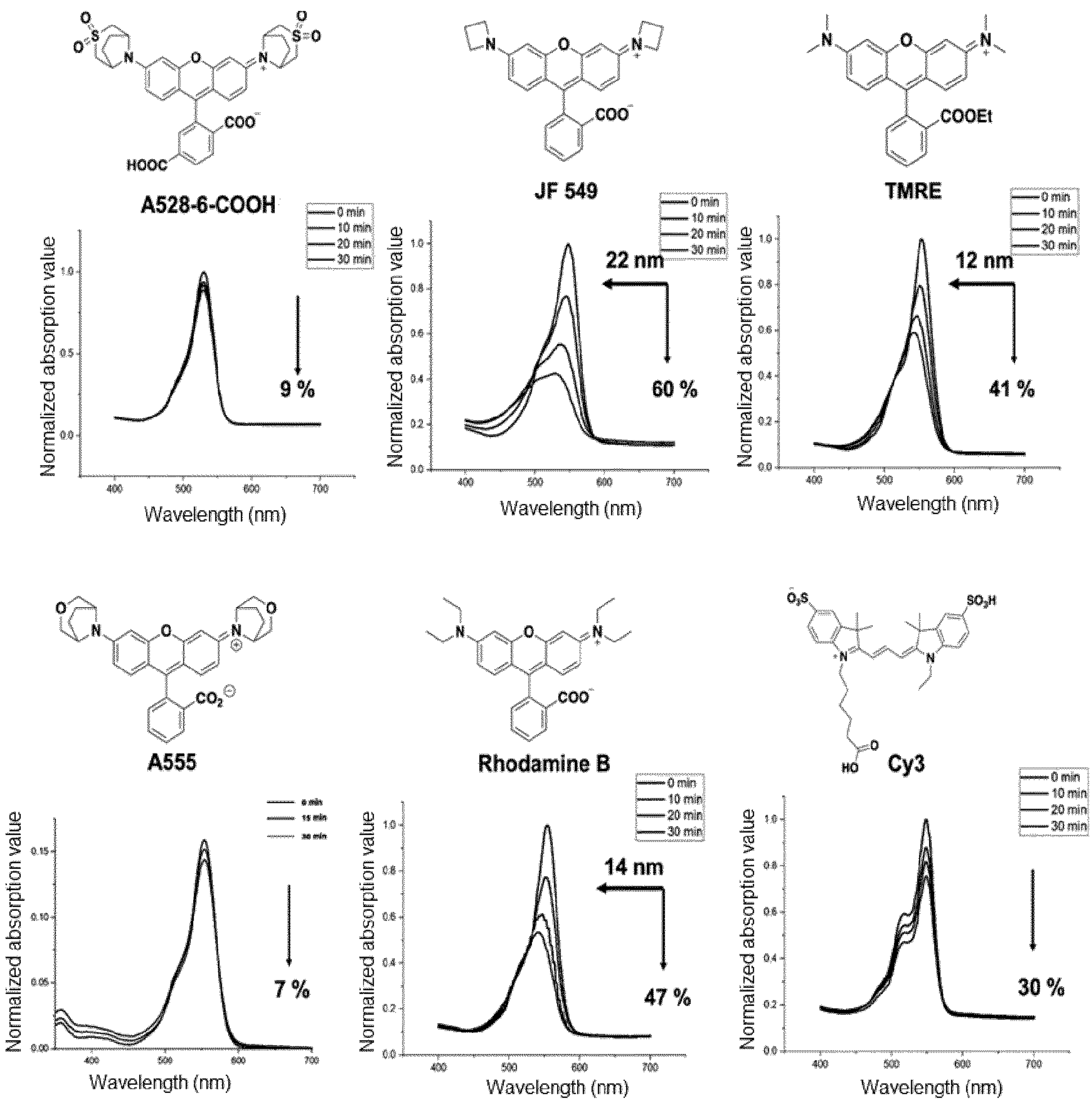
A528-6-COOH
JF 549
TMRE
A555
Rhodamine B
Cy3
0 min
10 min
20 min
30 min
9 %
22 nm
60 %
12 nm
41 %
7 %
14 nm
47 %
30 %
Normalized absorption value
Wavelength (nm)

Fig. 3

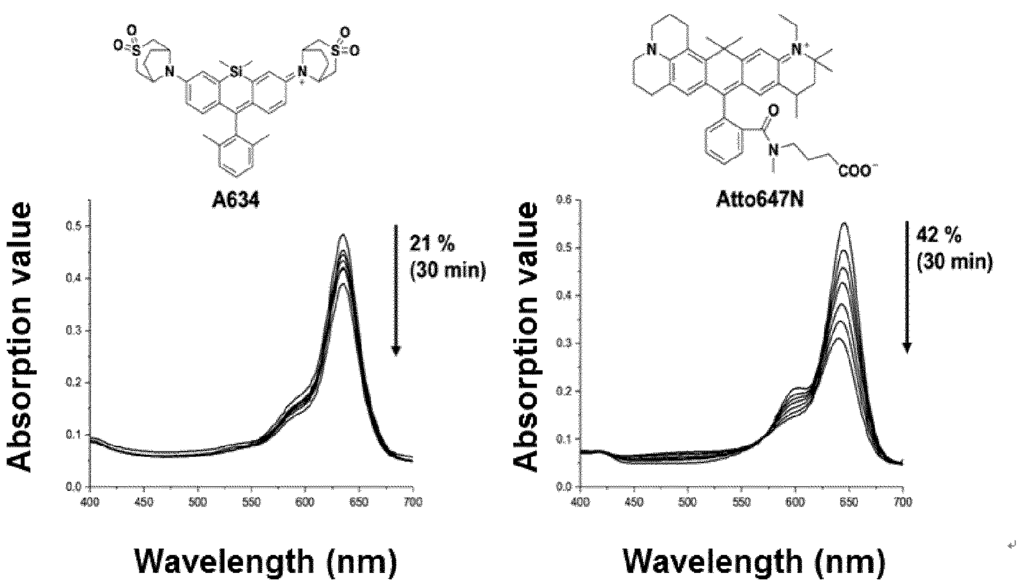
A634
Atto647N
Absorption value
21 %
(30 min)
42 %
(30 min)
Wavelength (nm)
Wavelength (nm)

Fig. 4

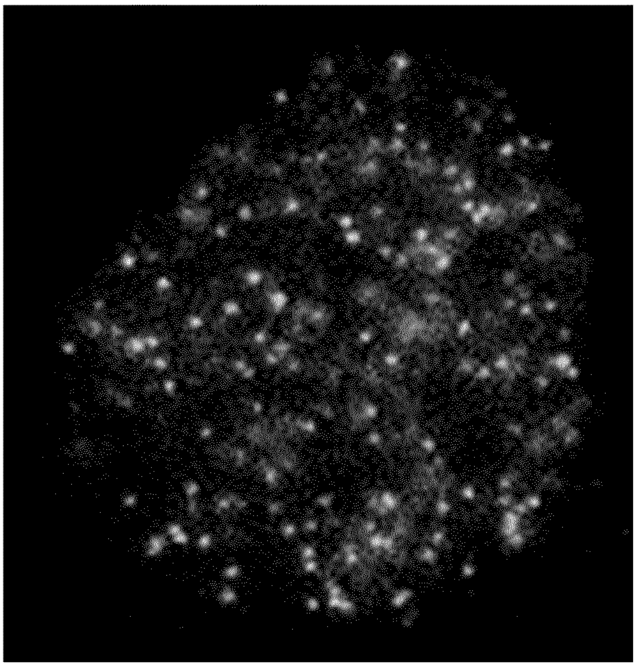

Fig. 5

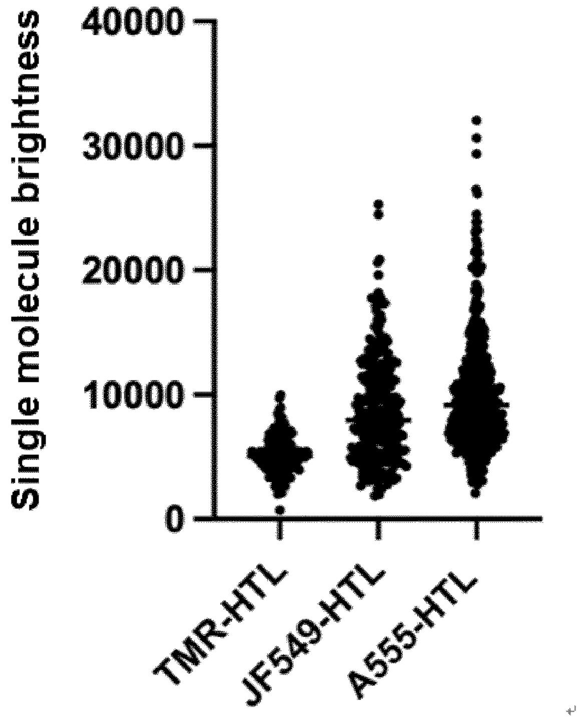
Single molecule brightness
40000
30000
20000
10000
0
TMR-HTL
JF549-HTL
A555-HTL

Fig. 6

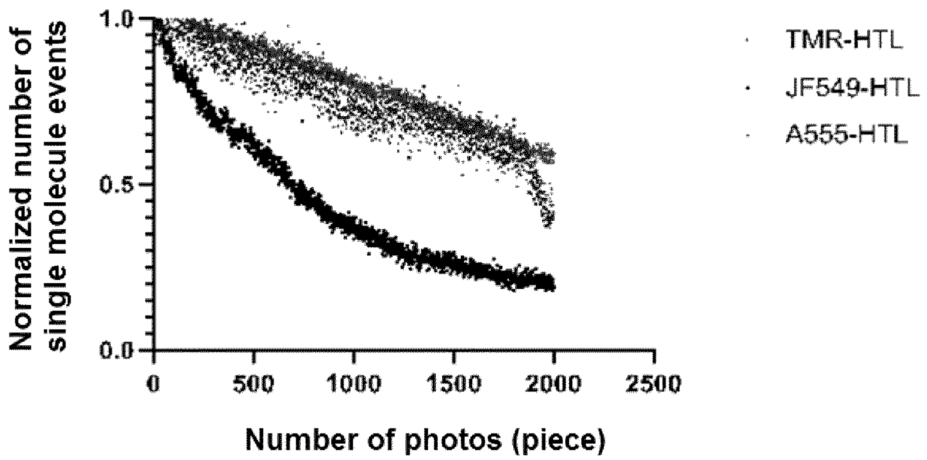
Normalized number of single molecule events
1.0
0.5
0.0
0
500
1000
1500
2000
2500
Number of photos (piece)
TMR-HTL
JF549-HTL
A555-HTL

Fig. 7

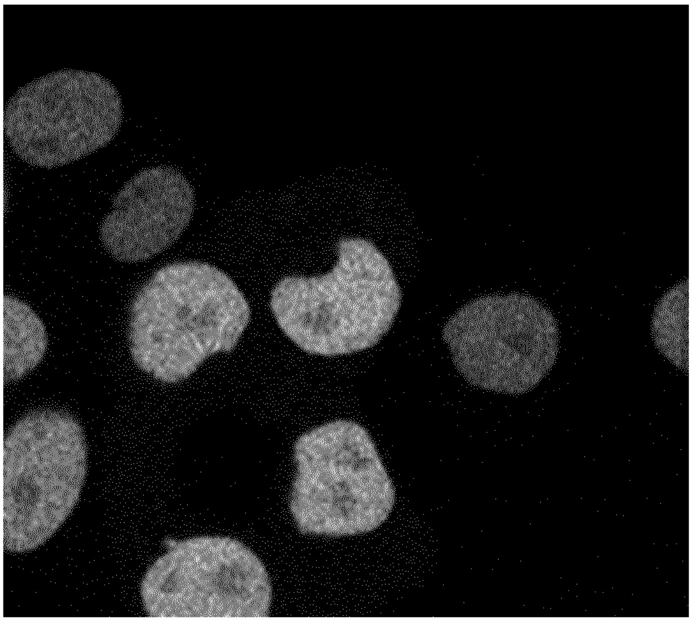

Fig. 8

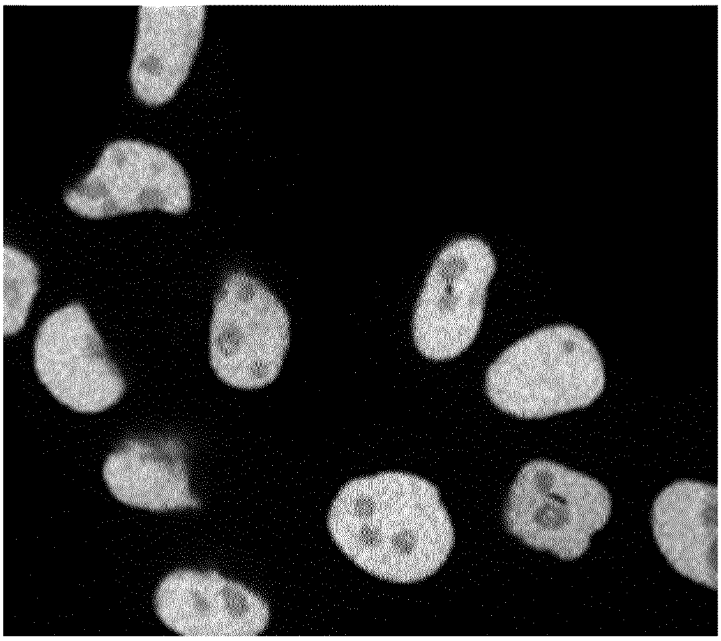

Fig. 9

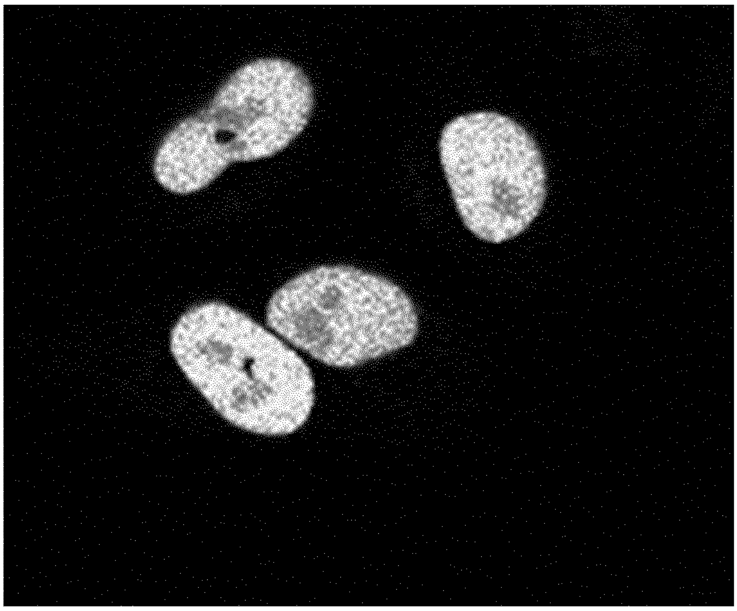

Fig. 10

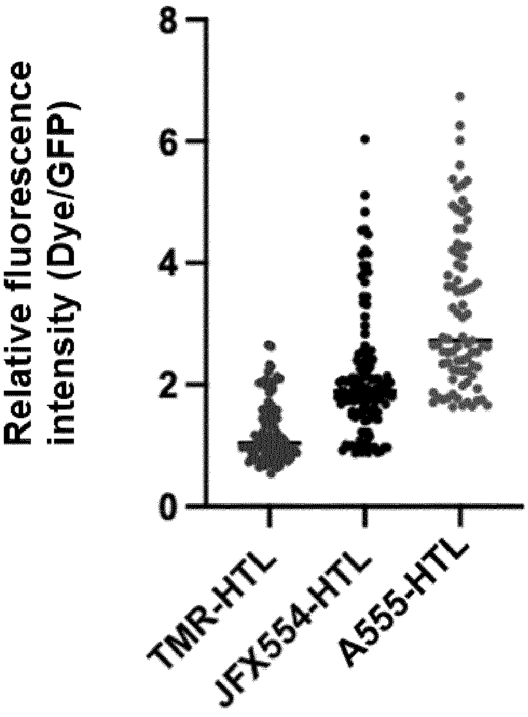
Relative fluorescence intensity (Dye/GFP)
8
6
4
2
0
TMR-HTL
JFX554-HTL
A555-HTL

Fig. 11

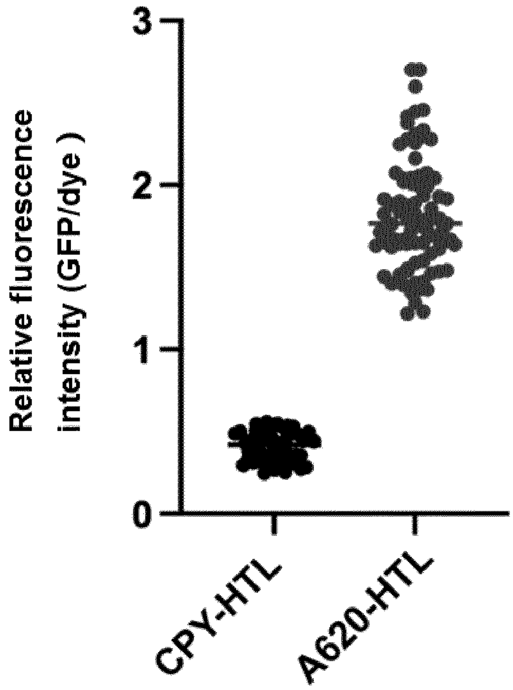
3
2
1
0
Relative fluorescence intensity (GFP/dye )
CPY-HTL
A620-HTL

Fig. 12

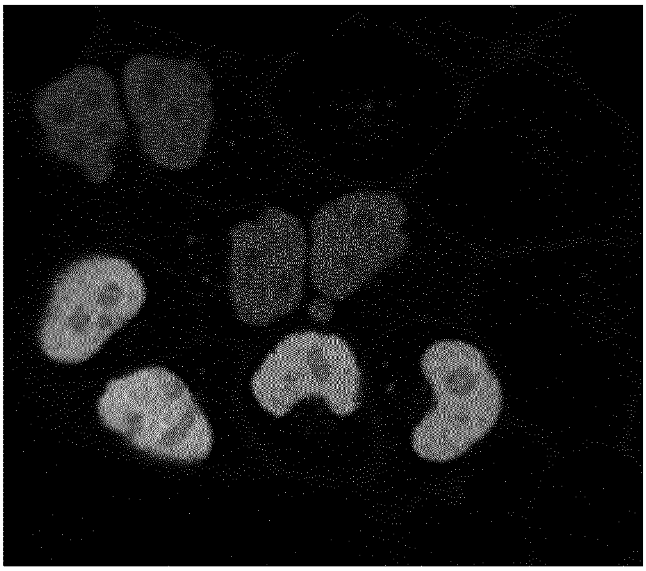

Fig. 13

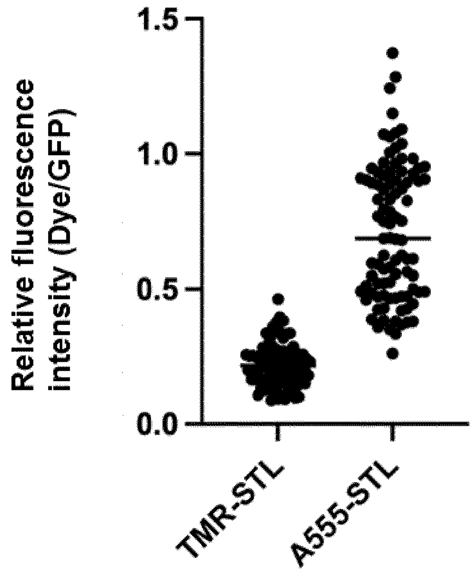
1.5
1.0
0.5
0.0
Relative fluorescence intensity (Dye/GFP)
TMR-STL
A555-STL

Fig. 14

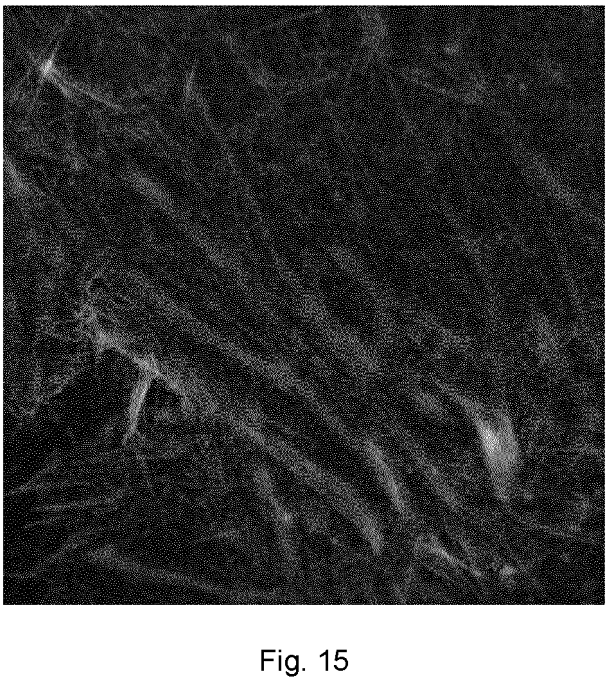

Fig. 15

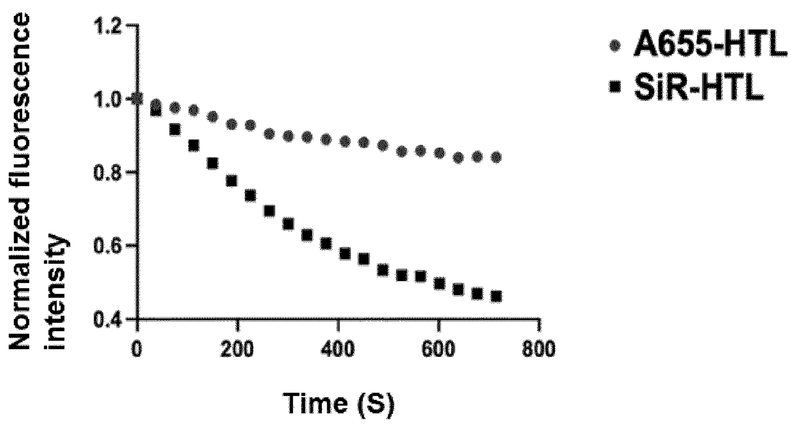
Normalized fluorescence intensity
1.2
1.0
0.8
0.6
0.4
0
200
400
600
800
Time (S)
A655-HTL
SiR-HTL

Fig. 16

**INTERNATIONAL SEARCH REPORT**

International application No.
**PCT/CN2023/117328**

**A. CLASSIFICATION OF SUBJECT MATTER**

C09B19/00(2006.01)i; C09B57/00(2006.01)i; C07D451/00(2006.01)i; A61K31/46(2006.01)i; G01N33/533(2006.01)i; G01N33/58(2006.01)i

According to International Patent Classification (IPC) or to both national classification and IPC

**B. FIELDS SEARCHED**

Minimum documentation searched (classification system followed by classification symbols)

C09B,C07D,A61K,G01N

Documentation searched other than minimum documentation to the extent that such documents are included in the fields searched

Electronic data base consulted during the international search (name of data base and, where practicable, search terms used)

CNTXT, ENTXTC, CJFD, VEN, WPABS, 百度, BAIDU, 万方, WANFANG, 超星读秀, DUXIU, Google-Scholar, Web of Science, STN: 北京大学, 南京浦海景珊生物技术有限公司, 陈知行, 张钧维, 刘明樵, 陈朋, 孙京府, 荧光, 染料, [3.2.1]桥环, 双环[3.2.1], 香豆素, 罗丹明, 荧光素, 双环, 桥环, dye+, fluoresce+, rhodamine+, coumarin+, cumarin+, oxazine+, +bicyclo+, +octyl+, 结构检索.

**C. DOCUMENTS CONSIDERED TO BE RELEVANT**

| Category* | Citation of document, with indication, where appropriate, of the relevant passages | Relevant to claim No. |
|---|---|---|
| X | WO 2010054183 A2 (HARVARD COLLEGE; SONG XIANGZHI; FOLEY JAMES W) 14 May 2010 (2010-05-14)<br>embodiments 2, 6, 10, and 20; page 3, line 13 to page 4, line 16; page 11, line 11 to page 12, line 3; page 15, line 1 to page 16, line 21; and page 35, lines 6-14 | 1-5, 9-10 |
| Y | WO 2010054183 A2 (HARVARD COLLEGE; SONG XIANGZHI; FOLEY JAMES W;) 14 May 2010 (2010-05-14)<br>embodiments 2, 6, 10, and 20; page 3, line 13 to page 4, line 16; page 11, line 11 to page 12, line 3; page 15, line 1 to page 16, line 21; and page 35, lines 6-14 | 6-8 |
| Y | CN 106471067 A (HOWARD HUGHES MEDICAL INSTITUTE) 01 March 2017 (2017-03-01)<br>figures 33, 36, and 38, and description, paragraphs 251, 254, and 256 | 6-8 |
| Y | WO 2019231497 A1 (HOWARD HUGHES MEDICAL INSTITUTE) 05 December 2019 (2019-12-05)<br>figures 68-70 | 6-8 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 March 2024** | **15 March 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2023/117328**

**C. DOCUMENTS CONSIDERED TO BE RELEVANT**

| Category* | Citation of document, with indication, where appropriate, of the relevant passages | Relevant to claim No. |
|---|---|---|
| A | CN 112812584 A (FUDAN UNIVERSITY) 18 May 2021 (2021-05-18)<br>entire document | 1-10 |

Form PCT/ISA/210 (second sheet) (July 2022)

**INTERNATIONAL SEARCH REPORT**
**Information on patent family members**

International application No.
**PCT/CN2023/117328**

| Patent document cited in search report | Publication date (day/month/year) | Patent family member(s) | Publication date (day/month/year) |
|---|---|---|---|
| WO 2010054183 A2 | 14 May 2010 | WO 2010054183 A3 | 30 September 2010 |
| CN 106471067 A | 01 March 2017 | EP 3126451 A1 | 08 February 2017 |
| | | EP 3126451 A4 | 20 September 2017 |
| | | EP 3126451 B1 | 09 September 2020 |
| | | US 2017045501 A1 | 16 February 2017 |
| | | US 9933417 B2 | 03 April 2018 |
| | | WO 2015153813 A1 | 08 October 2015 |
| | | US 2018284105 A1 | 04 October 2018 |
| | | US 10161932 B2 | 25 December 2018 |
| | | AU 2015240774 A1 | 13 October 2016 |
| | | AU 2015240774 B2 | 15 August 2019 |
| | | US 2018172677 A1 | 21 June 2018 |
| | | US 10018624 B1 | 10 July 2018 |
| | | US 2019107534 A1 | 11 April 2019 |
| | | US 10495632 B2 | 03 December 2019 |
| | | JP 2017519844 A | 20 July 2017 |
| | | JP 6606096 B2 | 13 November 2019 |
| | | CA 2944476 A1 | 08 October 2015 |
| | | CA 2944476 C | 15 June 2021 |
| WO 2019231497 A1 | 05 December 2019 | US 2021347994 A1 | 11 November 2021 |
| | | US 11787946 B2 | 17 October 2023 |
| | | US 2019367736 A1 | 05 December 2019 |
| | | US 11091643 B2 | 17 August 2021 |
| CN 112812584 A | 18 May 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023108090626


### Non-patent literature cited in the description


- *Nature Methods*, 2015, vol. 12, 244-250
- *J. Am. Chem. Soc.*, 2008, vol. 130 (52), 17652-17653
- *JACS Au*, 2021, vol. 1, 690-696
- *Angew. Chem. Int. Ed.*, 2016, vol. 55, 3290-3294
- *Nature Chemistry*, 2013, vol. 5, 132-139